(19) Europäisches Patentamt
European Patent Office
Office européen des brevets

(11) EP 0 728 758 B1

(12) EUROPÄISCHE PATENTSCHRIFT

(45) Veröffentlichungstag und Bekanntmachung des Hinweises auf die Patenterteilung:
**15.10.2003 Patentblatt 2003/42**

(21) Anmeldenummer: **96102446.0**

(22) Anmeldetag: **19.02.1996**

(51) Int Cl.[7]: **C07D 487/04**, C07D 487/14, C07D 471/14, C07D 513/14, A61K 31/40, A61K 31/425 // (C07D487/04, 209:00, 209:00), (C07D487/14, 209:00, 209:00, 209:00), (C07D471/14, 221:00, 209:00, 209:00), (C07D513/14, 277:00, 209:00, 209:00)

# (54) Dioxopyrrolo-pyrrolderivate

Dioxopyrrolo-pyrrole derivatives

Dérivés de dioxopyrrolo-pyrrole

(84) Benannte Vertragsstaaten:
**AT BE CH DE DK ES FR GB IT LI NL**

(30) Priorität: **27.02.1995 CH 55295**
**07.12.1995 CH 345795**

(43) Veröffentlichungstag der Anmeldung:
**28.08.1996 Patentblatt 1996/35**

(73) Patentinhaber: **F. HOFFMANN-LA ROCHE AG**
**4070 Basel (CH)**

(72) Erfinder:
- **Diederich, François**
**8006 Zürich (CH)**
- **Obst, Ulrike**
**8006 Zürich (CH)**
- **Wallbaum, Sabine**
**79639 Grenzach-Wyhlen (DE)**
- **Weber, Lutz**
**79639 Grenzach-Wyhlen (DE)**

(74) Vertreter: **Witte, Hubert, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 362 695**
**WO-A-94/14438**
**CH-A- 529 152**
**CH-A- 544 763**
**CH-A- 546 776**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 728 758 B1

## Beschreibung

Die Erfindung betrifft neue Dioxopyrrolo-pyrrolderivate, insbesondere Octahydro-1,3-dioxo-pyrrolo[3,4-c]pyrrolderivate der Formel

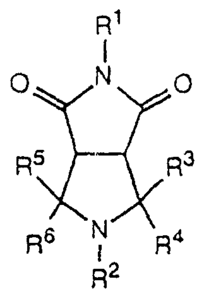

I

worin

| | |
|---|---|
| $R^1$ un $R^2$ | unabhängig voneinander H, nieder-Alkyl, Aryl, Heteroaryl, Cycloalkyl oder durch $CONH_2$ oder COO-nieder-Alkyl und/oder durch Aryl, Heteroaryl oder Cycloalkyl substituiertes nieder-Alkyl oder |
| $R^2$ | nieder-Alkanoyl, |
| $R^3$ | H, COOH, $CONH_2$, COO-nieder-Alkyl, CONH-nieder-Alkyl oder CON(nieder-Alkyl)$_2$ und |
| $R^4$, $R^5$ und $R^6$ | unabhängig voneinander H, nieder-Alkyl, Aryl, Aralkyl oder Cycloalkyl sind oder |
| $R^4$ und/oder eins von $R^5$ und $R^6$ | Heteroaryl oder durch OH, $SO_2H$, $SO_3H$, Guanidino, Aryl-niederalkoxy oder nieder-Alkyl-(O oder S) substituiertes nieder-Alkyl sind/ist oder |
| $R^2$ | zusammen mit $R^4$ eine Tri- oder Tetramethylengruppe G bildet, in der a) eine der Methylengruppen durch S, SO oder $SO_2$ ersetzt oder durch OH, nieder-Alkyl, nieder-Alkenyl oder Carboxyniederalkyl substituiert oder b) eine der Methylengruppen durch nieder-Alkenyl und eine andere durch nieder-Alkyl-COOH substituiert sein kann, |

wobei zumindest eins von $R^1$, $R^2$, $R^4$, $R^5$ und $R^6$ eine durch Amino, Amidino, Guanidino oder N-Hydroxyamidino substituierte Gruppe ist, wobei der Ausdruck "nieder" bis zu 6C-Atome enthaltende Gruppen bezeichnet, sowie Hydrate oder Solvate und physiologisch verwendbare Salze davon.

Ferner betrifft die Erfindung Verfahren zur Herstellung der obigen Verbindungen, pharmazeutische Präparate, die solche Verbindungen enthalten, sowie die Verwendung dieser Verbindungen bei der Herstellung von pharmazeutischen Präparaten.

Beispiele von physiologisch verwendbaren Salzen der Verbindungen der Formel I sind Salze mit physiologisch verträglichen Mineralsäuren, wie Salzsäure, Schwefelsäure, schweflige Säure oder Phosphorsäure; oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure. Die Verbindungen der Formel I mit sauren Gruppen, wie die Carboxygruppe, können auch Salze mit physiologisch verträglichen Basen bilden. Beispiele solcher Salze sind Alkalimetall-, Erdalkalimetall-, Ammonium- und Alkylammoniumsalze, wie das Na-, K-, Ca- oder Tetramethylammoniumsalz. Die Verbindungen der Formel I können auch in Form von Zwitterionen vorliegen.

Die Verbindungen der Formel I können solvatisiert, insbesondere hydratisiert sein. Die Hydratisierung kann im Zuge des Herstellungsverfahrens erfolgen oder allmählich als Folge hygroskopischer Eigenschaften einer zunächst wasserfreien Verbindung der Formel I auftreten.

Die Verbindungen der Formel I enthalten zumindest zwei asymmetrische C-Atome und können daher als Diastereomerengemisch, als Enantiomerengemisch oder als optisch reine Verbindung vorliegen.

Die vorliegenden Dioxopyrrolopyrrole haben eine andere Struktur als die in EP-A-0362695 beschriebenen Pyrrolocarbazole.

Im Rahmen der vorliegenden Erfindung bezeichnet "nieder" eine bis zu 6 C-Atome enthaltende geradkettige oder verzweigte Gruppe. Bevorzugte nieder-Alkyl- oder nieder-Alkanoylgruppen enthalten bis zu 4 C-Atome. Beispiele davon sind Methyl, Isopropyl, Butyl, Isobutyl, sec-Butyl und tert.Butyl bzw. Acetyl. "Aryl" allein oder in Kombination bezeichnet Gruppen, wie Phenyl, die substituiert sein können, z.B. durch Amidino, Guanidino, Hydroxyamidino Nitro,

Amino oder Methylendioxy. "Aralkyl" bezeichnet über nieder-Alkylen gebundenes Aryl, z.B. im Phenylring substituiertes Benzyl oder Phenethyl. "Cycloalkyl" bezeichnet gesättigte Gruppen mit 3 bis 7 C-Atome, wie Cyclohexyl. "Heteroaryl" bezeichnet 5- bis 10-gliedrige aromatische Gruppen, die z.B. aus zwei Ringen bestehen, ein (oder mehrere) N-Atom (e) enthalten und substituiert sein können, z.B. durch ein oder mehrere $NH_2$-Gruppen. Ein Beispiel ist Chinazolinyl, z. B. 2,4-Diaminochinazolin-6 oder 7-yl. Beispiele von durch Amino, Guanidino oder N-Hydroxyamidino substituierte Gruppen sind durch Amino substituiertes Chinazolinyl und (Amino, Amidino, Guanidino oder N-Hydroxyamidino)-(nieder-Alkyl, Phenyl oder Benzyl).

Beispiele von Verbindungen der Formel I sind diejenigen, worin $R^1$ bis $R^6$ die obigen Bedeutungen haben, jedoch $R^2$ keine Gruppe G mit $R^4$ bildet, insbesondere worin $R^2$ H, nieder-Alkyl, nieder-Alkanoyl oder durch $CONH_2$ substituiertes nieder-Alkyl, speziell H, Methyl, Carbamoylethyl oder Acetyl ist.

Weitere Beispiele von Verbindungen der Formel I sind diejenigen, worin $R^2$ zusammen mit $R^4$ eine wie in Anspruch 1 definierte Gruppe G bildet, nämlich der Formel

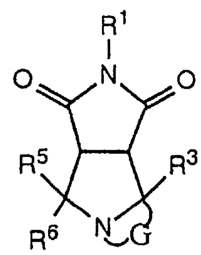

I'

insbesondere worin G eine Gruppe $(CH_2)_{3 \text{ oder } 4}$ ist, in der eine der $CH_2$-Gruppen durch S, CH-nieder-Alkyl oder CHOH ersetzt oder eine der $CH_2$-Gruppen durch nieder-Alkenyl und eine andere durch nieder-Alkyl-COOH substituiert sein kann, speziell worin G $(CH_2)_{3 \text{ oder } 4}$, $CH(CH_3)CH_2CH_2$, $CH_2SCH_2$, $CH_2S(O)_2CH_2$, $CH_2CH(OH)CH_2$ oder $CH(CH_2COOH)CH(\text{Isopropylen})CH_2$ ist.

Bevorzugt sind die Verbindungen der Formel I oder I', worin $R^1$ H, nieder-Alkyl, Aryl oder durch $CONH_2$ oder COO-nieder-Alkyl und/oder durch Aryl oder Cycloalkyl substituiertes nieder-Alkyl ist, insbesondere worin $R^1$ H, $CH_3$, Butyl, Phenyl, Benzyl, Phenethyl, Cyclohexylmethyl oder durch Nitro, Amino oder Methylendioxy substituiertes Benzyl, insbesondere Benzo[1,3]dioxol-5-ylmethyl ist.

Bevorzugte Verbindungen der Formel I' sind diejenigen, worin $R^1$ $CH_3$, Butyl, Phenyl, Benzyl, Cyclohexylmethyl, durch Methylendioxy substituiertes Benzyl oder durch $CONH_2$ oder COO-nieder-Alkyl und Aryl substituiertes nieder-Alkyl, insbesondere Benzo[1,3]dioxol-5-ylmethyl oder 1-(Carbamoyl oder Carbomethoxy)-2-phenethyl ist, speziell worin $R^3$ H oder COOH, insbesondere worin $R^5$ H und $R^6$ Aryl oder Heteroaryl, speziell Amidinophenyl, Guanidinophenyl oder Diaminochinazolin ist.

Bevorzugte Verbindungen der Formel I sind ferner diejenigen, worin $R^3$ H, COOH oder $CONH_2$, insbesondere worin $R^4$ H, nieder-Alkyl, Aryl, Aralkyl oder durch OH, $SO_2H$, Guanidino, Aralkoxy oder nieder-Alkylthio substituiertes nieder-Alkyl ist, speziell worin $R^4$ H, Methyl, Isopropyl, Isobutyl, 2-Butyl, tert.-Butyl, Phenyl, Benzyl, $CH_2OH$, $CH_2SO_2H$, Guanidinopropyl, Benzyloxymethyl oder $(CH_2)_2SCH_3$ ist.

Bevorzugte Verbindungen der Formel I sind auch diejenigen, worin $R^5$ und $R^6$ unabhängig voneinander H, nieder-Alkyl, Aryl oder Aralkyl, insbesondere H, Methyl, tert.-Butyl, Phenyl, Phenethyl, Amidinophenyl oder Hydroxyamidinophenyl sind.

Beispiele von bevorzugten Verbindungen der Formeln I bzw. I' sind

(1RS,3SR,3aRS,6aSR)-4-(5-Benzyl-2,3-dimethyl-4,6-dioxo-octahydropyrrolo[3,4-c]pyrrol-1-yl)-benzimidamid bzw.

(3aRS,4SR,8aRS,8bSR)-4-(2-Benzyl-1,3-dioxo-decahydro-pyrrolo[3,4-a]-pyrrolizin-4-yl)-benzimidamid

(3aRS,4SR,8aRS,8bSR)-4-{2-(Benzo[1,3]dioxol-5-ylmethyl)-1,3-dioxodecahydro-pyrrolo[3,4-a]pyrrolizin-4-yl}-benzimidamid

(3aRS,4SR,8aRS,8bSR)-4-(2-Cyclohexylmethyl-1,3-dioxo-decahydropyrrolo [3,4-a] pyrrolizin-4-yl)-benzimidamid

(5RS,5aSR,8aRS,8bRS)-4-{7-(Benzo[1,3]dioxol-5-ylmethyl)-6,8-dioxooctahydro-pyrrolo[3',4':3,4]pyrrolo[1,2-c]thiazol-5-yl}-benzimidamid

(3aR,4S,7S,8aR,8bS)-4-(2-Benzyl-7-hydroxy-1,3-dioxo-decahydropyrrolo[3,4-a]pyrrolizin-4-yl)-benzimidamid und

(3aRS,4SR,8aRS,8bSR)-*N*-{4-(2-Butyl-1,3-dioxo-decahydro-pyrrolo[3,4-a]-pyrrolizin-4-yl)-phenyl}-guanidin.

Weitere bevorzugte Verbindungen der Formel I sind:

(1RS,3RS,3aSR,6aRS)-1-(Benzyloxymethyl)-3-(4-carbamimidoyl-phenyl)-5-(4-nitro-benzyl)-4,6-dioxo-octahydro-pyrrolo[3,4-c]pyrrol-1-carbonsäure und
(1RS,3RS,3aSR,6aRS)-5-(4-Amino-benzyl)-1-(benzyloxy-methyl)-3-(4-carbamimidoyl-phenyl)-4,6-dioxo-octahydro-pyrrolo[3,4-c]pyrrol-1-carbonsäure.

Weitere Beispiele von Verbindungen der Formeln I bzw. I' sind:

(1RS,3SR,3aRS,6aSR)-5-Benzyl-1-(3-guanidino-propyl)-4,6-dioxooctahydro-3-phenyl-pyrrolo[3,4-c]pyrrol-1-carbonsäure
(1RS,3aSR,6aRS)-4-(5-Benzyl-2,3,3-trimethyl-4,6-dioxo-octahydropyrrolo[3,4-c]pyrrol-1-ylmethyl)-benzimidamid
(3aRS,4RS,6aSR)-4-[4-(Amino-hydroxyimino-methyl)-benzyl]-2-benzyl-5,6,6-trimethyl-tetrahydro-pyrrolo[3,4-c]pyrrol-1,3-dion
(1RS,3SR,3aRS,6aSR)-4-(5-Benzyl-3-methyl-4,6-dioxo-octahydropyrrolo[3,4-c]pyrrol-1-yl)-benzimidamid
(1RS,3SR,3aRS,6aSR)-4-(2-Acetyl-5-benzyl-3-methyl-4,6-dioxo-octahydropyrrolo[3,4-c]pyrrol-1-yl)-benzimidamid
(1RS,3SR,3aRS,6aSR)-4-(5-Butyl-2,3-dimethyl-4,6-dioxo-octahydropyrrolo[3,4-c]pyrrol-1-yl)-benzimidamid
( 1RS,3SR,3aRS,6aSR)-4-(2-Acetyl-5-butyl-3-methyl-4,6-dioxo-octahydropyrrolo[3,4-c]pyrrol-1-yl)-benzimidamid
(3aRS,4SR,6RS,6aSR)-4-[4-(Amino-hydroxyimino-methyl)-phenyl]-2-benzyl-5,6-dimethyl-tetrahydro-pyrrolo[3,4-c]pyrrol-1,3-dion
(1RS,3SR,3aRS,6aSR)-4-(5-Butyl-3-methyl-4,6-dioxo-octahydropyrrolo[3,4-c]pyrrol-1-yl)-benzimidamid
(1RS,3SR,3aRS,6aSR)-4-(5-Benzyl-3-isobutyl-4,6-dioxo-octahydropyrrolo[3,4-c]pyrrol-1-yl)-benzimidamid
(1RS,3SR,3aRS,6aSR)-4-(5-Benzyl-3-isobutyl-2-methyl-4,6-dioxooctahydro-pyrrolo[3,4-c]pyrrol-1-yl)-benzimidamid
(1RS,3RS,3aSR,6aRS)-{5-(Benzo[1,3]dioxol-5-ylmethyl)-3-(4-carbamimidoyl-phenyl)-4,6-dioxo-octahydro-pyrrolo[3,4-c]pyrrol-1-yl}methansulfinsäure
(1RS,3RS,3aRS,6aSR)-{5-(Benzo[1,3]dioxol-5-ylmethyl)-3-(4-carbamimidoyl-phenyl)-4,6-dioxo-octahydro-pyrrolo[3,4-c]pyrrol-1-yl}-methansulfinsäure
(1RS,3SR,3aRS,6aSR)-3-(4-Carbamimidoyl-phenyl)-1-(2-methylsulfanylethyl)-4,6-dioxo-5-phenyl-octahydro-pyrrolo[3,4-c]pyrrol-1-carbonsäure
(1RS,3SR,3aRS,6aSR)-4-{5-(Benzo[1,3)dioxol-5-ylmethyl)-3-hydroxymethyl-4,6-dioxo-octahydro-pyrrolo[3,4-c]pyrrol-1-yl}-benzimidamid
(1RS,3RS,3aSR,6aRS)-4-{5-(Benzo[1,3]dioxol-5-ylmethyl)-3-hydroxymethyl-4,6-dioxo-octahydro-pyrrolo[3,4-c]pyrrol-1-yl}-benzimidamid
3-*tert*.Butyl-1-(3-guanidino-propyl)-4,6-dioxo-octahydro-pyrrolo-[3,4-c]pyrrol-1-carboxamid
3-Phenyl-1-(3-guanidino-propyl)-4,6-dioxo-octahydro-pyrrolo-[3,4-c]pyrrol-1-carboxamid
3-(2-Phenyl-ethyl)-1-(3-guanidino-propyl)-4,6-dioxo-octahydro-pyrrolo-[3,4-c]pyrrol-1-carboxamid
5-Methyl-3-(2-phenyl-ethyl)-1-(3-guanidino-propyl)-4,6-dioxo-octahydropyrrolo[3,4-c]pyrrol-1-carboxamid
5-Butyl-3-(2-phenyl-ethyl)-1-(3-guanidino-propyl)-4,6-dioxo-octahydropyrrolo[3,4-c]pyrrol-1-carboxamid
5-Cyclohexylmethyl-3-(2-phenyl-ethyl)-1-(3-guanidino-propyl)-4,6-dioxooctahydro-pyrrolo[3,4-c]pyrrol-1-carboxamid
5-Benzyl-3-(2-phenyl-ethyl)-1-(3-guanidino-propyl)-4,6-dioxo-octahydropyrrolo[3,4-c]pyrrol-1-carboxamid
5-Phenyl-3-(2-phenyl-ethyl)-1-(3-guanidino-propyl)-4,6-dioxo-octahydropyrrolo[3,4-c]pyrrol-1-carboxamid
5-Butyl-3,3-diphenyl-1-(3-guanidino-propyl)-4,6-dioxo-octahydro-pyrrolo-[3,4-c]pyrrol-1-carboxamid
5-Butyl-3-phenyl-1-(3-guanidino-propyl)-4,6-dioxo-octahydro-pyrrolo-[3,4-c]pyrrol-1-carboxamid
5-Cyclohexylmethyl-3-phenyl-1-(3-guanidino-propyl)-4,6-dioxooctahydro-pyrrolo[3,4-c]pyrrol-1-carboxamid
5-Benzyl-3-phenyl-1-(3-guanidino-propyl)-4,6-dioxo-octahydro-pyrrolo-[3,4-c]pyrrol-1-carboxamid
3,5-Diphenyl-1-(3-guanidino-propyl)-4,6-dioxo-octahydro-pyrrolo-[3,4-c]pyrrol-1-carboxamid
5-Butyl-3-*tert*.butyl-1-(3-guanidino-propyl)-4,6-dioxo-octahydro-pyrrolo-[3,4-c]pyrrol-1-carboxamid
5-Butyl-1-(3-guanidino-propyl)-4,6-dioxo-octahydro-pyrrolo[3,4-c]pyrrol-1-carboxamid
1-(3-Guanidino-propyl)-4,6-dioxo-octahydro-5-phenyl-pyrrolo[3,4-c]-pyrrol-1-carboxamid
5-Methyl-3-(4-carbamimidoyl-phenyl)-4,6-dioxo-octahydro-pyrrolo-[3,4-c]pyrrol-1-carbonsäure
1,5-Dimethyl-3-(4-carbamimidoyl-phenyl)-4,6-dioxo-octahydro-pyrrolo-[3,4-c]pyrrol- 1-carbonsäure
5-Methyl-1-(2-propyl)-3-(4-carbamimidoyl-phenyl)-4,6-dioxo-octahydropyrrolo[3,4-c]pyrrol-1-carbonsäure
1-(2-Butyl)-5-methyl-3-(4-carbamimidoyl-phenyl)-4,6-dioxo-octahydropyrrolo[3,4-c]pyrrol-1-carbonsäure
5-Methyl-1-(2-methylsulfanyl-ethyl)-3-(4-carbamimidoyl-phenyl)-4,6-dioxo-octahydro-pyrrolo[3,4-c]pyrrol-1-carbonsäure

5-Butyl-3-(4-carbamimidoyl-phenyl)-4,6-dioxo-octahydro-pyrrolo-[3,4-c]pyrrol-1-carbonsäure
5-Butyl-1-methyl-3-(4-carbamimidoyl-phenyl)-4,6-dioxo-octahydropyrrolo[3,4-c]pyrrol-1-carbonsäure
5-Butyl-1-(2-propyl)-3-(4-carbamimidoyl-phenyl)-4,6-dioxo-octahydropyrrolo [3,4-c] pyrrol-1-carbonsäure
5-Butyl-1-(2-butyl)-3-(4-carbamimidoyl-phenyl)-4,6-dioxo-octahydropyrrolo[3,4-c]pyrrol-1-carbonsäure
5-Butyl-1-(2-methylsulfanyl-ethyl)-3-(4-carbamimidoyl-phenyl)-4,6-dioxo-octahydro-pyrrolo[3,4-c]pyrrol-1-carbonsäure
5-Cyclohexylmethyl-3-(4-carbamimidoyl-phenyl)-4,6-dioxo-octahydropyrrolo[3,4-c]pyrrol-1-carbonsäure
5-Cyclohexylmethyl-1-(2-propyl)-3-(4-carbamimidoyl-phenyl)-4,6-dioxooctahydro-pyrrolo[3,4-c]pyrrol-1-carbonsäure
1-(2-Butyl)-5-cyclohexylmethyl-3-(4-carbamimidoyl-phenyl)-4,6-dioxooctahydro-pyrrolo[3,4-c]pyrrol-1-carbonsäure
5-Cyclohexylmethyl-1-(2-methylsulfanyl-ethyl)-3-(4-carbamimidoylphenyl)-4,6-dioxo-octahydro-pyrrolo [3,4-c] pyrrol-1-carbonsäure
5-Phenyl-3-(4-carbamimidoyl-phenyl)-4,6-dioxo-octahydro-pyrrolo-[3,4-c]pyrrol-1-carbonsäure
1-Methyl-5-phenyl-3-(4-carbamimidoyl-phenyl)-4,6-dioxo-octahydropyrrolo[3,4-c]pyrrol- 1-carbonsäure
5-phenyl-1-(2-propyl)-3-(4-carbamimidoyl-phenyl)-4,6-dioxo-octahydropyrrolo[3,4-c]pyrrol-1-carbonsäure
1-(2-Butyl)-5-phenyl-3-(4-carbamimidoyl-phenyl)-4,6-dioxo-octahydropyrrolo[3,4-c]pyrrol-1-carbonsäure
1-(2-Methylsulfanyl-ethyl)-5-phenyl-3-(4-carbamimidoyl-phenyl)-4,6-dioxo-octahydro-pyrrolo[3,4-c]pyrrol-1-carbonsäure
5-Benzyl-3-(4-carbamimidoyl-phenyl)-4,6-dioxo-octahydro-pyrrolo-[3,4-c]pyrrol-1-carbonsäure
5-Benzyl-1-methyl-3-(4-carbamimidoyl-phenyl)-4,6-dioxo-octahydropyrrolo[3,4-c]pyrrol-1-carbonsäure
5-Benzyl-1-(2-propyl)-3-(4-carbamimidoyl-phenyl)-4,6-dioxo-octahydropyrrolo[3,4-c]pyrrol-1-carbonsäure
5-Benzyl-1-(2-butyl)-3-(4-carbamimidoyl-phenyl)-4,6-dioxo-octahydropyrrolo[3,4-c]pyrrol-1-carbonsäure
5-Benzyl-3-(4-carbamimidoyl-phenyl)-1-(2-methylsulfanyl-ethyl)-4,6-dioxo-octahydro-pyrrolo[3,4-c]pyrrol-1-carbonsäure
5-Methyl-1-(4-carbamimidoyl-phenyl)-2-(2-carbamoyl-ethyl)-4,6-dioxooctahydro-pyrrolo[3,4-c]pyrrol-3-carbonsäure
3,5-Dimethyl-1-(4-carbamimidoyl-phenyl)-2-(2-carbamoyl-ethyl)-4,6-dioxo-octahydro-pyrrolo[3,4-c]pyrrol-3-carbonsäure
5-Methyl-3-(prop-2-yl)-1-(4-carbamimidoyl-phenyl)-2-(2-carbamoylethyl)-4,6-dioxo-octahydro-pyrrolo[3,4-c]pyrrol-1-carbonsäure
5-Methyl-3-(2-methyl-propyl)-1-(4-carbamimidoyl-phenyl)-2-(2-carbamoyl-ethyl)-4,6-dioxo-octahydro-pyrrolo [3,4-c]pyrrol-1-carbonsäure
3-Hydroxymethyl-5-methyl-1-(4-carbamimidoyl-phenyl)-2-(2-carbamoylethyl)-4,6-dioxo-octahydro-pyrrolo[3,4-c] pyrrol-1-carbonsäure
3-Benzyl-5-methyl-1-(4-carbamimidoyl-phenyl)-2-(2-carbamoyl-ethyl)-4,6-dioxo-octahydro-pyrrolo[3,4-c]pyrrol-1-carbonsäure
5-Butyl-1-(4-carbamimidoyl-phenyl)-2-(2-carbamoyl-ethyl)-4,6-dioxooctahydro-pyrrolo[3,4-c]pyrrol-1-carbonsäure
5-Butyl-3-methyl-1-(4-caxbamimidoyl-phenyl)-2-(2-carbamoyl-ethyl)-4,6-dioxo-octahydro-pyrrolo[3,4-c]pyrrol-1-carbonsäure
5-Butyl-3-(prop-2-yl)-1-(4-carbamimidoyl-phenyl)-2-(2-carbamoyl-ethyl)-4,6-dioxo-octahydro-pyrrolo[3,4-c]pyrrol-1-carbonsäure
5-Butyl-3-(2-methyl-propyl)-1-(4-carbamimidoyl-phenyl)-2-(2-carbamoylethyl)-4,6-dioxo-octahydro-pyrrolo[3,4-c] pyrrol-1-carbonsäure
5-Butyl-3-hydroxymethyl-1-(4-carbamimidoyl-phenyl)-2-(2-carbamoylethyl)-4,6-dioxo-octahydro-pyrrolo[3,4-c] pyrrol-1-carbonsäure
3-Benzyl-5-butyl-1-(4-carbamimidoyl-phenyl)-2-(2-carbamoyl-ethyl)-4,6-dioxo-octahydro-pyrrolo[3,4-c]pyrrol-1-carbonsäure
3-Methyl-5-phenyl-1-(4-carbamimidoyl-phenyl)-2-(2-carbamoyl-ethyl)-4,6-dioxo-octahydro-pyrrolo[3,4-c]pyrrol-1-carbonsäure
5-Phenyl-3-(prop-2-yl)-1-(4-carbamimidoyl-phenyl)-2-(2-carbamoylethyl)-4,6-dioxo-octahydro-pyrrolo[3,4-c]pyrrol-1-carbonsäure
3-(2-Methyl-propyl)-5-phenyl-1-(4-carbamimidoyl-phenyl)-2-(2-carbamoyl-ethyl)-4,6-dioxo-octahydro-pyrrolo [3,4-c]pyrrol-1-carbonsäure
3-Hydroxymethyl-5-phenyl-1-(4-carbamimidoyl-phenyl)-2-(2-caxbamoylethyl)-4,6-dioxo-octahydro-pyrrolo[3,4-c] pyrrol-1-carbonsäure;
3-Benzyl-5-phenyl-1-(4-carbamimidoyl-phenyl)-2-(2-carbamoyl-ethyl)-4,6-dioxo-octahydro-pyrrolo[3,4-c]pyrrol-1-carbonsäure
5-Benzyl-3-methyl-1-(4-carbamimidoyl-phenyl)-2-(2-carbamoyl-ethyl)-4, 6-dioxo-octahydro-pyrrolo[3,4-c]pyrrol-1-carbonsäure
5-Benzyl-3-(2-methyl-propyl)-1-(4-carbamimidoyl-phenyl)-2-(2-carbamoyl-ethyl)-4,6-dioxo-octahydro-pyrrolo[3,4-c]pyrrol-1-carbonsäure
5-Benzyl-3-Hydroxymethyl-1-(4-carbamimidoyl-phenyl)-2-(2-carbamoylethyl)-4,6-dioxo-octahydro-pyrrolo[3,4-c]pyrrol-1-carbonsäure
3,5-Dibenzyl-1-(4-carbamimidoyl-phenyl)-2-(2-carbamoyl-ethyl)-4,6-dioxo-octahydro-pyrrolo[3,4-c]pyrrol-1-carbonsäure
5-(2-Phenyl-ethyl)-1-(4-carbamimidoyl-phenyl)-2-(2-carbamoyl-ethyl)-4,6-dioxo-octahydro-pyrrolo[3,4-c]pyrrol-1-carbonsäure
5-(2-Phenyl-ethyl)-3-(prop-2-yl)-1-(4-carbamimidoyl-phenyl)-2-(2-carbamoyl-ethyl)-4,6-dioxo-octahydro-pyrrolo[3,4-c]pyrrol-1-carbonsäure
3-(2-Methyl-propyl)-5-(2-phenyl-ethyl)-1-(4-carbamimidoyl-phenyl)-2-(2-carbamoyl-ethyl)-4,6-dioxo-octahydro-pyrrolo[3,4-c]pyrrol-1-carbonsäure
3-Hydroxymethyl-5-(2-phenyl-ethyl)-1-(4-carbamimidoyl-phenyl)-2-(2-carbamoyl-ethyl)-4,6-dioxo-octahydro-pyrrolo[3,4-c]pyrrol-1-carbonsäure
3-Benzyl-1-(4-carbamimidoyl-phenyl)-2-(2-carbamoyl-ethyl)-4,6-dioxooctahydro-5-(2-phenyl-ethyl)-pyrrolo[3,4-c]pyrrol-3-carbonsäure bzw.
(3aRS,4SR,8aRS,8bSR)-4-(2-Butyl-1,3-dioxo-decahydro-pyrrolo [3,4-a]-pyrrolizin-4-yl)-benzimidamid
(3aRS,4RS,9aSR,9bSR)-4-(2-Benzyl-1,3-dioxo-decahydro-pyrrolo[3,4-c]-indolizin-4-yl)-benzimidamid
(3aRS,4SR,9aRS,9bSR)-4-(2-Benzyl-1,3-dioxo-decahydro-pyrrolo[3,4-c]-indolizin-4-yl)-benzimidamid
(3aRS,4RS,8aSR,8bSR)-4-{2-(Benzo[1,3]dioxol-5-ylmethyl)-1,3-dioxodecahydro-pyrrolo[3,4-a]pyrrolizin-4-yl}-benzimidamid
(5RS,5aRS,8aSR,8bRS)-4-{7-(Benzo[1,3]dioxol-5-ylmethyl)-6,8-dioxooctahydro-pyrrolo[3',4':3,4]pyrrolo[1,2-c]thiazol-5-yl}-benzimidamid
(3aS,4R,8S,8aS,8bR)-4-{2-(Benzo[1,3]dioxol-5-ylmethyl)-8-methyl-1,3-dioxo-decahydro-pyrrolo[3,4-a]pyrrolizin-4-yl}-benzimidamid
(3aR,4R,7R,8aS,8bS)-4-(2-Benzyl-7-hydroxy-1,3-dioxo-decahydropyrrolo[3,4-a]pyrrolizin-4-yl)-benzimidamid
(R)-2-{(3aS,4S,8aR,8bR)- und -(3aR,4R,8aS,8bS)-4-(4-Carbamimidoylphenyl)-1,3-dioxo-decahydro-pyrrolo[3,4-a]pyrrolizin-2-yl}-3-phenylpropionsäuremethylester
(R)-2-{(3aR,4S,8aR,8bS)- und -(3aS,4R,8aR.8bR)-4-(4-Carbamimidoylphenyl)-1,3-dioxo-decahydro-pyrrolo[3,4-a]pyrrolizin-2-yl}-3-phenylpropionsäuremethylester
(1S,2S,5R,5aS,8aR)-{7-Benzyl-5-(4-carbamimidoyl-phenyl)-2-(propen-2-yl)-6,8-dioxo-decahydro-pyrrolo[3,4-a]pyrrolizin-1-yl)-essigsäure
(5RS,5aSR,8aRS,8bRS)-4-{7-(Benzo[1,3]dioxol-5-ylmethyl)-2,2,6,8-tetraoxo-octahydro-pyrrolo[3',4':3,4]pyrrolo[1,2-c]thiazol-5-yl}-benzimidamid
(3aRS,4SR,8aRS,8bSR)-*N*-{4-(2-Benzyl-1,3-dioxo-decahydro-pyrrolo-[3,4-a]pyrrolizin-4-yl)-phenyl}-guanidin
(3aRS,4SR,8aRS,8bSR)-*N*-[4-{2-(Benzo[1,3]dioxol-5-ylmethyl)-1,3-dioxodecahydro-pyrrolo[3,4-a]pyrrolizin-4-yl}-phenyl]-guanidin
(3aRS,4SR,8aRS,8bSR)-2-Benzyl-4-(2,4-diamino-quinazolin-6-yl)-hexahydro-pyrrolo[3,4-a]pyrrolizin-1,3-dion
(3aRS,4RS,8aSR,8bSR)-2-Benzyl-4-(2,4-diamino-quinazolin-6-yl)-hexahydro-pyrrolo[3,4-a]pyrrolizin-1,3-dion
(3aRS,4SR,8aRS,8bSR)-2-Benzyl-4-(2,4-diamino-quinazolin-7-yl)-hexahydro-pyrrolo[3,4-a]pyrrolizin-1,3-dion
{2-Methyl-4-(4-carbamimidoyl-phenyl)-1,3-dioxo-decahydro-pyrrolo-[3,4-a]pyrrolizin-8a-yl}-carbonsäure
{2-Butyl-4-(4-carbamimidoyl-phenyl)-1,3-dioxo-decahydro-pyrrolo-[3,4-a]pyrrolizin-8a-yl}-carbonsäure
{2-Cyclohexylmethyl-4-(4-carbamimidoyl-phenyl)-1,3-dioxo-decahydropyrrolo[3,4-a]pyrrolizin-8a-yl}-carbonsäure
{2-phenyl-4-(4-carbamimidoyl-phenyl)-1,3-dioxo-decahydro-pyrrolo-[3,4-a]pyrrolizin-8a-yl}-carbonsäure
{2-Benzyl-4-(4-carbamimidoyl-phenyl)-1,3-dioxo-decahydro-pyrrolo-[3,4-a]pyrrolizin-8a-yl}-carbonsäure und
2-{4-(4-Carbamimidoyl-phenyl)-1,3-dioxo-decahydro-pyrrolo[3,4-a]-pyrrolizin-2-yl}-3-phenyl-propionsäureamid.

Weitere Beispiele von Verbindungen der Formel I sind:

(1RS,3RS,3aSR,6aRS)-4-[3-Benzyloxy-methyl)-5-(4-nitro-benzyl)-4,6-dioxo-octahydropyrrolo[3,4-c]pyrrol-1-yl]-benzimidamid und
(1RS,3RS,3aSR,6aRS)-4-[5-(4-Amino-benzyl)-3-(benzyloxy-methyl)-4,6-dioxooctahydro-pyrrolo[3,4-c]pyrrol-1-yl]-benzimidamid.

Die erfindungsgemässen Verbindungen können dadurch hergestellt werden, dass man

a) ein Maleimid der Formel

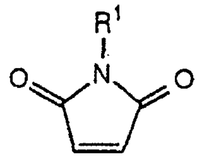

II

mit einer α-Aminocarbonsäure der Formel

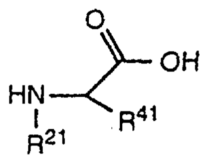

III

worin $R^{21}$ und $R^{41}$ die gleiche Bedeutung wie $R^2$ bzw. $R^4$ haben mit der Massgabe, dass $R^{21}$ nicht nieder-Alkanoyl ist und dass eine durch $R^{21}$ zusammen mit $R^{41}$ gebildete Gruppe G keine Gruppe SO oder $SO_2$ enthält, oder einem funktionellen Derivat davon und mit einer Verbindung der Formel

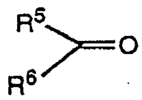

IV

umsetzt,

b) zur Herstellung einer Verbindung der Formel I, in der eins von $R^4$, $R^5$ und $R^6$ eine durch Amidino, N-Hydroxyamidino oder Guanidino substituierte Phenyl- oder nieder-Alkylgruppe ist, eine der Verbindung der Formel I entsprechende Verbindung, die eine durch CN oder $NO_2$ substituierte Phenyl- oder nieder-Alkylgruppe anstelle der durch Amidino, N-Hydroxyamidino oder Guanidino substituierten Phenyl- oder nieder-Alkylgruppe enthält, amidiniert, N-hydroxyamidiniert oder guanidiniert,

c) gewünschtenfalls eine in einer Verbindung der Formel I enthaltene reaktionsfähige Gruppe funktionell abwandelt, und

d) gewünschtenfalls eine Verbindung der Formel I in ein physiologisch verträgliches Salz überführt oder ein Salz einer Verbindung der Formel I in die freie Säure oder Base überführt.

Als funktionelle Derivate einer Säure der Formel III können nieder-Alkylester sowie als Ester oder Amid an einen festen Träger, z.B. einem Harz, gebundene Säuren der Formel III genannt werden. Zweckmässig wird die Reaktion a) des Maleinimids der Formel II mit der α-Aminosäure der Formel III und dem Keton oder dem Aldehyd der Formel IV in einem Lösungsmittel, wie DMF, DMSO, Acetonitril, THF, Diethylether, Benzol, Toluol, Essigsäure, Methanol oder Ethanol, bei einer Temperatur zwischen 0 °C und 160 °C durchgeführt. Besonders vorteilhaft führt man die Reaktion entweder bei erhöhter Temperatur in DMF, DMSO oder Toluol durch. Ebenso vorteilhaft kann die Reaktion bei erhöhter Temperatur in Methanol unter Zugabe katalytischer Mengen Essigsäure oder auch in Acetonitril bei Raumtemperatur unter Zugabe von Silberacetat und einer tertiären Stickstoffbase, wie Triethylamin, Diazabicyclooctan oder Ethylmorpholin durchgeführt werden. Die Reaktion a) kann besonders vorteilhaft so durchgeführt werden, dass entweder das Maleimid der Formel II oder die α-Aminocarbonsäure der Formel III über eine in den Resten $R^1$ oder $R^2$ enthaltene Carboxygruppe oder über die Carboxygruppe der α-Aminocarbonsäure der Formel III an eine feste Phase, wie ein Polystyrenharz, unter Ausbildung einer Ester- oder Amidgruppe gebunden ist und das Keton oder der Aldehyd der Formel IV und die α-Aminocarbonsäure der Formel III oder das Maleinimid der Formel II, welches nicht an die

feste Phase gebunden ist, in einem bis zu 20-fachen Ueberschuss im Vergleich zu der an der festen Phase gebundenen Verbindung in die Reaktion einsetzt.

Zur Umwandlung einer Cyangruppe in die Amidinogruppe nach der Verfahrensvariante b) kann man das Ausgangsnitril in einem Lösungsmittel, wie Ethanol oder Methanol, oder einem Lösungsmittelgemisch, wie Chloroform und Methanol oder Chloroform und Ethanol, mit einem trockenen Strom von Chlorwasserstoff, zweckmässig bei einer Temperatur unter 10°C begasen. Die Reaktionslösung wird mit einem Lösungsmittel, wie Diethylether, versetzt und das so ausgefallene Zwischenprodukt, ein Imidoether-hydrochlorid, abfiltriert. Danach kann man das Zwischenprodukt in Wasser lösen, mit einer Base, wie Natriumcarbonat oder Natriumhydroxid, neutralisieren und aus der wässrigen Phase mit einem Lösungsmittel, wie Dichlormethan, Chloroform oder Essigester, extrahieren. Der so erhaltene Imidoether wird in einem Lösungsmittel, wie Methanol oder Ethanol, entweder mit gasförmigem Ammoniak oder einem Ammoniaksalz, wie Ammoniumchlorid behandelt, zweckmässig bei einer Temperatur bis 80°C. Alternativ kann das abfiltrierte Zwischenprodukt gleich mit gasförmigem Ammoniak oder einem Ammoniaksalz in Methanol oder Ethanol behandelt werden. Zur Umwandlung einer Cyangruppe in eine N-Hydroxyamidinogruppe wird das Ausgangsnitril in einem Lösungsmittel, wie DMF, gelöst und zu einer Reaktionslösung aus einer anorganischen Base, wie Natriumhydrid oder Natriumhydroxid, und Hydroxylamin oder einem Salz davon mit einer anorganischen Säure, wie Hydroxylamin-hydrochlorid gegeben, zweckmässig bei einer Temperatur unter 0°C. Zur Umwandlung der Nitrogruppe in eine Guanidinogruppe wird das Ausgangsmaterial in einem Lösungsmittel, wie Ethanol oder Essigsäure, gelöst und unter einer Wasserstoffatmosphäre in Gegenwart eines Katalysators wie Pd/C hydriert. Das so erhaltene Amin wird in einem Lösungsmittel, wie DMF oder Methanol, in Gegenwart einer Base, wie Triethylamin, mit Formamidinsulfonsäure oder 3,5-Dimethyl-1-pyrazolylformamidiniumnitrat, zweckmässig bei einer Temperatur bis 80°C umgesetzt.

Als funktionelle Abwandlungen c) von in einer Verbindung der Formel I enthaltenen reaktionsfähigen Gruppen kommen die folgenden in Frage:

Eine Verbindung der Formel I, worin $R^2$ H ist, kann man mit einem die Gruppe $R^2$ ($\neq$ H) abgebenden Mittel umsetzen. So kann eine solche Verbindung in Ameisensäure mit einer Formalinlösung bei erhöhter Temperatur methyliert oder mit einem nieder-Alkanoylchlorid in einem Lösungsmittel, wie Pyridin oder Dichlormethan, in Gegenwart einer organischen Base, wie Pyridin, Triethylamin, Diazabicyclooctan oder Ethylmorpholin, niederalkanoyliert werden.

Eine Verbindung der Formel I, in der eine vorhandene Gruppe G ein Schwefelatom enthält, kann man mit einem Oxidationsmittel, wie einer Persäure, z.B. 3-Chlorperbenzoesäure, in einem Lösungsmittel, wie Methanol, Ethanol, Dichlormethan oder Dioxan, zunächst in das entsprechende Sulfoxid und letzteres gewünschtenfalls in das Sulfon überführen.

Die Verbindungen der Formeln II, III und IV, sowie die in der Verfahrensvariante b) verwendeten eine Gruppe CN oder $NO_2$ enthaltenden Ausgangsmaterialien sind bekannt oder können in Analogie zu den bekannten Verbindungen hergestellt werden. So kann man die Ausgangsnitrile durch Umsetzung eines entsprechenden Bromids in einem Lösungsmittel, wie DMF, mit Kupfercyanid oder einem Alkalicyanid und einem Kupfersalz bei einer Temperatur zwischen 100 und 160°C herstellen.

Zudem enthalten manche der nachfolgenden Beispiele detaillierte Angaben betreffend die Herstellung bestimmter Ausgangsmaterialien und Zwischenprodukten.

Die Verbindungen der Formel I, ihre Solvate und ihre Salze hemmen sowohl die durch Thrombin und Faktor Xa induzierte Plättchenaggregation als auch die durch Thrombin induzierte Gerinnung von Fibrinogen im Blutplasma. Die besagten Verbindungen beeinflussen sowohl die Plättchen induzierte als auch die plasmatische Blutgerinnung. Sie verhindern damit insbesondere die Entstehung von Gerinnungsthromben aber auch von plättchenreichen Thromben und können bei der Bekämpfung bzw. Verhütung von Krankheiten, wie Thrombose, Apoplexie, Herzinfarkt, Entzündung und Arteriosklerose verwendet werden. Ferner haben diese Verbindungen einen Effekt auf Tumorzellen und verhindern die Bildung von Metastasen. Somit können sie auch als Antitumor-Mittel eingesetzt werden.

Der Nachweis der Hemmung der amidolytischen Aktivität von Thrombin durch die obigen Verbindungen wurde wie nachstehend beschrieben erbracht.

Die Messungen wurden auf Mikrotiterplatten bei Raumtemperatur durchgeführt. Dafür wurden pro Vertiefung der Platte 150 µl Puffer (50 mM Tris, 100 mM NaCl, 0,1 % Polyaethylenglykol; pH 7,8) mit 50 µl der in DMSO gelösten und im Puffer verdünnten Hemmsubstanz vermischt und 25 µl humanes Thrombin (0,5 nM Endkonz.) zugegeben. Nach 10 Minuten Inkubation wurde die Reaktion durch Zugabe von chromogenem Substrat S-2238 (H-D-Phe-Pip-Arg-Paranitroanilin von Kabivitrum; 10 oder 50 µM Endkonz.) gestartet und die Hydrolyse des Substrates spektrophotometrisch auf einem kinetischen Mikrotiter-Plattenleser während 5 Minuten verfolgt. Nach graphischer Darstellung der Hemmkurven wurden die von Substrat- und Enzymkonzentration unabhängigen Hemmwerte $K_i$ nach der in Biochem. J. 55, 1953, 170-171 beschriebenen Methode bestimmt. Die Resultate sind aus folgender Tabelle ersichtlich:

| Produkt von Beispiel | $K_i$(µM) |
|---|---|
| 4.A)a) | 0,67 |

(fortgesetzt)

| Produkt von Beispiel | $K_i$(μM) |
|---|---|
| 4.A)f) | 0,22 |
| 4.A)g) | 0,09 |
| 7.A)g) | 0,35 |
| 7.A)i) | 0,44 |
| 7.A)o) | 0,67 |
| 9 | 0,11 |

Der Nachweis der Hemmung der amidolytischen Aktivität von Faktor Xa wurde analog zu der von Thrombin, jedoch mit dem chromogenen Substrat S-2222 (Bz-Ile-Glu-Gly-Arg-Paranitroanilin von Kabivitrum; 167 μM Endkonz.) erbracht:

| Produkt von Beispiel | Ki (μM) |
|---|---|
| 19 | 0.076 |
| 18 | 0.098 |

Wie eingangs erwähnt, sind Arzneimittel enthaltend eine Verbindung der Formel I, ein Solvat oder Salz davon, ebenfalls Gegenstand der vorliegenden Erfindung, weiterhin auch ein Verfahren zur Herstellung derartiger Arzneimittel, welches dadurch gekennzeichnet ist, dass man eine oder mehrere solcher Verbindungen, Solvate oder Salze und gewünschtenfalls andere therapeutisch wertvolle Stoffe in galenische Darreichungsform bringt. Diese Arzneimittel können oral, z.B. in Form von Dragées, Hart- und Weichgelatinekapseln, Lösungen, Emulsionen oder Suspensionen, oder rektal, z. B. in Form von Suppositorien, oder als Spray verabreicht werden. Die Verabreichung kann aber auch parenteral, z. B. in Form von Injektionslösungen, erfolgen.

Zur Herstellung von Tabletten, Lacktabletten, Dragées und Hartgelatinekapseln kann der Wirkstoff mit pharmazeutisch inerten, anorganischen oder organischen Excipientien vermischt werden. Als solche Excipientien kann man für Tabletten, Lacktabletten, Dragées und Hartgelatinekapseln z.B. Lactose, Maisstärke oder Derivate davon, Talk, Stearinsäure oder deren Salze, verwenden. Für Weichgelatinekapseln eignen sich als Excipientien z.B. vegetabile Oele, Wachse, Fette, halbfeste und flüssige Polyole; je nach Beschaffenheit des Wirkstoffs sind jedoch bei Weichgelatinekapseln überhaupt keine Excipientien erforderlich. Zur Herstellung der Lösungen und Sirupe eignen sich als Excipientien z.B. Wasser, Polyole, Saccharose, Invertzucker und Glucose, für Injektionslösungen eignen sich z.B. Wasser, Alkohole, Polyole, Glycerin und vegetabile Oele, und für Suppositorien eignen sich natürliche oder gehärtete Oele, Wachse, Fette, halbflüssige oder flüssige Polyole. Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten.

Für die Bekämpfung bzw. Verhütung der weiter oben genannten Krankheiten kann die Dosierung des Wirkstoffes innerhalb weiter Grenzen variieren und ist natürlich in jedem Einzelfall den individuellen Gegebenheiten anzupassen. Im allgemeinen dürfte bei oraler oder parenteraler, z.B. intravenöser oder subkutaner Verabreichung eine Dosis von etwa 0,1 bis 20 mg/kg, vorzugsweise von etwa 0,5 bis 4 mg/kg, pro Tag für den Erwachsenen angemessen sein, wobei aber die soeben angegebene obere Grenze auch über- oder unterschritten werden kann, wenn sich dies als angezeigt erweisen sollte.

**Beispiel 1**

871 mg (5 mmol) Arginin, 530 mg (5 mmol) Benzaldehyd, 1.122 g (6 mmol) N-Benzylmaleinimid und 25 Tropfen Essigsäure werden in 25 ml Methanol für 60 Stunden unter Rückfluss erhitzt. Nach dem Abkühlen wird der farblose Niederschlag abgesaugt und mit Ether gewaschen. Man isoliert 1.367 g (1RS,3SR,3aRS,6aSR)-5-Benzyl-1-(3-guanidino-propyl)-4,6-dioxooctahydro-3-phenyl-pyrrolo[3,4-c]pyrrol-1-carbonsäure Acetat (54 %) als farbloses Pulver. Fp.: ab 195 °C Zersetzung. ISP-MS: 450.4 ($[M+H]^+$, 100).

**Beispiel 2**

806 mg (1RS,3aSR,6aRS)-4-(5-Benzyl-2,3,3-trimethyl-4,6-dioxooctahydro-pyrrolo[3,4-c]pyrrol-1-ylmethyl)-benzonitril werden in 5 ml trockenem Chloroform und 1 ml Methanol gelöst. Bei 0 °C wird für 10 Minuten trockenes

HCl eingeleitet. Danach wird das Reaktionsgemisch für 2 Tage bei 4 °C stehen gelassen. Durch Zugabe von Diethylether wird das Hydrochlorid des Imidoethers ausgefällt. Dieser Feststoff wird getrocknet und mit 8 ml 5 %iger $NaHCO_3$-Lösung und 20 ml Chloroform versetzt. Es wird schnell ausgeschüttelt und die wässrige Phase noch zweimal mit Chloroform extrahiert. Die organische Phase wird mit $Na_2SO_4$ getrocknet und das Lösungsmittel entfernt. Der Rückstand wird in 7 ml Methanol gelöst, 150 mg $NH_4Cl$ in 1.5 ml $H_2O$ zugegeben und für 3.5 h bei 65 °C gerührt. Nach dem Abkühlen wird das Ammoniumchlorid mit Aceton ausgefällt und abfiltriert. Das Lösungsmittel wird entfernt, der Rückstand in Ethanol gelöst und mit Diethylether langsam das Amidin ausgefällt. Man isoliert 483 mg (1RS,3aSR,6aRS)-4-(5-Benzyl-2,3,3-trimethyl-4,6-dioxooctahydro-pyrrolo[3,4-c]pyrrol-1-ylmethyl)-benzimidamid Acetat als gelbliches Pulver. Fp.: 205-207 °C. ISP-MS: 405.4 ($[M+H]^+$, 100).

Herstellung des Ausgangsmaterials:

**2.a)** 8.83 g (36.2 mmol) 4-Bromphenylalanin, 4.74 g (81.7 mmol) Aceton und 7.00 g (37.43 mmol) N-Benzylmaleinimid werden in 140 ml Toluol unter Zugabe von Molekularsieb A4 für 68 h unter Rückfluss erhitzt. Das Lösungsmittel wird im Vakuum entfernt und der Rückstand chromatographisch getrennt (Hexan/Essigsäureethylester 1:1 + 1 % Triethylamin). Als Hauptprodukt werden 3.88 g (3aRS,4RS,6aSR)-2-Benzyl-4-(4-bromobenzyl)-6,6-dimethyl-tetrahydro-pyrrolo[3,4-c]pyrrol-1,3-dion als farbloses Öl erhalten, das nach Zugabe von wenig Methanol über Nacht zu farblosen Nadeln kristallisiert. Fp.: 142-143 °C. FAB-MS: 427 ($[M+H]^+$, 58); 257 (66); 240 (8); 200 (6); 91 (58). Als Nebenprodukt erhält man 2.12 g (3aRS,4SR,6aSR)-2-Benzyl-4-(4-bromobenzyl)-6,6-dimethyl-tetrahydro-pyrrolo[3,4-c]pyrrol-1,3-dion als farbloses Öl. FAB-MS: 427 ($[M+H]^+$,88); 257 (89); 91 (100).

**2.b)** Ein Gemisch von 3.88 g (9.1 mmol) (3aRS,4RS,6aSR)-2-Benzyl-4-(4-bromo-benzyl)-6,6-dimethyl-tetrahydro-pyrrolo[3,4-c]pyrrol-1,3-dion, 85%iger Ameisensäure (4.9 g, 90 mmol) und 35%-iger Formalin-Lösung (2.6 g, 30 mmol) werden für 5.5 h auf 100 °C erhitzt. Nach dem Abkühlen gibt man 1N NaOH (50 ml) zu und extrahiert das Gemisch viermal mit Dichlormethan. Die organische Phase wird eingeengt und der Rückstand chromatographisch getrennt (Hexan/Essigsäureethylester 3:1 + 1 % Triethylamin). Man isoliert 3.45 g (3aRS,4RS,6aSR)-2-Benzyl-4-(4-bromobenzyl)-5,6,6-trimethyl-tetrahydro-pyrrolo[3,4-c]pyrrol-1,3-dion als gelbliches Öl. FAB-MS: 441 ($[M+H]^+$, 40); 271 (100); 212 (5); 169 (11); 136 (14); 110 (26); 91 (42).

**2.c)** 3.45 g (7.82 mmol) (3aRS,4RS,6aSR)-2-Benzyl-4-(4-bromobenzyl)-5,6,6-trimethyl-tetrahydro-pyrrolo[3,4-c]pyrrol-1,3-dion und 3.52 g (39.1 mmol) Kupfer(I)cyanid werden unter Argon in 150 ml DMF suspendiert und für 56 h unter Rückfluss erhitzt. Nach dem Abkühlen werden ca 100 ml DMF entfernt, danach werden 150 ml Dichlormethan und 60 ml konzentrierte wässrige Ammoniaklösung zugegeben. Das Gemisch wird für einige Stunden stark gerührt. Die blaue wässrige Phase wird abgetrennt und die organische Phase noch zweimal mit Ammoniaklösung und einmal mit Wasser gewaschen. Das Lösungsmittel wird entfernt und der Rückstand chromatographisch gereinigt (Hexan/Essigsäureethylester 1:1 + 1 % Triethylamin). Man isoliert 1.91 g (1RS,3aSR,6aRS)-4-(5-Benzyl-2,3,3-trimethyl-4,6-dioxo-octahydro-pyrrolo[3,4-c]pyrrol-1-ylmethyl)-benzonitril als gelbliches Öl. FAB-MS: 775 ($[2M+H]^+$, 1); 663 (2); 388 ($[M+H]^+$, 58); 271 (100); 91 (42).

## Beispiel 3

2.19 g (31.56 mmol) Hydroxylamin-Hydrochlorid werden in 8 ml DMF suspendiert und auf 0 °C gekühlt. 0.79 g (26.3 mmol) 80%-iges Natriumhydrid werden langsam zugegeben. 1.02 g (2.63 mmol) (1RS,3aSR,6aRS)-4-(5-Benzyl-2,3,3-trimethyl-4,6-dioxo-octahydro-pyrrolo[3,4-c]pyrrol-1-ylmethyl)-benzonitril werden in DMF gelöst und zum Reaktionsgemisch gegeben. Die Kühlung wird danach nicht mehr erneuert. Es wird 2 Tage bei Raumtemperatur gerührt. Danach wird filtriert und das DMF am Hochvakuum entfernt. Das Gemisch wird chromatographisch gereinigt (RP18, Gradient von Wasser auf Methanol). Man isoliert 1.08 g (97 %) (3aRS,4RS,6aSR)-4-[4-(Amino-hydroxyimino-methyl)-benzyl]-2-benzyl-5,6,6-trimethyl-tetrahydropyrrolo[3,4-c]pyrrol-1,3-dion als farbloses Pulver. Fp.: 189-192 °C. ISP-MS: 421.5 ($[M+H]^+$, 100).

## Beispiel 4

Analog Beispiel 2 stellt man folgende Verbindungen her:

**4.A)a)** (1RS,3SR,3aRS,6aSR)-4-(5-Benzyl-2,3-dimethyl-4,6-dioxo-octahydropyrrolo[3,4-c]pyrrol-1-yl)-benzimidamid Hydrochlorid. Fp.: 201-203 °C. ISP-MS: 377.4 ($[M+H]^+$,100).

**4.A)b)** (1RS,3SR,3aRS,6aSR)-4-(5-Benzyl-3-methyl-4,6-dioxo-octahydropyrrolo[3,4-c]pyrrol-1-yl)-benzimidamid Hydrochlorid. Fp.: 188-191 °C. ISP-MS: 363.4 ([M+H]$^+$, 100).

**4.A)c)** (1RS,3SR,3aRS,6aSR)-4-(2-Acetyl-5-benzyl-3-methyl-4,6-dioxooctahydro-pyrrolo[3,4-c]pyrrol-1-yl)-benzimidamid Hydrochlorid. Fp.: 204-208 °C (Aceton). ISP-MS: 405 ([M+H]$^+$, 100).

**4.A)d)** (1RS,3SR,3aRS,6aSR)-4-(5-Butyl-2,3-dimethyl-4,6-dioxo-octahydropyrrolo[3,4-c]pyrrol-1-yl)-benzimidamid Hydrochlorid. Fp.: 171-176 °C. ISP-MS: 343.4 ([M+H]$^+$, 100).

**4.A)e)** (1RS,3SR,3aRS,6aSR)-4-(2-Acetyl-5-butyl-3-methyl-4,6-dioxooctahydro-pyrrolo[3,4-c]pyrrol-1-yl)-benzimidamid Hydrochlorid. Fp.: 203-206 °C. ISP-MS: 371.4 ([M+H]$^+$, 100).

**4.A)f)** (3aRS,4SR,8aRS,8bSR)-4-(2-Benzyl-1,3-dioxo-decahydro-pyrrolo[3,4-a]pyrrolizin-4-yl)-benzimidamid Hydrochlorid. Fp.: 202-205 °C. ISP-MS: 389.4 ([M+H]$^+$, 100); 309.4 (20); 195.4 (58); 158.2 (40).

**4.A)g)** (3aRS,4SR,8aRS,8bSR)-4-{2-(Benzo[1,3]dioxol-5-ylmethyl)-1,3-dioxodecahydro-pyrrolo[3,4-a]pyrrolizin-4-yl}-benzimidamid Hydrochlorid. Fp.: 177-181 °C. ISP-MS: 433.4 ([M+H]$^+$, 100).

Herstellung der Ausgangsstoffe:

**4.B)a)1)** Aus N-Benzylmaleinimid, D,L-Alanin und 4-Brombenzaldehyd wird nach Beispiel 2.a) in analoger Weise (3aRS,4SR,6RS,6aSR)-2-Benzyl-4-(4-bromo-phenyl)-6-methyl-tetrahydro-pyrrolo[3,4-c]pyrrol-1,3-dion hergestellt. Man isoliert 47% als farblose Kristalle. Fp.: 170-172 °C. FAB-MS: 799 ([2M+3H]$^+$, 10); 552 (3); (399 ([M+H]$^+$, 100); 238 (9); 211 (29); 91 (61).

**4.B)a)2)** Aus (3aRS,4SR,6RS,6aSR)-2-Benzyl-4-(4-bromo-phenyl)-6-methyltetrahydro-pyrrolo[3,4-c]pyrrol-1,3-dion wird nach Beispiel 2.b) in analoger Weise (3aRS,4SR,6RS,6aSR)-2-Benzyl-4-(4-bromo-phenyl)-5,6-dimethyltetrahydro-pyrrolo[3,4-c]pyrrol-1,3-dion hergestellt. Man isoliert 96 % als farblose Kristalle. Fp.: 166-168 °C. FAB-MS: 827 ([2M+3H]$^+$, 3); 566 (2); 413 (M+H]$^+$,100); 397 (31); 250 (8); 225 (12); 91 (46).

**4.B)a)3)** 400 mg (0.96 mmol) (3aRS,4SR,6RS,6aSR)-2-Benzyl-4-(4-bromophenyl)-5,6-dimethyl-tetrahydro-pyrrolo[3,4-c]pyrrol-1,3-dion und 360 mg (4.02 mmol) Kupfer(I)cyanid werden in 25 ml DMF suspendiert und unter Argon für 18 h unter Rückfluss erhitzt. Nach dem Abkühlen werden ca 15 ml DMF entfernt, danach werden 30 ml Dichlormethan und 20 ml konzentrierte wässrige Ammoniaklösung zugegeben. Das Gemisch wird für einige Stunden stark gerührt. Die blaue wässrige Phase wird abgetrennt und die organische Phase noch zweimal mit Ammoniaklösung und einmal mit Wasser gewaschen. Das Lösungsmittel wird entfernt und der Rückstand chromatographisch gereinigt (Hexan/Essigsäureethylester 2:1 + 1 % Triethylamin). Man isoliert 293 mg (1RS,3SR,3aRS,6aSR)-4-(5-Benzyl-2,3-dimethyl-4,6-dioxo-octahydro-pyrrolo[3,4-c]pyrrol-1-yl)-benzonitril (85 %) als farblose Kristalle. Fp.: 188-190 °C (Essigsäureethylester). FAB-MS: 719 ([2M+H]$^+$, 3); 512 (3); 360 ([M+H]$^+$,100); 344 (41); 289 (15); 107 (37); 91 (56).

**4.B)b)** Aus 4-Cyano-benzaldehyd, D,L-Alanin und N-Benzylmaleinimid wird nach Beispiel 2.a) in analoger Weise (1RS,3SR,3aRS,6aSR)-4-(5-Benzyl-3-methyl-4,6-dioxo-octahydro-pyrrolo[3,4-c]pyrrol-1-yl)-benzonitril hergestellt. Fp.: 169-170 °C. ISP-MS: 346.3 ([M+H]$^+$, 45); 279.3 (26); 267.2 (18); 199.3 (13); 171.4 (23); 158.2 (28); 149.2 (25); 134.1 (17).

**4.B)c)** Eine Lösung von 3.46 g (10 mmol) (1RS,3SR,3aRS,6aSR)-4-(5-Benzyl-3-methyl-4,6-dioxo-octahydro-pyrrolo[3,4-c]pyrrol-1-yl)-benzonitril in Acetonitril (10 ml) wird mit Pyridin (20 mmol) versetzt. Unter Eiskühlung wird langsam Acetanhydrid (20 mmol) zugetropft. Die Kühlung wird entfernt und das Reaktionsgemisch noch für 1.5 h rühren gelassen. Danach wird das Reaktionsgemisch in gesättigte NaCl-Lösung gegossen und mit Dichlormethan extrahiert. Das Lösungsmittel wird entfernt und der Rückstand chromatographisch getrennt (Hexan : Essigsäureethylester = 1:1). Man isoliert (1RS,3SR,3aRS,6aSR)-4-(2-Acetyl-5-benzyl-3-methyl-4,6-dioxo-octahydro-pyrrolo [3,4-c]pyrrol-1-yl)-benzonitril 83 % als farblosen Feststoff. Fp.: 107-109 °C. EI-MS: 387 ([M]$^+$, 72); 344 (35); 320 (28); 254 (18); 183 (16); 169 (17); 158 (53); 91 (86); 43 (100).

**4.B)d)1)** Nach Beispiel 2.b) wird in analoger Weise aus N-Butylmaleinimid, D,L-Alanin und 4-Cyano-benzaldehyd

(1RS,3SR,3aRS,6aSR)-4-(5-Butyl-3-methyl-4,6-dioxo-octahydro-pyrrolo[3,4-c]pyrrol-1-yl)-benzonitril hergestellt. Man isoliert mit 65 % Ausbeute ein gelbliches Öl. EI-MS: 311 ($M^+$, 12); 296 (9); 183 (8); 169 (13); 158 (100); 143 (5); 115 (4); 82 (5).

**4.B)d)2)** Ein Gemisch aus 2.84 g (1RS,3SR,3aRS,6aSR)-4-(5-Butyl-3-methyl-4,6-dioxo-octahydro-pyrrolo[3,4-c]pyrrol-1-yl)-benzonitril, 85%iger Ameisensäure (4.9 g, 90 mmol) und 35%iger Formalin-Lösung (2.6 g, 30 mmol) werden für 5.5 h auf 100 °C erhitzt. Nach dem Abkühlen gibt man 1N NaOH (50 ml) zu und extrahiert das Gemisch viermal mit Dichlormethan. Die organische Phase wird eingeengt und der Rückstand chromatographisch getrennt (Hexan/Essigsäureethylester 3:1 + 1 % Triethylamin). Man isoliert 2.52 g (1RS,3SR,3aRS,6aSR)-4-(5-Butyl-2,3-dimethyl-4,6-dioxooctahydro-pyrrolo[3,4-c]pyrrol-1-yl)-benzonitril (85 %) als farbloses Öl. EI-MS: 325 ($M^+$, 8); 310 (95); 223 (10); 183 (24); 171 (40); 157 (10); 143 (9); 70 (15); 56 (17); 43 (100).

**4.B)e)** Nach Beispiel 4.B)c) wird in analoger Weise (1RS,3SR,3aRS,6aSR)-4-(2-Acetyl-5-butyl-3-methyl-4,6-dioxo-octahydro-pyrrolo[3,4-c]pyrrol-1-yl)-benzonitril hergestellt. Man isoliert 2.71 g (77 %) als farblosen Feststoff. Fp.: 140-141 °C (Essigsäureethylester). ISP-MS: 354.3 ($[M+H]^+$, 100); 158.1 (30).

**4.B)f)** Ein Gemisch aus L-Prolin (20 mmol), 4-Cyano-benzaldehyd ( 20 mmol) und N-Benzylmaleinimid (20 mmol) wird in DMF (20 ml) für 5 h auf 80 °C erhitzt. Das Lösungsmittel wird im Hochvakuum entfernt und der Rückstand chromatographisch über Kieselgel getrennt (Eluent Hexan: Essigsäureethylester = 1:1 + 1% Triethylamin ). Man isoliert in 32 % Ausbeute (3aRS,4SR,8aRS,8bSR)-4-(2-Benzyl-1,3-dioxo-decahydro-pyrrolo[3,4-a]pyrrolizin-4-yl)-benzonitril als farblose Kristalle. Fp.: 191-193 °C (Methanol). EI-MS: 371 ($[M]^+$, 7); 184 (100); 156 (11); 91 (12)

**4.B)g)** Nach Beispiel 4.B)f) wird aus L-Prolin, N-Piperonylmaleinimid und 4-Cyano-benzaldehyd in analoger Weise ein diastereomeres Gemisch von 4-(Decahydro-2-piperonyl-1,3-dioxo-pyrrolo(3,4-a]pyrrolizin-4-yl)-benzonitril hergestellt, das durch Chromatographie an Kieselgel (Eluent Hexan : Essigsäureethylester = 1:1 + 1% Triethylamin) getrennt wird. Man isoliert in 37 % Ausbeute (3aRS,4SR,8aRS,8bSR)-4-{2-(Benzo[1,3]dioxol-5-ylmethyl)-1,3-dioxo-decahydro-pyrrolo[3,4-a]pyrrolizin-4-yl}-benzonitril als farblose Nadeln. Fp.: 176-179 °C (Methanol). ISP-MS: 416.4 ($[M+H]^+$, 100); 289.4 (15); 277.3 (55); 267.3 (24).

Ebenso isoliert man 50 % (3aRS,4RS,8aSR,8bSR)-4-(2-(Benzo[1,3]dioxol-5-ylmethyl)-1,3-dioxo-decahydro-pyrrolo[3,4-a]pyrrolizin-4-yl}-benzonitrilals gelblichen Schaum. Fp.: 61-63 °C. ISP-MS: 416.4 ($[M+H]^+$, 100).

## Beispiel 5

841 mg (12.12 mmol) Hydroxylamin-Hydrochlorid werden in 4 ml DMF suspendiert und auf 0 °C gekühlt. 303 mg (10.1 mmol) 80%iges Natriumhydrid werden langsam zugegeben. 365 mg (1.01 mmol) (1RS,3SR,3aRS, 6aSR)-4-(5-Benzyl-2,3-dimethyl-4,6-dioxo-octahydro-pyrrolo[3,4-c]pyrrol-1-yl)-benzonitril werden in DMF gelöst und zum Reaktionsgemisch gegeben. Die Kühlung wird danach nicht mehr erneuert. Es wird 5 Tage bei Raumtemperatur rühren gelassen. Danach wird filtriert und das Lösungsmittel am Hochvakuum entfernt. Das Gemisch wird chromatographisch gereinigt (RP18, Gradient von Wasser auf Methanol). Man isoliert 191 mg (48 %) (3aRS,4SR,6RS,6aSR)-4-[4-(Amino-hydroxyimino-methyl)-phenyl]-2-benzyl-5,6-dimethyl-tetrahydro-pyrrolo[3,4-c]pyrrol-1,3-dion als farbloses Pulver. Fp.: 194-196 °C. ISP-MS: 393 ($[M+H]^+$, 100); 303 (20); 219 (10).

## Beispiel 6

800 mg (1RS,3SR,3aRS,6aSR)-4-(5-Butyl-3-methyl-4,6-dioxo-octahydropyrrolo[3,4-c]pyrrol-1-yl)-benzonitril werden in 5 ml trockenem Chloroform und 1 ml Methanol gelöst. Bei 0 °C wird für 10 Minuten trockenes HCl eingeleitet. Danach wird das Reaktionsgemisch für 2 Tage bei 4 °C stehen gelassen. Durch Zugabe von Diethylether wird das Hydrochlorid des Imidoethers ausgefällt. Dieser Feststoff wird mit einer gesättigten Ammoniaklösung in Methanol versetzt und 2 Stunden refluxiert. Danach wird das Lösungsmittel entfernt, und der Rückstand an RP-18 Kieselgel chromatographiert (Gradient von Wasser auf Methanol). Man isoliert in 55 % Ausbeute (1RS,3SR,3aRS,6aSR)-4-(5-Butyl-3-methyl-4,6-dioxo-octahydropyrrolo[3,4-c]pyrrol-1-yl)-benzimidamid Hydrochlorid als farblosen Feststoff. Fp.: ab 180 °C Zers. ISP-MS: 329.4 ($[M+H]^+$, 100).

## Beispiel 7

Analog Beispiel 6 stellt man folgende Verbindungen her:

**7.A)a)** (1RS,3SR,3aRS,6aSR)-4-(5-Benzyl-3-isobutyl-4,6-dioxo-octahydropyrrolo[3,4-c]pyrrol-1-yl)-benzimidamid Hydrochlorid. Farbloser Schaum. Fp.: 152-157 °C. ISP-MS: 405.4 ($[M+H]^+$, 100); 315.4 (26).

**7.A)b)** (1RS,3SR,3aRS,6aSR)-4-(5-Benzyl-3-isobutyl-2-methyl-4,6-dioxooctahydro-pyrrolo[3,4-c]pyrrol-1-yl)-benzimidamid Hydrochlorid. Farbloser Schaum. Fp.: 157-162 °C. ISP-MS: 419.5 ($[M+H]^+$, 100).

**7.A)c)** (3aRS,4SR,8aRS,8bSR)-4-(2-Butyl-1,3-dioxo-decahydro-pyrrolo[3,4-a]pyrrolizin-4-yl)-benzimidamid Hydrochlorid. Öl. ISP-MS: 355.4 ($[M+H]^+$, 100).

**7.A)d)** (3aRS,4RS,9aSR,9bSR)-4-(2-Benzyl-1,3-dioxo-decahydro-pyrrolo[3,4-c]indolizin-4-yl)-benzimidamid Hydrochlorid. Farbloser Schaum. Fp.: > 295 °C. ISP-MS: 403.4 ($[M+H]^+$, 100).

**7.A)e)** (3aRS,4SR,9aRS,9bSR)-4-(2-Benzyl-1,3-dioxo-decahydro-pyrrolo[3,4-c]indolizin-4-yl)-benzimidamid Hydrochlorid. Farbloser Schaum. Fp.: 248-250 °C. ISP-MS: 403.4 ($[M+H]^+$, 100).

**7.A)f)** (3aRS,4RS,8aSR,8bSR)-4-{2-(Benzo[1,3]dioxol-5-ylmethyl)-1,3-dioxodecahydro-pyrrolo[3,4-a]pyrrolizin-4-yl}-benzirnidamid Hydrochlorid. Farbloser Schaum. Fp.: 195-200 °C. ISP-MS: 433.5 ($[M+H]^+$, 100).

**7.A)g)** (3aRS,4SR,8aRS,8bSR)-4-(2-Cyclohexylmethyl-1,3-dioxo-decahydropyrrolo[3,4-a]pyrrolizin-4-yl)-benzimidamid Hydrochlorid. Gelblicher Feststoff. Fp.: 104-110 °C. ISP-MS: 395.4 ($[M+H]^+$, 100).

**7.A)h)** (5RS,5aRS,8aSR,8bRS)-4-{7-(Benzo[1,3]dioxol-5-ylmethyl)-6,8-dioxooctahydro-pyrrolo[3',4':3,4] pyrrolo [1,2-c] thiazol-5-yl}-benzimidamid Hydrochlorid. Farbloser Schaum. Fp.: 182-188 °C. ISP-MS: 451 ($[M+H]^+$, 100).

**7.A)i)** (5RS,5aSR,8aRS,8bRS)-4-{7-(Benzo[1,3]dioxol-5-ylmethyl)-6,8-dioxooctahydro-pyrrolo [3',4':3,4]pyrrolo [1,2-c] thiazol-5-yl}-benzimidamid Hydrochlorid. Farbloser Feststoff. Fp.: 226-229 °C, ab 245 °C Zers. ISP-MS: 451 ($[M+H]^+$, 100).

**7.A)j)** (1RS,3RS,3aSR,6aRS)-{5-(Benzo[1,3]dioxol-5-ylmethyl)-3-(4-carbamimidoyl-phenyl)-4,6-dioxo-octahydro-pyrrolo[3,4-c]pyrrol-1-yl}methansulfinsäure. Farbloser Feststoff. Fp.: 253-256 °C. ISP-MS: 471.3 ($[M+H]^+$, 100).

**7.A)k)** (1RS,3RS,3aRS,6aSR)-{5-(Benzo[1,3]dioxol-5-ylmethyl)-3-(4-carbamimidoyl-phenyl)-4,6-dioxo-octahydro-pyrrolo[3,4-c]pyrrol-1-yl}methansulfinsäure. Farbloser Feststoff. Fp.: ab 230 °C Zers. ISP-MS: 471 ($[M+H]^+$, 100).

**7.A)l)** (3aS,4R,8S,8aS,8bR)-4-{2-{Benzo[1,3]dioxol-5-ylmethyl)-8-methyl-1,3-dioxo-decahydro-pyrrolo[3,4-a]pyrrolizin-4-yl}-benzimidamid Hydrochlorid. Farbloser Feststoff. Fp.: ab 220 °C Zers. ISP-MS: 447.2 ($[M+H]^+$, 100).

**7.A)m)** (1RS,3SR,3aRS,6aSR)-3-(4-Carbamimidoyl-phenyl)-1-(2-methylsulfanyl-ethyl)-4,6-dioxo-5-phenyl-octahydro-pyrrolo[3,4-c]pyrrol-1-carbonsäure. Farbloser Feststoff. ISP-MS: 454 ($[M+H]^+$, 100).

**7.A)n)** (3aR,4R,7R,8aS,8bS)-4-(2-Benzyl-7-hydroxy-1,3-dioxo-decahydropyrrolo[3,4-a]pyrrolizin-4-yl)-benzimidamid Hydrochlorid. Farbloser Feststoff. ISP-MS: 405.1 ($[M+H]^+$, 100).

**7.A)o)** (3aR,4S,7S,8aR,8bS)-4-(2-Benzyl-7-hydroxy-1,3-dioxo-decahydropyrrolo[3,4-a]pyrrolizin-4-yl)-benzimidamid Hydrochlorid. Farbloser Feststoff. ISP-MS: 405 ($[M+H]^+$, 100).

**7.A)p)** 1:1 Mischung von (R)-2-{(3aS,4S,8aR,8bR)- und -(3aR,4R,8aS,8bS)-4-(4-Carbamimidoyl-phenyl)-1,3-dioxo-decahydro-pyrrolo[3,4-a]pyrrolizin-2-yl}-3-phenyl-propionsäuremethylester Hydrochlorid. Farbloser Feststoff. ISP-MS: 461.5 ($[M+H]^+$, 100).

**7.A)q)** 1:1 Mischung von (R)-2-{(3aR,4S,8aR,8bS)- und -(3aS,4R,8aR.8bR)-4-(4-Carbamimidoyl-phenyl)-1,3-dioxo-decahydro-pyrrolo[3,4-a]pyrrolizin-2-yl}-3-phenyl-propionsäuremethylester Hydrochlorid. Farbloser Feststoff. Fp.: ab 182 °C Zers. ISP-MS: 461.5 ($[M+H]^+$, 100).

**7.A)r)** (1RS,3SR,3aRS,6aSR)-4-{5-(Benzo[1,3]dioxol-5-ylmethyl)-3-hydroxymethyl-4,6-dioxo-octahydro-pyrrolo [3,4-c]pyrrol-1-yl}-benzimidamid Hydrochlorid. Farbloser Feststoff. Fp.: ab 197 °C Zers. ISP-MS: 423.4 ($[M+H]^+$,

100).

**7.A)s)** (1RS,3RS,3aSR,6aRS)-4-{5-(Benzo[1,3]dioxol-5-ylmethyl)-3-hydroxymethyl-4,6-dioxo-octahydro-pyrrolo [3,4-c]pyrrol-1-yl}-benzimidamid Hydrochlorid. Farbloser Feststoff. ISP-MS: 423 ($[M+H]^+$, 100).

**7.A)t)** (1S,2S,5R,5aS,8aR)-{7-Benzyl-5-(4-carbamimidoyl-phenyl)-2-(propen-2-yl)-6,8-dioxo-decahydro-pyrrolo [3,4-a]pyrrolizin-1-yl}-essigsäure. Farbloser Feststoff. ISP-MS: 487 ($[M+H]^+$, 100).

Herstellung der entsprechenden Ausgangsstoffe:

**7.B)a)** Analog Beispiel 4.B)g) stellt man aus 4-Cyano-benzaldehyd, L-Leucin und N-Benzylmaleinimid (1RS,3SR, 3aRS,6aSR)-4-(5-Benzyl-3-isobutyl-4,6-dioxo-octahydro-pyrrolo[3,4-c]pyrrol-1-yl)-benzonitril her. Farblose Prismen. Fp.: 152-153 °C (Methanol). EI-MS: 387 ($[M]^+$, 4); 330 (100); 200 (6); 169 (48); 130 (7); 91 (57)

**7.B)b)** Durch Methylieren von (1RS,3SR,3aRS,6aSR)-4-(5-Benzyl-3-isobutyl-4,6-dioxo-octahydro-pyrrolo[3,4-c] pyrrol-1-yl)-benzonitril nach Beispiel 2.b) erhält man (1RS,3SR,3aRS,6aSR)-4-(5-Benzyl-3-isobutyl-2-methyl-4,6-dioxooctahydro-pyrrolo[3,4-c]pyrrol-1-yl)-benzonitril. Farblose Nadeln. Fp.: 173-174 °C (Methanol). ISP-MS: 402.5 ($[M+H]^+$, 100).

**7.B)c)** Analog Beispiel 4.B)g) stellt man aus 4-Cyano-benzaldehyd, L-Prolin und N-Butylmaleinimid (3aRS,4RS, 8aSR,8bSR)-4-(2-Butyl-decahydro-1,3-dioxo-pyrrolo[3,4-a]pyrrolizin-4-yl)-benzonitril her. Gelbes Öl. $^1$H-NMR (DMSO-D6): 7.74 (d, ArH); 7.49 (d, ArH); 4.22 (d, 4-H).

**7.B)d)** Analog Beispiel 4.B)g) stellt man aus 4-Cyano-benzaldehyd, D,L-Pipecolinsäure und N-Benzylmaleinimid (3aRS,4RS,9aSR,9bSR)-4-(2-Benzyl-1,3-dioxo-decahydro-pyrrolo[3,4-c]indolizin-4-yl)-benzonitril her. Farblose Prismen. Fp.: 153-154 °C (Methanol). ISP-MS: 386.4 ($[M+H]^+$, 100).

**7.B)e)** Durch Chromatographieren des Rohproduktes (Hexan : Essigsäureethylester = 1:1) aus Beispiel 7.B)d) erhält man auch (3aRS,4SR,9aRS,9bSR)-4-(2-Benzyl-1,3-dioxo-decahydro-pyrrolo[3,4-c]indolizin-4-yl)-benzonitril als farblose Prismen. Fp.: 165-167 °C (Methanol). ISP-MS: 386.4 ($[M+H]^+$, 100).

**7.B)f)** siehe Beispiel 4.B)g)

**7.B)g)** Analog Beispiel 4.B)g) stellt man aus 4-Cyano-benzaldehyd, L-Prolin und N-Cyclohexylmethylmaleinimid (3aRS,4SR,8aRS,8bSR)-4-(2-Cyclohexylmethyl-1,3-dioxo-decahydro-pyrrolo[3,4-a]pyrrolizin-4-yl)-benzonitril. her. Farblose Kristalle. Fp.: 119-122 °C (Essigsäure-ethylester/Hexan). ISP-MS: 378.4 ($[M+H]^+$, 100).

**7.B)h)** Analog Beispiel 4.B)g) stellt man aus 4-Cyano-benzaldehyd, L-Thioprolin und N-Piperonylmaleinimid (5RS, 5aRS,8aSR,8bRS)-4-{7-(Benzo[1,3]-dioxol-5-ylmethyl)-6,8-dioxo-octahydro-pyrrolo[3',4':3,4]pyrrolo[1,2-c]thiazol-5-yl}-benzonitril her. Farblose Nadeln. Fp.: 142-143 °C (Methanol). ISP-MS: 434.2 ($[M+H]^+$, 100).

**7.B)i)** Aus dem Rohprodukt von Beispiel 7.B)h) erhält man durch Chromatographie (Hexan : Essigsäureethylester = 1:1) ebenfalls (5RS,5aSR,8aRS, 8bRS)-4-{7-(Benzo[1,3]dioxol-5-ylmethyl)-6,8-dioxo-octahydro-pyrrolo-[3',4': 3,4]pyrrolo[1,2-c]thiazol-5-yl}-benzonitril. Farblose Kristalle. Fp.: 183-184 °C (Methanol). FAB-MS: 472 ($[M+K]^+$, 25); 456 ($[M+Na]^+$, 54); 434 ($[M+H]^+$, 100).

**7.B)j)** 340 mg (5RS,5aSR,8aRS,8bRS)-4-{7-(Benzo[1,3]dioxol-5-ylmethyl)-6,8-dioxo-octahydro-pyrrolo[3',4':3,4] pyrrolo[1,2-c]thiazol-5-yl}-benzonitril werden mit 300 mg 3-Chlorperbenzoesäure in Dichlormethan 4 Stunden bei Raumtemperatur gerührt. Das Rohprodukt (5RS,5aSR,8aRS,8bRS)-4-{7-(Benzo[1,3]dioxol-5-ylmethyl)-2,2,6,8-tetraoxo-octahydro-pyrrolo[3',4':3,4]-pyrrolo[1,2-c]thiazol-5-yl}-benzonitril wird direkt in 7.A)j) als Ausgangsverbindung eingesetzt. ISP-MS: 466.1 ($[M+H]^+$, 100).

**7.B)k)** 340 mg (5RS,5aRS,8aSR,8bRS)-4-{7-(Benzo[1,3]dioxol-5-ylmethyl)-6,8-dioxo-octahydro-pyrrolo[3',4':3,4] pyrrolo [1,2-c]thiazol-5-yl}-benzonitril werden mit 300 mg 3-Chlorperbenzoesäure in Dichlormethan 4 Stunden bei Raumtemperatur gerührt. Das Rohprodukt (5RS,5aRS,8aSR,8bRS)-4-{7-(Benzo[1,3]dioxol-5-ylmethyl)-2,2,6,8-tetraoxo-octahydro-pyrrolo[3',4':3,4]-pyrrolo[1,2-c]thiazol-5-yl}-benzonitril wird direkt in 7.A)k) als Ausgangsverbindung eingesetzt. ISP-MS: 466.1 ($[M+H]^+$, 100).

**7.B)l)** Analog Beispiel 4.B)g) erhält man aus 4-Cyano-benzaldehyd, (2S,3S)-3-Methylprolin und N-Piperonylmaleinimid (3aS,4R,8S,8aS,8bR)-4-{2-(Benzo[1,3]dioxol-5-ylmethyl)-8-methyl-1,3-dioxo-decahydro-pyrrolo [3,4-a]pyrrolizin-4-yl}-benzonitril als gelben Schaum. $^1$H-NMR (DMSO-D6): 7.85 (d, ArH); 7.63 (d, ArH); 4.24 (d, 4-H).

**7.B)m)** Analog Beispiel 1 erhält man aus L-Methionin, N-Phenylmaleinimid und 4-Cyano-benzaldehyd (1RS,3SR, 3aRS,6aSR)-3-(4-Cyanophenyl)-1-(2-methylsulfanyl-ethyl)-4,6-dioxo-5-phenyl-octahydro-pyrrolo[3,4-c]pyrrol-1-carbonsäure einen gelben Schaum, der aus Ethanol umkristallisiert wird. ISP-MS: 434.3 ([M-H]$^-$, 100).

**7.B)n)** Analog Beispiel 4.B)g) stellt man aus 4-Cyano-benzaldehyd, (2S,3R)-Hydroxyprolin und N-Benzylmaleinimid (3aR,4R,7R,8aS,8bS)-4-(2-Benzyl-7-hydroxy-1,3-dioxo-decahydro-pyrrolo[3,4-a]pyrrolizin-4-yl)-benzonitril her. Gelber Schaum. $^1$H-NMR (DMSO-D6): 7.65 (d, ArH); 7.40 (d, ArH); 4.79 (d, 4-H).

**7.B)o)** Analog Beispiel 4.B)g) erhält man aus 4-Cyano-benzaldehyd, (2S,3S)-Hydroxyprolin und N-Benzylmaleinimid (3aR,4S,7S,8aR,8bS)-4-(2-Benzyl-7-hydroxy-1,3-dioxo-decahydro-pyrrolo[3,4-a]pyrrolizin-4-yl)-benzonitril als gelben Schaum. $^1$H-NMR (DMSO-D6): 7.62 (d, ArH); 7.40 (d, ArH); 4.35 (d, 4-H).

**7.B)p)** Aus (2R)-2-(N-Maleinimido)-3-phenyl-propionsäuremethylester, L-Prolin und 4-Cyano-benzaldehyd erhält man analog zu Beispiel 4.B)g) eine 1:1 Mischung aus (R)-2-{(3aS,4S,8aR,8bR)- und -(3aR,4R,8aS,8bS)-4-(4-Cyanophenyl)-1,3-dioxo-decahydro-pyrrolo[3,4-a]pyrrolizin-2-yl}-3-phenylpropionsäuremethylester Hydrochlorid. Gelber Schaum. $^1$H-NMR (DMSO-D6): 7.85 (d, ArH); 7.82 (d, ArH); 7.61 (d, ArH); 7.46 (d, ArH); 3.70 (d, 4-H); 3.68 (d, 4-H).

**7.B)q)** Durch Chromatographie des Rohproduktes aus Beispiel 7.B)p) wird auch eine 1:1 Mischung von (R)-2-{(3aR,4S,8aR,8bS)- und -(3aS,4R,8aR.8bR)-4-(4-Cyano-phenyl)-1,3-dioxo-decahydro-pyrrolo[3,4-a]pyrrolizin-2-yl}-3-phenyl-propionsäuremethylester Hydrochlorid isoliert. $^1$H-NMR (DMSO-D6): 7.74 (d, ArH); 7.52 (d, ArH); 7.60 (d, ArH); 7.32 (d, ArH); 4.19 (d, 4-H); 4.08 (d, 4-H).

**7.B)r)** Aus N-Piperonylmaleinimid, 4-Cyano-benzaldehyd und L-Serin wird analog zu Beispiel 4.B)g) (1RS,3SR, 3aRS,6aSR)-4-{5-(Benzo[1,3]dioxol-5-ylmethyl)-3-hydroxymethyl-4,6-dioxo-octahydro-pyrrolo[3,4-c]pyrrol-1-yl} benzonitril hergestellt. ISP-MS: 406 ([M+H]$^+$, 100).

**7.B)s)** Durch Chromatographie des Rohproduktes aus Beispiel 7.B)s) wird auch (1RS,3RS,3aSR,6aRS)-4-{5-(Benzo[1,3]dioxol-5-ylmethyl)-3-hydroxymethyl-4,6-dioxo-octahydro-pyrrolo[3,4-c]pyrrol-1-yl}-benzonitril isoliert. ISP-MS: 406 ([M+H]$^+$, 100).

**7.B)t)** Aus N-Piperonylmaleinimid, 4-Cyano-benzaldehyd und Kainsäure wird analog zu Beispiel 4.B)g) (1S,2S, 5R,5aS,8aR)-{7-Benzyl-(5-cyano-phenyl)-2-(propen-2-yl)-6,8-dioxo-decahydro-pyrrolo[3,4-a]pyrrolizin-1-yl}-essigsäure hergestellt. Farbloser Feststoff. $^1$H-NMR (DMSO-D6): 7.63 (d, ArH); 7.40 (d, ArH); 4.44 (d, 4-H).

## Beispiel 8

150 mg (5RS,5aSR,8aRS,8bRS)-4-{7-(Benzo[1,3]dioxol-5-ylmethyl)-6,8-dioxo-octahydro-pyrrolo[3',4':3,4] pyrrolo[1,2-c]thiazol-5-yl}-benzimidamid Hydrochlorid (Beispiel 7.A)i))werden in 20 ml Dichlormethan gelöst und mit 170 mg 3-Chlorperbenzoesäure versetzt und bei Raumtemperatur 4 Stunden gerührt. Danach werden 130 mg eines farblosen Feststoffes abfiltriert, der durch Chromatographie an RP-18 mit einem Wasser / Methanol Eluent weiter gereinigt werden kann. Man erhält so 80 mg (5RS,5aSR,8aRS,8bRS)-4-{7-(Benzo[1,3]dioxol-5-ylmethyl)-2,2,6,8-tetraoxo-octahydro-pyrrolo-[3',4':3,4]pyrrolo[1,2-c]thiazol-5-yl}-benzimidamid Hydrochlorid. ISP-MS: 483.4 ([M+H]$^+$, 100).

## Beispiel 9

Ein Gemisch aus L-Prolin (20 mmol), 4-Nitrobenzaldehyd ( 20 mmol) und N-Butylmaleinimid (20 mmol) wird in DMF (20 ml) für 24 h auf 80 °C erhitzt. Das Lösungsmittel wird im Hochvakuum entfernt und der Rückstand chromatographisch über Kieselgel getrennt (Eluent Hexan: Essigsäureethylester = 1:1 + 1% Triethylamin ). Man erhält in 32% Ausbeute (3aRS,4SR,8aRS,8bSR)-2-Butyl-4-(4-nitro-phenyl)-hexahydropyrrolo[3,4-a]pyrrolizin-1,3-dion als gelben Feststoff ($R_f$= 0.35). Dieses Produkt wird in 50 ml Ethanol mit 200 mg 10% Palladium auf Kohle versetzt und unter Wasserstoff innerhalb 8 Stunden reduziert. Nach Abfiltrieren des Katalysators und entfernen des Lösungsmittels im

Vakuum erhält man (3aRS,4SR,8aRS,8bSR)-4-(4-Amino-phenyl)-2-butyl-hexahydro-pyrrolo[3,4-a]pyrrolizin-1,3-dion als hellgelbes Öl ($R_f$= 0.23). 120 mg dieses Rohproduktes wird mit 80 mg 3,5-Dimethyl-pyrazolyl-1-carboximidamidinium Nitrat in 20 ml DMF versetzt und 48 Stunden bei 80 °C erhitzt. Nach dem Entfernen des Lösungsmittels im Vakuum wird das verbleibende Material an einer RP-18 Chromatographiesäule mit einem Wasser / Methanolgradienten getrennt. Man erhält in 12 % Ausbeute (3aRS,4SR,8aRS,8bSR)-*N*-{4-(2-Butyl-1,3-dioxodecahydro-pyrrolo[3,4-a]pyrrolizin-4-yl)-phenyl}-guanidinium Nitrat als hellgelbes Öl. ISP-MS: 483.4 ($[M+H]^+$, 100).

**Beispiel 10**

In Analogie zu Vorschrift 9 werden folgende Verbindungen hergestellt:

**10.a)** aus L-Prolin, 4-Nitrobenzaldehyd und N-Benzylmaleinimid (3aRS,4SR,8aRS,8bSR)-*N*-{4-(2-Benzyl-1,3-dioxo-decahydro-pyrrolo[3,4-a]pyrrolizin-4-yl)-phenyl}-guanidinium Nitrat als hellgelbe Kristalle. ISP-MS: 404.5 ($[M+H]^+$, 100).

**10.b)** aus L-Prolin, 4-Nitrobenzaldehyd und N-Piperonylmaleinimid (3aRS,4SR,8aRS,8bSR)-*N*-[4-{2-(Benzo[1,3]dioxol-5-ylmethyl)-1,3-dioxodecahydro-pyrrolo[3,4-a]pyrrolizin-4-yl}-phenyl]-guanidinium Nitrat als gelbe Kristalle. ISP-MS: 448.3 ($[M+H]^+$, 100).

**Beispiel 11**

In Analogie zu Vorschrift 4.B)f) werden folgende Verbindungen hergestellt:

**11.A)a)** Aus L-Prolin, 2,4-Diamino-quinazolin-6-carbaldehyd und N-Benzylmaleinimid (3aRS,4SR,8aRS,8bSR)-2-Benzyl-4-(2,4-diaminoquinazolin-6-yl)-hexahydro-pyrrolo[3,4-a]pyrrolizin-1,3-dion als hellgelbe Kristalle (ISP-MS: 429.3 ($[M+H]^+$, 100)) und
(3aRS,4RS,8aSR,8bSR)-2-Benzyl-4-(2,4-diamino-quinazolin-6-yl)-hexahydro-pyrrolo[3,4-a]pyrrolizin-1,3-dion als hellgelbe Kristalle (ISP-MS: 429.3 ($[M+H]^+$, 100)).

**11.A)b)** Aus L-Prolin, 2,4-Diamino-quinazolin-7-carbaldehyd und N-Benzylmaleinimid (3aRS,4SR,8aRS,8bSR)-2-Benzyl-4-(2,4-diaminoquinazolin-7-yl)-hexahydro-pyrrolo[3,4-a]pyrrolizin-1,3-dion als hellgelbe Kristalle. ISP-MS: 429.3 ($[M+H]^+$, 100).

Herstellung der Ausgangsstoffe

**11.B)a)** 3,4-Diamino-quinazolin-6-carbaldehyd wird nach E.F. Elslager, J. Clarke, L.M. Werbel, D.F. Worth *J. Med. Chem.* Vol. 15, 827 (1972) hergestellt.

**11.B)b)** 2,4-Diamino-quinazolin-7-carbaldehyd wird in Analogie zu 11.B)a) aus 2-Amino-benzo-1,4-dinitril nach J. Griffiths, B. Roozpeikar *J. Chem. Soc., Perkin Trans.* I 42 (1976) hergestellt.

**Beispiel 12**

5g (1.5 mmol) Fmoc-TGR-Harz nach H. Rink *Tetrahedron Lett.* **28**, 3787 (1987) werden für 30 min mit einer 20 %igen Lösung von Piperidin in DMF geschüttelt. Anschliessend wird das Harz mit DMF gewaschen. Es werden 1.19 g (3 mmol) Fmoc-Arginin, 950 mg (6 mmol) 1-Hydroxy-benzotriazol (HOBT - enthält ca. 20 % Wasser), 570 mg Diisopropylcarbodiimid und DMF zugesetzt und für 1 h geschüttelt. Das Harz wird mit DMF gewaschen und für 30 min mit einer 20 %igen Lösung von Piperidin in DMF geschüttelt. Danach wird mit DMF, Isopropanol, Wasser und Methanol gewaschen. Anschliessend wird das Harz in gleichen Portionen auf 30 Reaktionsgefässe verteilt, dann werden in jeweils 6 dieser Gefässe 0.5 mmol jeweils einer Carbonylverbindung (a. Paraformaldehyd, b. Pivalaldehyd, c. Benzaldehyd, d. Benzophenon, bzw. e. Phenylpropionaldehyd) gegeben, wonach dann jeweils so 0.5 mmol eines Maleinimids (Maleinimid, N-Methyl-, N-Butyl-, N-Cyclohexylmethyl-, N-Phenyl- bzw. N-Benzyl-maleinimid) zugegeben werden, dass jedes Gefäss eine andere Paarung aus je einer Carbonylverbindung und einem Maleinimid enthält. Als Lösungsmittel werden je Gefäss ca. 5 ml eines Gemisches aus 150 ml Methanol und 8 ml Essigsäure zugegeben und die Ansätze für 22 h unter Rückfluss erhitzt. Nach dem Abkühlen wird das Lösungsmittel abgesaugt und das Harz je zweimal mit Dimethylsulfoxid, Isopropanol, Wasser und Isopropanol gewaschen. Das Harz wird dann mit je ca. 5 ml eines Gemisches aus 40 % Trifluoressigsäure, 50 % Dichlormethan, 5 % Wasser und 5 % Isopropanol versetzt und für 2 h geschüttelt. Nach dem Abfiltrieren werden die 30 Filtratlösungen eingeengt. Man erhält so 30 Rohprodukte als gelbe Öle. Für massenspektroskopische und HPLC-Untersuchungen werden ca. je 0.5 mg entnommen und in 100 µl Wasser und 100 µl Acetonitril aufgenommen. Der Rest wird in 0.5 ml DMSO gelöst und mit dem Thrombintest nach R. Lottenberg, J.A. Hall, J.W. Fenton, G.M. Jackson *Thrombosis Research* **28**, 313 (1982) unter Verwendung des chromogenen Substrates S-2238 bei 405 nm Wellenlänge spektrofotometrisch das Rohprodukt mit der höchsten Hemmwirkung ermittelt.

Mit Hilfe von Ionenspray-Massenspektroskopie (ISP-MS) konnte die Anwesenheit der folgenden 3,5-substituierten 1-(3-Guanidino-propyl)-4,6-dioxo-octahydro-pyrrolo[3,4-c]pyrrol-1-carboxamide nachgewiesen werden:

a) 3-*tert.*Butyl-1-(3-guanidino-propyl)-4,6-dioxo-octahydro-pyrrolo-[3,4-c]pyrrol-1-carboxamid;
b ) 3-Phenyl-1-(3-guanidino-propyl)-4,6-dioxo-octahydro-pyrrolo-[3,4-c]pyrrol-1-carboxamid;
c) 3-(2-Phenyl-ethyl)-1-(3-guanidino-propyl)-4,6-dioxo-octahydro-pyrrolo-[3,4-c]pyrrol-1-carboxamid;
d) 5-Methyl-3-(2-phenyl-ethyl)-1-(3-guanidino-propyl)-4,6-dioxo-octahydropyrrolo[3,4-c]pyrrol-1-carboxamid;
e) 5-Butyl-3-(2-phenyl-ethyl)-1-(3-guanidino-propyl)-4,6-dioxo-octahydropyrrolo[3,4-c]pyrrol-1-carboxamid;
f) 5-Cyclohexylmethyl-3-(2-phenyl-ethyl)-1-(3-guanidino-propyl)-4,6-dioxooctahydro-pyrrolo[3,4-c]pyrrol-1-carboxamid;
g) 5-Benzyl-3-(2-phenyl-ethyl)-1-(3-guanidino-propyl)-4,6-dioxo-octahydropyrrolo[3,4-c]pyrrol-1-carboxamid;
h) 5-Phenyl-3-(2-phenyl-ethyl)-1-(3-guanidino-propyl)-4,6-dioxo-octahydropyrrolo[3,4-c]pyrrol-1-carboxamid;
i) 5-Butyl-3,3-diphenyl-1-(3-guanidino-propyl)-4,6-dioxo-octahydro-pyrrolo-[3,4-c]pyrrol-1-carboxamid;
j) 5-Butyl-3-phenyl-1-(3-guanidino-propyl)-4,6-dioxo-octahydro-pyrrolo-[3,4-c]pyrrol-1-carboxamid;
k) 5-Cyclohexylmethyl-3-phenyl-1-(3-guanidino-propyl)-4,6-dioxooctahydro-pyrrolo[3,4-c]pyrrol-1-carboxamid;
l) 5-Benzyl-3-phenyl-1-(3-guanidino-propyl)-4,6-dioxo-octahydro-pyrrolo-[3,4-c]pyrrol-1-carboxamid;
m) 3,5-Diphenyl-1-(3-guanidino-propyl)-4,6-dioxo-octahydro-pyrrolo-[3,4-c]pyrrol-1-carboxamid;
n) 5-Butyl-3-*tert.*butyl-1-(3-guanidino-propyl)-4,6-dioxo-octahydro-pyrrolo-[3,4-c]pyrrol-1-carboxamid;
o) 5-Butyl-1-(3-guanidino-propyl)-4,6-dioxo-octahydro-pyrrolo[3,4-c]pyrrol-1-carboxamid; bzw.
p) 1-(3-Guanidino-propyl)-4,6-dioxo-octahydro-5-phenyl-pyrrolo[3,4-c]-pyrrol-1-carboxamid.

Von den genannten Cycloaddukten zeigte das Rohprodukt, welches 1-(3-Guanidino-propyl)-4,6-dioxo-octahydro-3,5-diphenyl-pyrrolo[3,4-c]pyrrol-1-carboxamid enthielt, die grösste Hemmwirkung im Thrombintest.

## Beispiel 13

Je 80 mg mit einer Fmoc-geschützten Aminosäure (Glycin, L-Alanin, L-Valin, L-Isoleucin, L-Methionin, L-Prolin, entspricht jeweils ca. 0.016 mmol) derivatisiertes TGA-Harz nach E. Bayer, W. Rapp *Chem. Pept. Prot.* **3**, 3 (1986) werden für 30 min in 6 Reaktionsgefässen mit einer 20 %igen Lösung von Piperidin in DMF geschüttelt und danach mit DMF gewaschen. Anschliessend werden die 6 Harzportionen in jeweils 5 Gefässe gleichmässig verteilt, wonach in jedes dieser 30 Gefässe 0.16 mmol 4-Cyano-benzaldehyd, 0.16 mmol Triethylamin und 0.24 mmol Silberacetat gegeben wird. Dazu wird jeweils so 0.16 mmol eines Maleinimids (N-Methyl-, N-Butyl-, N-Cyclohexylmethyl-, N-Phenyl- bzw. N-Benzyl-maleinimid) zugegeben, dass jedes Gefäss eine andere Paarung aus je einem Aminosäureharz und einem Maleinimid enthält. Als Lösungsmittel werden je Gefäss ca. 3 ml Acetonitril gegeben und 1 Woche bei Raumtemperatur geschüttelt. Nach dem Abkühlen wird das Lösungsmittel abgesaugt und das Harz je zweimal mit Acetonitril, Isopropanol, Wasser und Isopropanol gewaschen. Die 30 Harzportionen werden im Hochvakuum 48 Stunden getrocknet und danach bei 0 °C mit 8 ml eines mit trocknem Chlorwasserstoff gesättigtem 5:1 Gemisches aus Chloroform und Methanol versetzt und bei 4 °C 48 Stunden stehen gelassen. Danach wird das Lösungsmittel bei 30°C im Vakuum entfernt. Nach Zugabe von 8 ml einer mit Ammoniak gesättigten Methanollösung wird 3 Stunden bei 65 °C erhitzt. Nach dem Abfiltrieren werden die 30 Filtratlösungen eingeengt. Man erhält so 30 Rohprodukte als farblose bis gelbe Feststoffe. Für massenspektroskopische und HPLC-Untersuchungen werden ca. je 0.5 mg entnommen und in 100 µl Wasser und 100 µl Acetonitril aufgenommen. Der Rest wird in 0.5 ml DMSO gelöst und nach Beispiel 12 das Rohprodukt mit der höchsten Hemmwirkung ermittelt.

Mit Hilfe von Ionenspray-Massenspektroskopie (ISP-MS) konnte die Anwesenheit der folgenden 1,5-substituierten 3-(4-Carbamimidoyl-phenyl)-4,6-dioxo-octahydro-pyrrolo[3,4-c]pyrrol-1-carbonsäuren nachgewiesen werden:

a) 5-Methyl-3-(4-carbamimidoyl-phenyl)-4,6-dioxo-octahydro-pyrrolo-[3,4-c]pyrrol-1-carbonsäure;
b) 1,5-Dimethyl-3-(4-carbamimidoyl-phenyl)-4,6-dioxo-octahydro-pyrrolo-[3,4-c]pyrrol-1-carbonsäure;
c) 5-Methyl-1-(2-propyl)-3-(4-carbamimidoyl-phenyl)-4,6-dioxo-octahydropyrrolo[3,4-c]pyrrol-1-carbonsäure;
d) 1-(2-Butyl)-5-methyl-3-(4-carbamimidoyl-phenyl)-4,6-dioxo-octahydropyrrolo[3,4-c]pyrrol-1-carbonsäure;
e) 5-Methyl-1-(2-methylsulfanyl-ethyl)-3-(4-carbamimidoyl-phenyl)-4,6-dioxo-octahydro-pyrrolo[3,4-c]pyrrol-1-carbonsäure;

f) 5-Butyl-3-(4-carbamimidoyl-phenyl)-4,6-dioxo-octahydro-pyrrolo-[3,4-c]pyrrol-1-carbonsäure;
g) 5-Butyl-1-methyl-3-(4-carbamimidoyl-phenyl)-4,6-dioxo-octahydropyrrolo[3,4-c]pyrrol-1-carbonsäure;
h) 5-Butyl-1-(2-propyl)-3-(4-carbamimidoyl-phenyl)-4,6-dioxo-octahydropyrrolo[3,4-c]pyrrol-1-carbonsäure;
i) 5-Butyl-1-(2-butyl)-3-(4-carbamimidoyl-phenyl)-4,6-dioxo-octahydropyrrolo[3,4-c]pyrrol-1-carbonsäure;
j) 5-Butyl-1-(2-methylsulfanyl-ethyl)-3-(4-carbamimidoyl-phenyl)-4,6-dioxo-octahydro-pyrrolo[3,4-c]pyrrol-1-carbonsäure;
k) 5-Cyclohexylmethyl-3-(4-carbamimidoyl-phenyl)-4,6-dioxo-octahydropyrrolo[3,4-c]pyrrol-1-carbonsäure;
l) 5-Cyclohexylmethyl-1-(2-propyl)-3-(4-carbamimidoyl-phenyl)-4,6-dioxooctahydro-pyrrolo[3,4-c]pyrrol-1-carbonsäure;
m) 1-(2-Butyl)-5-cyclohexylmethyl-3-(4-carbamimidoyl-phenyl)-4,6-dioxooctahydro-pyrrolo[3,4-c]pyrrol-1-carbonsäure;
n) 5-Cyclohexylmethyl-1-(2-methylsulfanyl-ethyl)-3-(4-carbamimidoylphenyl)-4,6-dioxo-octahydro-pyrrolo[3,4-c] pyrrol-1-carbonsäure;
o) 5-Phenyl-3-(4-carbamimidoyl-phenyl)-4,6-dioxo-octahydro-pyrrolo-[3,4-c]pyrrol-1-carbonsäure;
p) 1-Methyl-5-phenyl-3-(4-carbamimidoyl-phenyl)-4,6-dioxo-octahydropyrrolo[3,4-c]pyrrol-1-carbonsäure;
q) 5-phenyl-1-(2-propyl)-3-(4-carbamimidoyl-phenyl)-4,6-dioxo-octahydropyrrolo[3,4-c]pyrrol-1-carbonsäure;
r) 1-(2-Butyl)-5-phenyl-3-(4-carbamimidoyl-phenyl)-4,8-dioxo-octahydropyrrolo[3,4-c]pyrrol-1-carbonsäure;
s) 1-(2-Methylsulfanyl-ethyl)-5-phenyl-3-(4-carbamimidoyl-phenyl)-4,6-dioxo-octahydro-pyrrolo[3,4-c]pyrrol-1-carbonsäure;
t) 5-Benzyl-3-(4-carbamimidoyl-phenyl)-4,6-dioxo-octahydro-pyrrolo-[3,4-c]pyrrol-1-carbonsäure;
u) 5-Benzyl-1-methyl-3-(4-carbamimidoyl-phenyl)-4,6-dioxo-octahydropyrrolo[3,4-c]pyrrol-1-carbonsäure;
v) 5-Benzyl-1-(2-propyl)-3-(4-carbamimidoyl-phenyl)-4,6-dioxo-octahydropyrrolo[3,4-c]pyrrol-1-carbonsäure;
w) 5-Benzyl-1-(2-butyl)-3-(4-carbamimidoyl-phenyl)-4,6-dioxo-octahydropyrrolo[3,4-c]pyrrol-1-carbonsäure; bzw.
x) 5-Benzyl-3-(4-carbamimidoyl-phenyl)-1-(2-methylsulfanyl-ethyl)-4,6-dioxo-octahydro-pyrrolo[3,4-c]pyrrol-1-carbonsäure.

Ebenso konnten die folgenden 2-substituierten {4-(4-Carbamimidoylphenyl)-1,3-dioxo-decahydro-pyrrolo [3,4-a]pyrrolizin-8a-yl}-carbonsäuren nachgewiesen werden:

a') {2-Methyl-4-(4-carbamimidoyl-phenyl)-1,3-dioxo-decahydro-pyrrolo-[3,4-a]pyrrolizin-8a-yl}-carbonsäure;
b') {2-Butyl-4-(4-carbamimidoyl-phenyl)-1,3-dioxo-decahydro-pyrrolo-[3,4-a]pyrrolizin-8a-yl}-carbonsäure;
c') {2-Cyclohexylmethyl-4-(4-carbamimidoyl-phenyl)-1,3-dioxo-decahydropyrrolo[3,4-a]pyrrolizin-8a-yl}-carbonsäure;
d') {2-phenyl-4-(4-carbamimidoyl-phenyl)-1,3-dioxo-decahydro-pyrrolo-[3,4-a]pyrrolizin-8a-yl}-carbonsäure; bzw.
e') {2-Benzyl-4-(4-carbamimidoyl-phenyl)-1,3-dioxo-decahydro-pyrrolo-[3,4-a]pyrrolizin-8a-yl}-carbonsäure.

Von den genannten Cycloaddukten zeigte das Rohprodukt, welches 3-(4-Carbamimidoyl-phenyl)-5-cyclohexylmethyl-1-(2-methylsulfanyl-ethyl)-4,6-dioxo-octahydro-pyrrolo[3,4-c]pyrrol-1-carbonsäure enthält, die grösste Hemmwirkung im Thrombintest.

## Beispiel 14

20 g MBHA-Harz (entspricht 22 mmol) werden mit 10 g 3-Brompropionsäure und 7.8 ml Diisopropylcarbodiimid in Dimethylformamid 2 Stunden bei Raumtemperatur geschüttelt. Das Harz wird mit mit Dimethylformamid und Dimethylsulfoxid gewaschen. Je 1 g des Harzes wird danach mit jeweils 10 mmol einer Aminosäure (Glycin, L-Alanin, L-Valin, L-Phenylalanin, L-Leucin und L-Serin) und 100 mg Kaliumiodid versetzt und 8 ml Dimethylsulfoxid als Lösungsmittel zugegeben und danach 48 Stunden bei Raumtemperatur geschüttelt. Jedes dieser Harze wird in 5 gleiche Teile geteilt und 1 mmol 4-Cyano-benzaldehyd sowie jeweils 1 mmol eines Maleinimids (N-Methyl-, N-Butyl-, N-Phenyl-, N-Benzylmaleinimid bzw. N-Phenethyl-maleinimid) in 7 ml Dimethylformamid zugegeben und danach 60 Stunden bei 95 °C gehalten, wobei das Harz mit einem leichten Stickstoffstrom durchmischt wird. Danach wird abgesaugt und das Harz mit Dimethylformamid, Isopropanol, Wasser, Isopropanol und Methylenchlorid gewaschen. Die 30 Harzportionen werden im Hochvakuum 48 Stunden getrocknet und danach bei 0 °C mit 8 ml eines mit trocknem Chlorwasserstoff gesättigtem 5:1 Gemisches aus Chloroform und Methanol versetzt und bei 4 °C 24 Stunden stehen gelassen. Danach wird das Lösungsmittel bei 30 °C im Vakuum entfernt. Nach Zugabe von 8 ml einer mit Ammoniak gesättigten Methanollösung wird 4 Stunden bei 65 °C erhitzt. Nach dem Abfiltrieren werden die 30 Filtratlösungen eingeengt. Man erhält so 30 Rohprodukte als farblose bis gelbe Feststoffe. Für massenspektroskopische und HPLC-Untersuchungen werden ca. je 0.5 mg entnommen und in 100 µl Wasser und 100 µl Acetonitril aufgenommen. Der Rest wird in 0.5 ml DMSO gelöst und nach Beispiel 12 das Rohprodukt mit der höchsten Hemmwirkung ermittelt.

Mit Hilfe von Ionenspray-Massenspektroskopie (ISP-MS) konnte die Anwesenheit der folgenden 3,5-substituierten 1-(4-carbamimidoyl-phenyl)-2-(2-carbamoyl-ethyl)-4,6-dioxo-octahydro-pyrrolo[3,4-c]pyrrol-3-carbonsäuren nachgewiesen werden:

a) 5-Methyl-1-(4-carbamimidoyl-phenyl)-2-(2-carbamoyl-ethyl)-4,6-dioxooctahydro-pyrrolo[3,4-c]pyrrol-3-carbonsäure;
b) 3,5-Dimethyl-1-(4-carbamimidoyl-phenyl)-2-(2-carbamoyl-ethyl)-4,6-dioxo-octahydro-pyrrolo[3,4-c]pyrrol-3-carbonsäure;
c) 5-Methyl-3-(prop-2-yl)-1-(4-carbamimidoyl-phenyl)-2-(2-carbamoylethyl)-4,6-dioxo-octahydro-pyrrolo[3,4-c]pyrrol-3-carbonsäure;
d) 5-Methyl-3-(2-methyl-propyl)-1-(4-carbamimidoyl-phenyl)-2-(2-carbamoyl-ethyl)-4,6-dioxo-octahydro-pyrrolo[3,4-c]pyrrol-3-carbonsäure;
e) 3-Hydroxymethyl-5-methyl-1-(4-carbamimidoyl-phenyl)-2-(2-carbamoylethyl)-4,6-dioxo-octahydro-pyrrolo[3,4-c]pyrrol-3-carbonsäure;
f) 3-Benzyl-5-methyl-1-(4-carbamimidoyl-phenyl)-2-(2-carbamoyl-ethyl)-4,6-dioxo-octahydro-pyrrolo[3,4-c]pyrrol-3-carbonsäure;
g) 5-Butyl-1-(4-carbamimidoyl-phenyl)-2-(2-carbamoyl-ethyl)-4,6-dioxooctahydro-pyrrolo[3,4-c]pyrrol-3-carbonsäure;
h) 5-Butyl-3-methyl-1-(4-carbamimidoyl-phenyl)-2-(2-carbamoyl-ethyl)-4,6-dioxo-octahydro-pyrrolo[3,4-c]pyrrol-3-carbonsäure;
i) 5-Butyl-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure;
j) 5-Butyl-3-(2-methyl-propyl)-1-(4-carbamimidoyl-phenyl)-2-(2-carbamoylethyl)-4,6-dioxo-octahydro-pyrrolo[3,4-c]pyrrol-3-carbonsäure;
k) 5-Butyl-3-hydroxymethyl-1-(4-carbamimidoyl-phenyl)-2-(2-carbamoylethyl)-4,6-dioxo-octahydro-pyrrolo[3,4-c]pyrrol-3-carbonsäure;
l) 3-Benzyl-5-butyl-1-(4-carbamimidoyl-phenyl)-2-(2-carbamoyl-ethyl)-4,6-dioxo-octahydro-pyrrolo[3,4-c]pyrrol-3-carbonsäure;
m) 3-Methyl-5-phenyl-1-(4-carbamimidoyl-phenyl)-2-(2-carbamoyl-ethyl)-4,6-dioxo-octahydro-pyrrolo[3,4-c]pyrrol-3-carbonsäure;
n) 5-Phenyl-3-(prop-2-yl)-1-(4-carbamimidoyl-phenyl)-2-(2-carbamoylethyl)-4,6-dioxo-octahydro-pyrrolo[3,4-c]pyrrol-3-carbonsäure;
o) 3-(2-Methyl-propyl)-5-phenyl-1-(4-carbamimidoyl-phenyl)-2-(2-carbamoyl-ethyl)-4,6-dioxo-octahydro-pyrrolo[3,4-c]pyrrol-3-carbonsäure;
p) 3-Hydroxymethyl-5-phenyl-1-(4-carbamimidoyl-phenyl)-2-(2-carbamoylethyl)-4,6-dioxo-octahydro-pyrrolo[3,4-c]pyrrol-3-carbonsäure;
q) 3-Benzyl-5-phenyl-1-(4-carbamimidoyl-phenyl)-2-(2-carbamoyl-ethyl)-4,6-dioxo-octahydro-pyrrolo[3,4-c]pyrrol-3-carbonsäure;
r) 5-Benzyl-3-methyl-1-(4-carbamimidoyl-phenyl)-2-(2-carbamoyl-ethyl)-4,6-dioxo-octahydro-pyrrolo[3,4-c]pyrrol-3-carbonsäure;
s) 5-Benzyl-3-(2-methyl-propyl)-1-(4-carbamimidoyl-phenyl)-2-(2-carbamoyl-ethyl)-4,6-dioxo-octahydro-pyrrolo[3,4-c]pyrrol-3-carbonsäure;
t) 5-Benzyl-3-Hydroxymethyl-1-(4-carbamimidoyl-phenyl)-2-(2-carbamoylethyl)-4,6-dioxo-octahydro-pyrrolo[3,4-c]pyrrol-3-carbonsäure;
u) 3,5-Dibenzyl-1-(4-carbamimidoyl-phenyl)-2-(2-carbamoyl-ethyl)-4,6-dioxo-octahydro-pyrrolo[3,4-c]pyrrol-3-carbonsäure;
v) 5-(2-Phenyl-ethyl)-1-(4-carbamimidoyl-phenyl)-2-(2-caxbamoyl-ethyl)-4,6-dioxo-octahydro-pyrrolo[3,4-c]pyrrol-3-carbonsäure;
w) 5-(2-Phenyl-ethyl)-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure;
x) 3-(2-Methyl-propyl)-5-(2-phenyl-ethyl)-1-(4-carbamimidoyl-phenyl)-2-(2-carbamoyl-ethyl)-4,6-dioxo-octahydro-pyrrolo[3,4-c]pyrrol-3-carbonsäure;
y) 3-Hydroxymethyl-5-(2-phenyl-ethyl)-1-(4-carbamimidoyl-phenyl)-2-(2-carbamoyl-ethyl)-4,6-dioxo-octahydro-pyrrolo[3,4-c]pyrrol-3-carbonsäure; bzw.
z) 3-Benzyl-1-(4-carbamimidoyl-phenyl)-2-(2-carbamoyl-ethyl)-4,6-dioxooctahydro-5-(2-phenyl-ethyl)-pyrrolo[3,4-c]pyrrol-3-carbonsäure, wobei 1-(4-Carbamimidoyl-phenyl)-2-(2-carbamoyl-ethyl)-3-hydroxymethyl-4,6-dioxooctahydro-5-phenyl-pyrrolo[3,4-c]pyrrol-3-carbonsäure die höchsten Hemmwirkung zeigt.

**Beispiel 15**

Je 1 g MBHA-Harz (entspricht 1.1 mmol) werden mit je 3.3 mmol einer Fmoc-geschützen Aminosäure (Glycin, L-Leucin, L-Phenylalanin, bzw. D-Phenylalanin) und 3.3 mmol Diisopropylcarbodiimid in 7 ml Dimethylformamid 3 Stunden bei Raumtemperatur geschüttelt. Das Harz wird mit Dimethylformamid und Dimethylsulfoxid gewaschen und anschliessend für 30 min in 4 Reaktionsgefässen mit einer 20 %igen Lösung von Piperidin in DMF geschüttelt und dann mit DMF gewaschen. Zu den 4 Reaktionsansätzen werden je 5.5 mmol Maleinsäureanhydrid in 10 ml Dichlormethan gegeben und über 60 Stunden bei Raumtemperatur geschüttelt. Nach Absaugen der Reaktionslösungen und Waschen mit Dichlormethan werden je 2 g Natriumacetat und 10 ml Acetanhydrid zugegeben und bei 100 °C 1 Stunde erhitzt. Anschliessend wird abgesaugt und 4-mal mit Wasser, einer 2 N Natriumcarbonatlösung, Wasser, Isopropanol und Dichlormethan gewaschen. Die 4 Reaktionsansätze werden jeweils auf 5 Gefässe verteilt und in jedes jeweils 1 mmol einer Aminosäure (Glycin, L-Alanin, L-Prolin, L-Hydroxyprolin und L-Serin), sowie 1 mmol 4-Cyano-benzaldehyd in 3 ml DMF gegeben. Die Reaktion wird dann 30 Stunden bei 60 °C und 5 Stunden bei 100 °C durchgeführt. Danach wird abgesaugt und das Harz mit Dimethylformamid, Isopropanol, Wasser, Isopropanol und Methylenchlorid gewaschen. Die 20 Harzportionen werden im Hochvakuum 48 Stunden getrocknet und danach bei 0 °C mit 8 ml eines mit trocknem Chlorwasserstoff gesättigtem 5:1 Gemisches aus Chloroform und Methanol versetzt und bei 4 °C 24 Stunden stehen gelassen. Danach wird das Lösungsmittel bei 30 °C im Vakuum entfernt. Nach Zugabe von 8 ml einer mit Ammoniak gesättigten Methanollösung wird 4 Stunden bei 65 °C erhitzt. Nach dem Abfiltrieren werden die 20 Filtratlösungen eingeengt. Man erhält so 20 Rohprodukte als farblose bis gelbe Feststoffe. Die Rohprodukte werden in 0.5 ml DMSO gelöst und nach Beispiel 12 das Rohprodukt mit der höchsten Hemmwirkung ermittelt. Das Rohprodukt, welches nach Ionenspray-Massenspektroskopie (ISP-MS: 446.2 $[M+H]^+$) 2-{4-(4-Carbamimidoyl-phenyl)-1,3-dioxo-decahydro-pyrrolo[3,4-a]pyrrolizin-2-yl}-3-phenyl-propionsäureamid enthält, zeigt die höchste Hemmwirkung im Thrombintest.

**Beispiel 16**

592 mg O-Benzylserin, 393 mg 4-Cyano-benzaldehyd und 697 mg N-(4-Nitro-benzyl)-maleinimid in 25 ml DMF werden 5 Stunden bei 110°C erhitzt. Nach dem Abkühlen wird das Lösungsmittel am Vakuum entfernt. Das Rohprodukt wird über Kieselgel mit Hexan/Essigester filtriert und das Lösungsmittel entfernt. Danach wird in einem Gemisch aus Methanol/Chloroform (1:5) gelöst und unter Eiskühlung trockner Chlorwasserstoff eingeleitet. Nach 14 Stunden Stehen bei 4°C wird die Lösung mit Ether versetzt, der Niederschlag wird abfiltriert und danach mit einer Ammoniak gesättigten Methanollösung 3 Stunden am Rückfluss gekocht. Das Lösungsmittel wird am Vakuum entfernt. Das so erhaltene Rohprodukt wird mit 3 ml einer 1 N Salzsäure versetzt und erneut eingedampft. Der gelbliche Rückstand wird über eine RP-18 Chromatographiesäule mit einem Wasser/Methanolgradienten getrennt. Man isoliert 67 mg (1RS,3RS,3aSR,6aRS)-4-[3-(Benzyloxy-methyl)-5-(4-nitrobenzyl)-4,6-dioxo-octahydropyrrolo[3,4-c]pyrrol-1-yl]-benzimidamid Hydrochlorid als gelbliches Pulver. ISP-MS: 514 ($[M1H]^+$, 100).

**Beispiel 17**

Aus (1RS,3RS,3aSR,6aRS)-4-[3-(Benzyloxy-methyl)-5-(4-nitro-benzyl)-4,6-dioxo-octahydro-pyrrolo[3,4-c]pyrrol-1-yl]-benzimidamid Hydrochlorid erhält man durch Hydrieren mit 10% Pd auf Kohle in Eisessig bei Raumtemperatur (1RS,3RS,3aSR,6aRS)-4-[5-(4-Amino-benzyl)-3-(benzyloxymethyl)-4,6-dioxo-octahydro-pyrrolo[3,4-c]pyrrol-1-yl]-benzimidamid Hydrochlorid als gelbliches Pulver. ISP-MS: 484 ($[M+H]^+$, 100).

**Beispiel 18**

592 mg O-Benzylserin, 393 mg 4-Cyano-benzaldehyd, 697 mg N-(4-Nitrobenzyl)-maleinimid, 10 Tropfen Essigsäure in 25 ml Methanol werden für 3 Tage unter Rückfluss erhitzt. Nach dem Abkühlen wird das Lösungsmittel am Vakuum entfernt und das Rohprodukt mit Ether verrieben. Das gelbe, kristalline Rohprodukt wird abfiltriert und mit Ether gewaschen. Danach wird in einem Gemisch aus Methanol/Chloroform (1:5) gelöst und unter Eiskühlung trockener Chlorwasserstoff eingeleitet. Nach 14 Stunden Stehen bei 4°C wird die Lösung mit Ether versetzt, der farblose Niederschlag wird abfiltriert und danach mit einer Ammoniak gesättigten Methanollösung 3 Stunden am Rückfluss gekocht. Das Lösungsmittel wird am Vakuum entfernt. Das so erhaltene Rohprodukt wird mit 3 ml einer 1 N Salzsäure versetzt und erneut eingedampft. Der gelbliche Rückstand wird über eine RP-18 Chromatographiesäule mit einem Wasser/Methanolgradienten getrennt. Man isoliert 355 mg (1RS,3RS,3aSR,6aRS)-1-(Benzyloxy-methyl)-3-(4-carbamimidoyl-phenyl)-5-(4-nitro-benzyl)-4,6-dioxo-octahydro-pyrrolo[3,4-c]pyrrol-1-carbonsäure Hydrochlorid als gelbliches Pulver. ISP-MS: 558 ($[M+H]^+$, 100).

**Beispiel 19**

Aus (1RS,3RS,3aSR,6aRS)-1-(Benzyloxy-methyl)-3-(4-carbamimidoylphenyl)-5-(4-nitro-benzyl)-4,6-dioxo-octahydro-pyrrolo[3,4-c]pyrrol-1carbonsäure Hydrochlorid erhält man durch Hydrieren mit 10% Pd auf Kohle in Eisessig bei Raumtemperatur (1RS,3RS,3aSR,6aRS)-5-(4-Aminobenzyl)-1-(benzyloxy-methyl)-3-(4-carbamimidoyl-phenyl)-4,6-dioxooctahydro-pyrrolo[3,4-c]pyrrol-1-carbonsäure Hydrochlorid als gelbliches Pulver. ISP-MS : 528 ($[M+H]^+$, 100).

Eine Verbindung der Formel I, ein Solvat oder Salz davon kann man in an sich bekannter Weise als Wirkstoff zur Herstellung von pharmazeutischen Präparaten, z.B. von Tabletten und Kapseln folgender Zusammensetzung, verwenden:

| Beispiel A | |
|---|---|
| | pro Tablette |
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

| Beispiel B | |
|---|---|
| | pro Kapsel |
| Wirkstoff | 100,0 mg |
| Maisstärke | 20,0 mg |
| Milchzucker | 95,0 mg |
| Talk | 4,5 mg |
| Magensiumstearat | 0,5 mg |
| | 220,0 mg |

**Patentansprüche**

**1.** Octahydro-1,3-dioxo-pyrrolo[3,4-c]pyrrolderivate der Formel

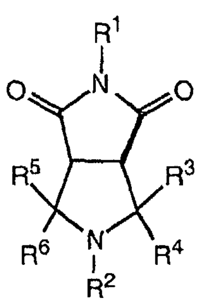

I

worin

| | |
|---|---|
| $R^1$ und $R^2$ | unabhängig voneinander H, nieder-Alkyl, Aryl, Heteroaryl, $C_{3-7}$-Cycloalkyl oder durch $CONH_2$ oder COO-nieder-Alkyl und/oder durch Aryl, Heteroaryl oder $C_{3-7}$-Cycloalkyl substituiertes nieder-Alkyl oder |
| $R^2$ | nieder-Alkanoyl, |
| $R^3$ | H, COOH, $CONH_2$, COO-nieder-Alkyl, CONH-nieder-Alkyl oder CON(nieder-Alkyl)$_2$ und |

| | |
|---|---|
| $R^4$, $R^5$ und $R^6$ | unabhängig voneinander H, nieder-Alkyl, Aryl, Aryl-nieder-Alkyl oder $C_{3-7}$-Cycloalkyl sind oder |
| $R^4$ und/oder eins von $R^5$ und $R^6$ | Heteroaryl oder durch OH, $SO_2H$ $SO_3H$ Guanidino, Aryl-nieder-Alkoxy oder nieder-Alkyl-(O oder S) substituiertes nieder-Alkyl sind/ist oder |
| $R^2$ | zusammen mit $R^4$ eine Tri- oder Tetramethylengruppe G bildet, in der a) eine der Methylengruppen durch S, SO oder $SO_2$ ersetzt oder durch OH, nieder-Alkyl, nieder-Alkenyl oder Carboxy-nieder-Alkyl substituiert oder b) eine der Methylengruppen durch nieder-Alkenyl und eine andere durch nieder-Alkyl-COOH substituiert sein kann, |

wobei zumindest eins von $R^1$, $R^2$, $R^4$, $R^5$ und $R^6$ eine durch Amino, Amidino, Guanidino oder N-Hydroxyamidino substituierte Gruppe ist,
wobei der Ausdruck "nieder" eine bis zu 6 C-Atome enthaltende geradkettige oder verzweigte Gruppe bezeichnet, der Ausdruck "Aryl" Phenyl bezeichnet welches durch Amidino, Guanidino, Hydroxyamidino, Nitro oder Methylendioxy substituiert sein kann, der Ausdruck "Heteroaryl" 5- bis 10-gliedrige aromatische Gruppen bezeichnet die ein oder mehrere N-Atome enthalten und durch eine oder mehrere $NH_2$-Gruppen substituiert sein können,
sowie Hydrate oder Solvate und physiologisch verwendbare Salze davon.

2. Verbindungen nach Anspruch 1, worin $R^1$ bis $R^6$ die gleichen Bedeutungen wie in Anspruch 1 haben, jedoch $R^2$ keine Gruppe G mit $R^4$ bildet, insbesondere worin $R^2$ H, nieder-Alkyl, nieder-Alkanoyl oder durch $CONH_2$ substituiertes nieder-Alkyl, speziell H, Methyl, Carbamoylethyl oder Acetyl ist, worin der Ausdruck "nieder" die gleiche Bedeutung wie in Anspruch 1 hat.

3. Verbindungen nach Anspruch 1, worin $R^2$ zusammen mit $R^4$ eine wie in Anspruch 1 definierte Gruppe G bildet, nämlich der Formel

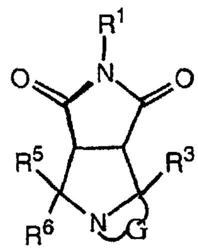

I'

insbesondere worin G eine Gruppe $(CH_2)_{3\text{ oder }4}$ ist, in der eine der $CH_2$-Gruppen durch S, CH-nieder-Alkyl oder CHOH ersetzt oder eine der $CH_2$-Gruppen durch nieder-Alkenyl und eine andere durch nieder-Alkyl-COOH substituiert sein kann, speziell worin G $(CH_2)_{3\text{ oder }4}$, $CH(CH_3)CH_2CH_2$, $CH_2SCH_2$, $CH_2S(O)_2CH_2$, $CH_2CH(OH)CH_2$ oder $CH(CH_2COOH)CH$-(Isopropylen)$CH_2$ ist, worin der Ausdruck "nieder" die gleiche Bedeutung wie in Anspruch 1 hat.

4. Verbindungen nach Anspruch 1, 2 oder 3, worin $R^1$ H, nieder-Alkyl, Aryl oder durch $CONH_2$ oder COO-nieder-Alkyl und/oder durch Aryl oder $C_{3-7}$-Cycloalkyl substituiertes nieder-Alkyl ist, worin die Ausdrücke "Aryl" und "nieder" die gleiche Bedeutung wie in Anspruch 1 haben.

5. Verbindungen nach Anspruch 1, 2 oder 4, worin $R^1$ H, $CH_3$, Butyl, Phenyl, Benzyl, Phenethyl, Cyclohexylmethyl oder durch Nitro, Amino oder Methylendioxy substituiertes Benzyl, insbesondere Benzo[1,3]dioxol-5-ylmethyl ist.

6. Verbindungen nach Anspruch 1, 3 oder 4, worin $R^1$ $CH_3$, Butyl, Phenyl, Benzyl, Cyclohexylmethyl, durch Methylendioxy substituiertes Benzyl oder durch $CONH_2$ oder COO-nieder-Alkyl und Aryl substituiertes nieder-Alkyl, insbesondere Benzo[1,3]dioxol-5-ylmethyl oder 1-(Carbamoyl oder Carbomethoxy)-2-phenethyl ist, worin die Ausdrücke "Aryl" und "nieder" die gleiche Bedeutung wie in Anspruch 1 haben.

7. Verbindungen nach Anspruch 1, 2, 4 oder 5, worin $R^3$ H, COOH oder $CONH_2$, insbesondere worin $R^4$ H, nieder-Alkyl, Aryl, Aryl-nieder-Alkyl oder durch OH, $SO_2H$ Guanidino, Aryl-nieder-Alkoxy oder nieder-Alkylthio substituiertes nieder-Alkyl ist, worin die Ausdrücke "Aryl" und "nieder" die gleiche Bedeutung wie in Anspruch 1 haben.

**8.** Verbindungen nach Anspruch 7, worin $R^4$ H, Methyl, Isopropyl, Isobutyl, 2-Butyl, tert.-Butyl, Phenyl, Benzyl, $CH_2OH$ $CH_2SO_2H$, Guanidinopropyl, Benzyloxymethyl oder $(CH_2)_2SCH_3$ ist.

**9.** Verbindungen nach einem der Ansprüche 1, 2, 4, 5, 7 oder 8, worin $R^5$ und $R^6$ unabhängig voneinander H, nieder-Alkyl, Aryl oder Aryl-nieder-Alkyl, insbesondere H, Methyl, tert.-Butyl, Phenyl, Phenethyl, Amidinophenyl oder Hydroxyamidinophenyl sind, worin die Ausdrücke "Aryl" und "nieder" die gleiche Bedeutung wie in Anspruch 1 haben.

**10.** Verbindungen nach einem der Ansprüche 1, 3, 4 oder 6, worin $R^3$ H oder COOH, insbesondere worin $R^5$ H und $R^6$ Aryl oder Heteroaryl, speziell Amidinophenyl, Guanidinophenyl oder Diaminochinazolin ist, worin die Ausdrücke "Aryl" und "Heteroaryl" die gleiche Bedeutung wie in Anspruch 1 haben.

**11.** Verbindungen nach einem der Ansprüche 1, 2, 4, 5 und 7-9:

(1RS,3SR,3aRS,6aSR)-4-(5-Benzyl-2,3-dimethyl-4,6-dioxo-octahydropyrrolo[3,4-c]pyrrol-1-yl)-benzimidamid
(1RS,3RS,3aSR,6aRS)-1-(Benzyloxy-methyl)-3-(4-carbamimidoyl-phenyl)-5-(4-nitro-benzyl)-4,6-dioxo-octahydro-pyrrolo[3,4-c]pyrrol-1-carbonsäure
(1RS,3RS,3aSR,6aRS)-5-(4-Amino-benzyl)-1-(benzyloxy-methyl)-3-(4-carbamimidoyl-phenyl)-4,6-dioxo-octahydro-pyrrolo[3,4-c]pyrrol-1-carbonsäure.

**12.** Verbindungen nach einem der Ansprüche 1, 3, 4, 6 und 10:

(3aRS,4SR,8aRS,8bSR)-4-(2-Benzyl-1,3-dioxo-decahydro-pyrrolo[3,4-a]-pyrrolizin-4-yl)-benzimidamid
(3aRS,4SR,8aRS,8bSR)-4-{2-(Benzo[1,3]dioxol-5-ylmethyl)-1,3-dioxodecahydro-pyrrolo [3,4-a]pyrrolizin-4-yl}-benzimidamid
(3aRS,4SR,8aRS,8bSR)-4-(2-Cyclohexylmethyl-1,3-dioxo-decahydropyrrolo[3,4-a]pyrrolizin-4-yl)-benzimidamid
(5RS,5aSR,8aRS,8bRS)-4-{7-(Benzo[1,3]dioxol-5-ylmethyl)-6,8-dioxooctahydro-pyrrolo[3',4':3,4]pyrrolo[1,2-c]thiazol-5-yl}-benzimidamid
(3aR,4S,7S,8aR,8bS)-4-(2-Benzyl-7-hydroxy-1,3-dioxo-decahydropyrrolo[3,4-a]pyrrolizin-4-yl)-benzimidamid
(3aRS,4SR,8aRS,8bSR)-N-{4-(2-Butyl-1,3-dioxo-decahydro-pyrrolo-[3,4-a]pyrrolizin-4-yl)-phenyl}-guanidin.

**13.** Verbindungen nach einem der Ansprüche 1, 2, 4, 5 und 7-9:

(1RS,3SR,3aRS,6aSR)-5-Benzyl-1-(3-guanidino-propyl)-4,6-dioxooctahydro-3-phenyl-pyrrolo[3,4-c]pyrrol-1-carbonsäure
(1RS,3aSR,6aRS)-4-(5-Benzyl-2,3,3-trimethyl-4,6-dioxo-octahydropyrrolo[3,4-c]pyrrol-1-ylmethyl)-benzimidamid
(3aRS,4RS,6aSR)-4-[4-(Amino-hydroxyimino-methyl)-benzyl]-2-benzyl-5,6,6-trimethyl-tetrahydro-pyrrolo[3,4-c]pyrrol-1,3-dion
(1RS,3SR,3aRS,6aSR)-4-(5-Benzyl-3-methyl-4,6-dioxo-octahydropyrrolo[3,4-c]pyrrol-1-yl)-benzimidamid
(1RS,3SR,3aRS,6aSR)-4-(2-Acetyl-5-benzyl-3-methyl-4,6-dioxo-octahydropyrrolo[3,4-c]pyrrol-1-yl)-benzimidarnid
(1RS,3SR,3aRS,6aSR)-4-(5-Butyl-2,3-dimethyl-4,6-dioxo-octahydropyrrolo[3,4-c]pyrrol-1-yl)-benzimidamid
(1RS,3SR,3aRS,6aSR)-4-(2-Acetyl-5-butyl-3-methyl-4,6-dioxo-octahydropyrrolo[3,4-c]pyrrol-1-yl)-benzimidamid
(3aRS,4SR,6RS,6aSR)-4-[4-(Amino-hydroxyimino-methyl)-phenyl]-2-benzyl-5,6-dimethyl-tetrahydro-pyrrolo[3,4-c]pyrrol-1,3-dion
(1RS,3SR,3aRS,6aSR)-4-(5-Butyl-3-methyl-4,6-dioxo-octahydropyrrolo[3,4-c]pyrrol-1-yl)-benzimidamid
(1RS,3SR,3aRS,6aSR)-4-(5-Benzyl-3-isobutyl-4,6-dioxo-octahydropyrrolo[3,4-c]pyrrol-1-yl)-benzimidamid
(1RS,3SR,3aRS,6aSR)-4-(5-Benzyl-3-isobutyl-2-methyl-4,6-dioxooctahydro-pyrrolo [3,4-c]pyrrol-1-yl)-benzimidamid
(1RS,3RS,3aSR,6aRS)-{5-(Benzo[1,3]dioxol-5-ylmethyl)-3-(4-carbamimidoyl-phenyl)-4,6-dioxo-octahydro-pyrrolo[3,4-c]pyrrol-1-yl}methansulfinsäure
(1RS,3RS,3aRS,6aSR)-{5-(Benzo[1,3]dioxol-5-ylmethyl)-3-(4-carbamimidoyl-phenyl)-4,6-dioxo-octahydro-pyrrolo[3,4-c]pyrrol-1-yl}methansulfinsäure
(1RS,3SR,3aRS,6aSR)-3-(4-Carbamimidoyl-phenyl)-1-(2-methylsulfanylethyl)-4,6-dioxo-5-phenyl-octahydxo-pyrrolo[3,4-c]pyrrol-1-carbonsäure
(1RS,3SR,3aRS,6aSR)-4-{5-(Benzo[1,3]dioxol-5-ylmethyl)-3-hydroxymethyl-4,6-dioxo-octahydro-pyrrolo [3,4-c]pyrrol-1-yl}-benzimidamid
(1RS,3RS,3aSR,6aRS)-4-{5-(Benzo[1,3]dioxol-5-ylmethyl)-3-hydroxymethyl-4,6-dioxo-octahydro-pyrrolo [3,4-c]pyrrol-1-yl}-benzimidamid
*3-tert.* Butyl-1-(3-guanidino-propyl)-4,6-dioxo-octahydro-pyrrolo-[3,4-c]pyrrol-1-carboxamid
3-Phenyl-1-(3-guanidino-propyl)-4,6-dioxo-octahydro-pyrrolo[3,4-c]pyrrol-1-carboxamid
3-(2-Phenyl-ethyl)-1-(3-guanidino-propyl)-4,6-dioxo-octahydro-pyrrolo [3,4-c]pyrrol-2-carboxamid
5-Methyl-3-(2-phenyl-ethyl)-1-(3-guanidino-propyl)-4,6-dioxo-octahydropyrrolo[3,4-c]pyrrol-1-carboxamid
5-Butyl-3-(2-phenyl-ethyl)-1-(3-guanidino-propyl)-4,6-dioxo-octahydropyrrolo[3,4-c]pyrrol-1-carboxamid
5-Cyclohexylmethyl-3-(2-phenyl-ethyl)-1-(3-guanidino-propyl)-4,6-dioxooctahydro-pyrrolo[3,4-c]pyrrol-1 - carboxamid
5-Benzyl-3-(2-phenyl-ethyl)-1-(3-guanidino-propyl)-4,6-dioxo-octahydropyrrolo[3,4-c]pyrrol-1-carboxamid
5-Phenyl-3-(2-phenyl-ethyl)-1-(3-guanidino-propyl)-4,6-dioxo-octahydropyrrolo[3,4-c]pyrrol-1-carboxamid
5-Butyl-3,3-diphenyl-1-(3-guanidino-propyl)-4,6-dioxo-octahydro-pyrrolo-[3,4-c]pyrrol-1-carboxamid
5-Butyl-3-phenyl-1-(3-guanidino-propyl)-4,6-dioxo-octahydro-pyrrolo-[3,4-c]pyrrol-1-carboxamid
5-Cyclohexylmethyl-3-phenyl-1-(3-guanidino-propyl)-4,6-dioxo-octahydropyrrolo[3,4-c]pyrrol-1-carboxaznid
5-Benzyl-3-phenyl-1-(3-guanidino-propyl)-4,6-dioxo-octahydro-pyrrolo-[3,4-c]pyrrol-1-carboxamid
3,5-Diphenyl-1-(3-guanidino-propyl)-4,6-dioxo-octahydro-pyrrolo-[3,4-c]pyrrol-1-carboxamid
5-Butyl-3-*tert*.butyl-1-(3-guanidino-propyl)-4,6-dioxo-octahydro-pyrrolo-[3,4-c]pyrrol-1-carboxamid
5-Butyl-1-(3-guanidino-propyl)-4,6-dioxo-octahydro-pyrrolo[3,4-c]pyrrol-1-carboxamid
1-(3-Guanidino-propyl)-4,6-dioxo-octahydro-5-phenyl-pyrrolo[3,4-c]pyrrol-1-carboxamid
5-Methyl-3-(4-carbamimidoyl-phenyl)-4,6-dioxo-octahydro-pyrrolo-[3,4-c]pyrrol-1-carbonsäure
1,5-Dimethyl-3-(4-carbamimidoyl-phenyl)-4,6-dioxo-octahydro-pyrrolo-[3,4-c]pyrrol-1-carbonsäure
5-Methyl-1-(2-propyl)-3-(4-carbamimidoyl-phenyl)-4,6-dioxo-octahydropyrrolo[3,4-c]pyrrol-1-carbonsäure
1-(2-Butyl)-5-methyl-3-(4-carbamimidoyl-phenyl)-4,6-dioxo-octahydropyrrolo[3,4-c]pyrrol-1-carbonsäure
5-Methyl-1-(2-methylsulfanyl-ethyl)-3-(4-carbamimidoyl-phenyl)-4,6-dioxo-octahydro-pyrrolo[3,4-c]pyrrol-1-carbonsäure
5-Butyl-3-(4-carbamimidoyl-phenyl)-4,6-dioxo-octahydro-pyrrolo-[3,4-c]pyrrol-1-carbonsäure
5-Butyl-1-methyl-3-(4-carbamimidoyl-phenyl)-4,6-dioxo-octahydropyrrolo[3,4-c]pyrrol-1-carbonsäure
5-Butyl-1-(2-propyl)-3-(4-carbamimidoyl-phenyl)-4,6-dioxo-octahydropyrrolo[3,4-c]pyrrol-1-carbonsäure
5-Butyl-1-(2-butyl)-3-(4-carbamimidoyl-phenyl)-4,6-dioxo-octahydropyrrolo[3,4-cjpyrrol- 1-carbonsäure
5-Butyl-1-(2-methylsulfanyl-ethyl)-3-(4-carbamimidoyl-phenyl)-4,6-dioxooctahydro-pyrrolo[3,4-c]pyrrol-1-carbonsäure
5-Cyclohexylmethyl-3-(4-carbamimidoyl-phenyl)-4,6-dioxo-octahydropyrrolo[3,4-c]pyrrol-1-carbonsäure
5-Cyclohexylmethyl-1-(2-propyl)-3-(4-carbamimidoyl-phenyl)-4,6-dioxooctahydro-pyrrolo[3,4-c]pyrrol-1-carbonsäure
1-(2-Butyl)-5-cyclohexylmethyl-3-(4-carbamirnidoyl-phenyl)-4,6-dioxooctahydro-pyrrolo[3,4-c]pyrrol-1-carbonsäure
5-Cyclohexylmethyl-1-(2-methylsulfanyl-ethyl)-3-(4-carbamimidoylphenyl)-4,6-dioxo-octahydro-pyrrolo [3,4-c]pyrrol-1-carbonsäure
5-Phenyl-3-(4-carbamimidoyl-phenyl)-4,6-dioxo-octahydro-pyrrolo-[3,4-c]pyrrol-1-carbonsäure
1-Methyl-5-phenyl-3-(4-carbamimidoyl-phenyl)-4,6-dioxo-octahydropyrrolo[3,4-c]pyrrol-1-carbonsäure
5-phenyl-1-(2-propyl)-3-(4-carbamimidoyl-phenyl)-4,6-dioxo-octahydropyrrolo[3,4-c]pyrrol-1-carbonsäure
1-(2-Butyl)-5-phenyl-3-(4-carbamimidoyl-phenyl)-4,6-dioxo-octahydropyrrolo[3,4-c]pyrrol-1-carbonsäure
1-(2-Methylsulfanyl-ethyl)-5-phenyl-3-(4-carbamimidoyl-phenyl)-4,6-dioxo-octahydro-pyrrolo[3,4-c]pyrrol-1-carbonsäure
5-Benzyl-3-(4-carbamimidoyl-phenyl)-4,6-dioxo-octahydro-pyrrolo-[3,4-c]pyrrol-1-carbonsäure
5-Benzyl-1-methyl-3-(4-carbamimidoyl-phenyl)-4,6-dioxo-octahydropyrrolo[3,4-c]pyrrol-1-carbonsäuxe
5-Benzyl-1-(2-propyl)-3-(4-carbamimidoyl-phenyl)-4,6-dioxo-octahydropyrrolo[3,4-c]pyrrol-1-carbonsäure
5-Benzyl-1-(2-butyl)-3-(4-carbamimidoyl-phenyl)-4,6-dioxo-octahydropyrrolo[3,4-c]pyrrol-1-carbonsäure
5-Benzyl-3-(4-carbamimidoyl-phenyl)-1-(2-methylsulfanyl-ethyl)-4,6-dioxo-cetahydro-pyrrolo[3,4-c]pyrrol-1-carbonsäure
5-Methyl-1-(4-carbamimidoyl-phenyl)-2-(2-carbamoyl-ethyl)-4,6-dioxooctahydro-pyrrolo[3,4-c]pyrrol-3-carbonsäure
3,5-Dimethyl-1-(4-carbamimidoyl-phenyl)-2-(2-carbamoyl-ethyl)-4,6-dioxo-octahydro-pyrrolo[3,4-c]pyrrol-3-carbonsäure
5-Methyl-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure
5-Methyl-3-(2-methyl-propyl)-1-(4-carbamimidoyl-phenyl)-2-(2-caxbamoyl-ethyl)-4,6-dioxo-octahydro-pyrrolo[3,4-c]pyrrol-3-carbonsäure
3-Hydroxymethyl-5-methyl-1-(4-carbamimidoyl-phenyl)-2-(2-carbamoylethyl)-4,6-dioxo-octahydro-pyrrolo[3,4-c]pyrrol-3-carbonsäure
3-Benzyl-5-methyl-1-(4-carbamimidoyl-phenyl)-2-(2-carbamoyl-ethyl)-4,6-dioxo-octahydro-pyrrolo[3,4-c]pyrrol-3-carbonsäure
5-Butyl-1-(4-carbamimidoyl-phenyl)-2-(2-carbamoyl-ethyl)-4,6-dioxooctahydro-pyrrolo[3,4-c]pyrrol-3-carbonsäure
5-Butyl-3-methyl-1-(4-carbamimidoyl-phenyl)-2-(2-carbamoyl-ethyl)-4,6-dioxo-octahydro-pyrrolo[3,4-c]pyrrol-3-carbonsäure
5-Butyl-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure
5-Butyl-3-(2-methyl-propyl)-1-(4-carbamimidoyl-phenyl)-2-(2-carbamoylethyl)-4,6-dioxo-octahydro-pyrrolo[3,4-c]pyrrol-3-carbonsäure
5-Butyl-3-hydroxymethyl-1-(4-carbamimidoyl-phenyl)-2-(2-carbamoylethyl)-4,6-dioxo-octahydro-pyrrolo[3,4-c]pyrrol-3-carbonsäure
3-Benzyl-5-butyl-1-(4-carbamimidoyl-phenyl)-2-(2-carbamoyl-ethyl)-4,6-dioxo-octahydro-pyrrolo[3,4-c]pyrrol-3-carbonsäure
3-Methyl-5-phenyl-1-(4-carbamimidoyl-phenyl)-2-(2-carbamoyl-ethyl)-4,6-dioxo-octahydro-pyrrolo[3,4-c]pyrrol-3-carbonsäure
5-Phenyl-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure
3-(2-Methyl-propyl)-5-phenyl-1-(4-carbamimidoyl-phenyl)-2-(2-carbamoylethyl)-4,6-dioxo-octahydro-pyrrolo[3,4-c]pyrrol-3-carbonsäure
3-Hydroxymethyl-5-phenyl-1-(4-carbamimidoyl-phenyl)-2-(2-carbamoylethyl)-4,6-dioxo-octahydro-pyrrolo[3,4-c]pyrrol-3-carbonsäure
3-Benzyl-5-phenyl-1-(4-carbamimidoyl-phenyl)-2-(2-carbamoyl-ethyl)-4,6-dioxo-octahydro-pyrrolo[3,4-c]pyrrol-3-carbonsäure
5-Benzyl-3-methyl-1-(4-carbamimidoyl-phenyl)-2-(2-carbamoyl-ethyl)-4,6-dioxo-octahydro-pyrrolo[3,4-c]pyrrol-3-carbonsäure
5-Benzyl-3-(2-methyl-propyl)-1-(4-carbamimidoyl-phenyl)-2-(2-carbamoylethyl)-4,6-dioxo-octahydro-pyrrolo[3,4-c]pyrrol-3-carbonsäure
5-Benzyl-3-Hydroxymethyl-1-(4-carbamimidoyl-phenyl)-2-(2-carbamoylethyl)-4,6-dioxo-octahydro-pyrrolo[3,4-c]pyrrol-3-carbonsäure
3,5-Dibenzyl-1-(4-carbamimidoyl-phenyl)-2-(2-carbamoyl-ethyl)-4,6-dioxooctahydro-pyrrolo[3,4-c]pyrrol-3-carbonsäure
5-(2-Phenyl-ethyl)-1-(4-carbamimidoyl-phenyl)-2-(2-carbamoyl-ethyl)-4,6-dioxo-octahydro-pyrrolo[3,4-c]pyrrol-3-carbonsäure
5-(2-Phenyl-ethyl)-3-(prop-2-yl)-1-(4-carbamimidoyl-phenyl)-2-(2-carbamoyl-ethyl)-4,6-dioxo-octahydro-pyrrolo[3,4-c]pyrrol-3-carbonsäure
3-(2-Methyl-propyl)-5-(2-phenyl-ethyl)-1-(4-carbamimidoyl-phenyl)-2-(2-carbamoyl-ethyl)-4,6-dioxo-octahydro-pyrrolo[3,4-c]pyrrol-3-carbonsäure
3-Hydroxymethyl-5-(2-phenyl-ethyl)-1-(4-carbamimidoyl-phenyl)-2-(2-carbamoyl-ethyl)-4,6-dioxo-octahydro-pyrrolo[3,4-c]pyrrol-3-carbonsäure
3-Benzyl-1-(4-carbamimidoyl-phenyl)-2-(2-carbamoyl-ethyl)-4,6-dioxooctahydro-5-(2-phenyl-ethyl)-pyrrolo[3,4-c]pyrrol-3-carbonsäure
(1RS,3RS,3aSR,6aRS)-4-[3-Benzyloxy-methyl)-5-(4-nitro-benzyl)-4,6-dioxo-octahydro-pyrrolo[3,4-c]pyrrol-1-yl]-benzimidamid
(1RS,3RS,3aSR,6aRS)-4-[5-(4-Aminobenzyl)-3-(benzyloxy-methyl)-4,6-dioxo-octahydro-pyrrolo[3,4-c]pyrrol-1-yl]benzimidamid.

**14.** Verbindungen nach einem der Ansprüche 1, 3, 4, 6 und 10:

(3aRS,4SR,8aRS,8bSR)-4-(2-Butyl-1,3-dioxo-decahydro-pyrrolo[3,4-a]-pyrrolizin-4-yl)-benzimidamid
(3aRS,4RS,9aSR,9bSR)-4-(2-Benzyl-1,3-dioxo-decahydro-pyrrolo[3,4-c]-indolizin-4-yl)-benzimidamid
(3aRS,4SR,9aRS,9bSR)-4-(2-Benzyl-1,3-dioxo-decahydro-pyrrolo[3,4-c]-indolizin-4-yl)-benzimidamid
(3aRS,4RS,8aSR,8bSR)-4-{2-(Benzo[1,3]dioxol-5-ylmethyl)-1,3-dioxodecahydro-pyrrolo[3,4-a]pyrrolizin-4-yl}-benzimidamid
(5RS,5aRS,8aSR,8bRS)-4-{7-(Benzo[1,3]dioxol-5-ylmethyl)-6,8-dioxooctahydro-pyrrolo[3',4':3,4]pyrrolo[1,2-c]thiazol-5-yl}-benzimidamid
(3aS,4R,8S,8aS,8bR)-4-{2-(Benzo[1,3]dioxol-5-ylmethyl)-8-methyl-1,3-dioxo-decahydro-pyrrolo[3,4-a]pyrrolizin-4-yl}-benzimidamid
(3aR,4R,7R,8aS,8bS)-4-(2-Benzyl-7-hydroxy-1,3-dioxo-decahydropyrrolo[3,4-a]pyrrolizin-4-yl)-benzimidamid
(R)-2-{(3aS,4S,8aR,8bR)- und -(3aR,4R,8aS,8bS)-4-(4-Carbamimidoylphenyl)-1,3-dioxo-decahydro-pyrrolo[3,4-a]pyrrolizin-2-yl}-3-phenylpropionsäuremethylester
(R)-2-{(3aR,4S,8aR,8bS)- und -(3aS,4R,8aR.8bR)-4-(4-Carbamimidoylphenyl)-1,3-dioxo-decahydro-pyrrolo[3,4-a]pyrrolizin-2-yl}-3-phenylpropionsäuremethylester
(1S,2S,5R,5aS,8aR)-{7-Benzyl-5-(4-carbamimidoyl-phenyl)-2-(propen-2-yl)-6,8-dioxo-decahydro-pyrrolo[3,4-a]pyrrolizin-1-yl}-essigsäure
(5RS,5aSR,8aRS,8bRS)-4-{7-(Benzo[1,3]dioxol-5-ylmethyl)-2,2,6,8-tetraoxo-octahydro-pyrrolo [3',4':3,4]pyrrolo[1,2-c]thiazol-5-yl}-benzimidamid
(3aRS,4SR,8aRS,8bSR)-*N*-{4-(2-Benzyl-1,3-dioxo-decahydro-pyrrolo-[3,4-a]pyrrolizin-4-yl)-phenyl}-guanidin
(3aRS,4SR,8aRS,8bSR)-*N*-[4-(2-(Benzo[1,3]dioxol-5-ylmethyl)-1,3-dioxodecahydro-pyrrolo[3,4-a]pyrrolizin-4-yl}-phenyl]-guanidin
(3aRS,4SR,8aRS,8bSR)-2-Benzyl-4-(2,4-diamino-quinazolin-6-yl)-hexahydro-pyrrolo[3,4-a]pyrrolizin-1,3-dion
(3aRS,4RS,8aSR,8bSR)-2-Benzyl-4-(2,4-diamino-quinazolin-6-yl)-hexahydro-pyrrolo[3,4-a]pyrrolizin-1,3-dion
(3aRS,4SR,8aRS,8bSR)-2-Benzyl-4-(2,4-diamino-quinazolin-7-yl)-hexahydro-pyrrolo[3,4-a]pyrrolizin-1,3-dion
{2-Methyl-4-(4-carbamimidoyl-phenyl)-1,3-dioxo-decahydro-pyrrolo-[3,4-a]pyrrolizin-8a-yl}-carbonsäure
{2-Butyl-4-(4-carbamimidoyl-phenyl)-1,3-dioxo-decahydro-pyrrolo-[3,4-a]pyrrolizin-8a-yl}-carbonsäure
{2-Cyclohexylmethyl-4-(4-carbamimidoyl-phenyl)-1,3-dioxo-decahydropyrrolo[3,4-a]pyrrolizin-8a-yl}-carbonsäure
{2-phenyl-4-(4-carbamimidoyl-phenyl)-1,3-dioxo-decahydro-pyrrolo-[3,4-a]pyrrolizin-8a-yl}-carbonsäure
{2-Benzyl-4-(4-carbamimidoyl-phenyl)-1,3-dioxo-decahydro-pyrrolo-[3,4-a]pyrrolizin-8a-yl}-carbonsäure und
2-{4-(4-Carbamimidoyl-phenyl)-1,3-dioxo-decahydro-pyrrolo[3,4-a]-pyrrolizin-2-yl}-3-phenyl-propionsäureamid.

**15.** Verbindungen nach einem der Ansprüche 1 bis 14 zur Verwendung als Heilmittel, insbesondere als Hemmer der durch Thrombin oder Faktor Xa induzierten Plättchenaggregation und Gerinnung von Fibrinogen im Blutplasma.

**16.** Verfahren zur Herstellung der Verbindungen nach einem der Ansprüche 1 bis 14, **dadurch gekennzeichnet, dass** man

a) ein Maleimid der Formel

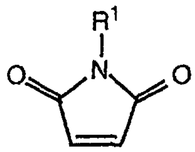

II

mit einer α-Aminocarbonsäure der Formel

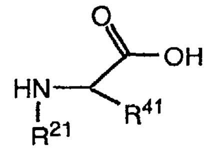

III

worin $R^{21}$ und $R^{41}$ die gleiche Bedeutung wie $R^2$ bzw. $R^4$ in Anspruch 1 haben mit der Massgabe, dass $R^{21}$ nicht nieder-Alkanoyl ist und dass eine durch $R^{21}$ zusammen mit $R^{41}$ gebildete Gruppe G keine Gruppe SO oder $SO_2$ enthält,
oder einem funktionellen Derivat davon und mit einer Verbindung der Formel

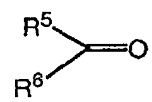

IV

umsetzt,

b) zur Herstellung einer Verbindung der Formel I, in der eins von $R^4$, $R^5$ und $R^6$ eine durch Amidino, N-Hydroxyamidino oder Guanidino substituierte Phenyl- oder nieder-Alkylgruppe ist, eine der Verbindung der Formel I entsprechende Verbindung, die eine durch CN oder $NO_2$ substituierte Phenyloder nieder-Alkylgruppe anstelle der durch Amidino, N-Hydroxyamidino oder Guanidino substituierten Phenyl- oder nieder-Alkylgruppe enthält, amidiniert, N-hydroxyamidiniert oder guanidiniert,

c) gewünschtenfalls eine in einer Verbindung der Formel I enthaltene reaktionsfähige Gruppe funktionell abwandelt, und

d) gewünschtenfalls eine Verbindung der Formel I in ein physiologisch verträgliches Salz überführt oder ein Salz einer Verbindung der Formel I in die freie Säure oder Base überführt,

worin der Ausdruck "nieder" die gleiche Bedeutung wie in Anspruch 1 hat.

**17.** Pharmazeutische Präparate enthaltend eine Verbindung nach einem der Ansprüche 1 bis 14 als Wirkstoff.

**18.** Verwendung einer Verbindung nach einem der Ansprüche 1 bis 14 zur Herstellung von Arzneimitteln zur Behandlung oder Prophylaxe von Krankheiten, die durch Thrombin oder durch Faktor Xa induzierter Plättchenaggregation oder Gerinnung von Fibrinogen im Blutplasma verursacht sind.

## Claims

**1.** Octahydro-1,3-dioxo-pyrrolo[3,4-c]pyrrole derivatives of the formula

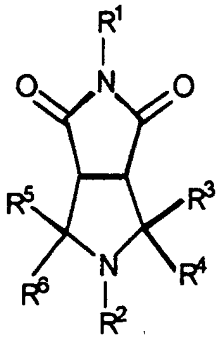

I

wherein

| | |
|---|---|
| $R^1$ and $R^2$ | are each independently H, lower-alkyl, aryl, heteroaryl, $C_{3-7}$-cycloalkyl or lower-alkyl substituted by $CONH_2$ or COO-lower-alkyl and/or by aryl, heteroaryl or $C_{3-7}$-cycloalkyl or |
| $R^2$ | is lower-alkanoyl, |
| $R^3$ | is H, COOH, $CONH_2$, COO-lower-alkyl, CONH-lower-alkyl or CON(lower-alkyl)$_2$ and |
| $R^4$, $R^5$ and $R^6$ | are each independently H, lower-alkyl, aryl, aryl-lower-alkyl or $C_{3-7}$-cycloalkyl or |
| $R^4$ and/or one of $R^5$ and $R^6$ | are/is heteroaryl or lower-alkyl substituted by OH, $SO_2H$, $SO_3H$, guanidino, aryl-lower-alkoxy or lower-alkyl-(O or S) or |
| $R^2$ | and $R^4$ together form a tri- or tetramethylene group G in which a) one of the methylene groups can be replaced by S, SO or $SO_2$ or substituted by OH, lower-alkyl, lower-alkenyl or carboxy-lower-alkyl or b) one of the methylene groups can be substituted by lower-alkenyl and another can be substituted by lower-alkyl-COOH, |

whereby at least one of $R^1$, $R^2$, $R^4$, $R^5$ and $R^6$ is a group substituted by amino, amidino, guanidino or N-hydroxyamidino,
whereby the term "lower" denotes a straight-chain or branched group containing up to 6 C atoms, the term "phenyl" denotes phenyl which can be substituted by amidino, guanidino, hydroxyamidino, nitro or methylenedioxy, the term "heteroaryl" denotes 5- to 10-membered aromatic groups which contain one or more N atoms and which can be substituted by one or more $NH_2$ groups,
as well as hydrates or solvates and physiologically usable salts thereof.

2. Compounds according to claim 1, wherein $R^1$ to $R^6$ have the same significances as in claim 1, but $R^2$ does not form a group G with $R^4$, especially in which $R^2$ is H, lower-alkyl, lower-alkanoyl or lower-alkyl substituted by $CONH_2$, particularly H, methyl, carbamoylethyl or acetyl, wherein the term "lower" has the same significance as in claim 1.

3. Compounds according to claim 1, wherein $R^2$ and $R^4$ together form a group G as defined in claim 1, namely of the formula

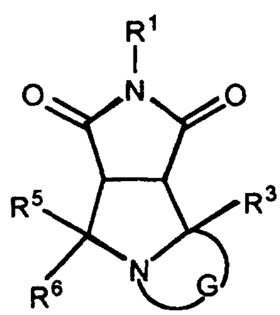

I'

especially in which G is a group $(CH_2)_{3\text{ or }4}$ in which one of the $CH_2$ groups can be replaced by S, CH-lower-alkyl or CHOH or one of the $CH_2$ groups can be substituted by lower-alkenyl and another can be substituted by lower-alkyl-COOH, particularly in which G is $(CH_2)_{3\text{ or }4}$, $CH(CH_3)CH_2CH_2$, $CH_2SCH_2$, $CH_2S(O)_2CH_2$, $CH_2CH(OH)CH_2$ or $CH(CH_2COOH)CH$-(isopropylene)$CH_2$, wherein the term "lower" has the same significance as in claim 1.

4. Compounds according to claim 1, 2 or 3, wherein $R^1$ is H, lower-alkyl, aryl or lower-alkyl substituted by $CONH_2$ or COO-lower-alkyl and/or by aryl or $C_{3-7}$-cycloalkyl, wherein the terms "aryl" and "lower" have the same significance as in claim 1.

5. Compounds according to claim 1, 2 or 4, wherein $R^1$ is H, $CH_3$, butyl, phenyl, benzyl, phenethyl, cyclohexylmethyl or benzyl substituted by nitro, amino or methylenedioxy, especially benzol[1,3]dioxol-5-ylmethyl.

6. Compounds according to claim 1, 3 or 4, wherein $R^1$ is $CH_3$, butyl, phenyl, benzyl, cyclohexylmethyl, benzyl substituted by methylenedioxy or lower-alkyl substituted by $CONH_2$ or COO-lower-alkyl and aryl, especially benzo[1,3]dioxol-5-ylmethyl or 1-(carbamoyl or carbomethoxy)-2-phenethyl, wherein the terms "aryl" and "lower" have the same significance as in claim 1.

**7.** Compounds according to claim 1, 2, 4 or 5, wherein $R^3$ is H, COOH or $CONH_2$, especially in which $R^4$ is H, lower-alkyl, aryl, aryl-lower-alkyl or lower-alkyl substituted by OH, $SO_2H$, guanidino, aryl-lower-alkoxy or lower-alkylthio, wherein the terms "aryl" and "lower" have the same significance as in claim 1.

**8.** Compounds according to claim 7, wherein $R^4$ is H, methyl, isopropyl, isobutyl, 2-butyl, tert.-butyl, phenyl, benzyl, $CH_2OH$, $CH_2SO_2H$ guanidino-propyl, benzyloxymethyl or $(CH_2)_2SCH_3$.

**9.** Compounds according to any one of claims 1, 2, 4, 5, 7 or 8, wherein $R^5$ and $R^6$ are each independently H, lower-alkyl, aryl or aryl-lower-alkyl, especially H, methyl, tert.-butyl, phenyl, phenethyl, amidinophenyl or hydroxyamidinophenyl, wherein the terms "aryl" and "lower" have the same significance as in claim 1.

**10.** Compounds according to any one of claims 1, 3, 4 or 6, wherein $R^3$ is H or COOH, especially in which $R^5$ is H and $R^6$ is aryl or heteroaryl, particularly amidinophenyl, guanidinophenyl or diaminoquinazoline, wherein the terms "aryl" and " heteroaryl" have the same significance as in claim 1.

**11.** Compounds according to any one of claims 1, 2, 4, 5 and 7-9:

(11RS,3SR,3aRS,6aSR)-4-(5-Benzyl-2,3-dimethyl-4,6-dioxo-octahydro-pyrrolo[3,4-c]-pyrrol-1-yl) -benzimidamide
(1RS,3RS,3aSR,6aRS)-1-(benzyloxy-methyl)-3-(4-carbamimidoyl-phenyl)-5-(4-nitrobenzyl)-4,6-dioxo-octahydro-pyrrolo[3,4-c]pyrrole-1-carboxylic acid
(1RS,3RS,3aSR,6aRS)-5-(4-amino-benzyl)-1-(benzyloxy-methyl)-3-(4-carbamimidoyl-phenyl)-4,6-dioxo-octahydro-pyrrolo[3,4-c]pyrrole-1-carboxylic acid.

**12.** Compounds according to any one of claims 1, 3, 4, 6 and 10:

(3aRS,4SR,8aRS,8bSR)-4-(2-Benzyl-1,3-dioxo-decahydro-pyrrolo[3,4-a]pyrrolizin-4-yl)-benzimidamide
(3aRS,4SR,8aRS,8bSR)-4-{2-(benzo[1,3]dioxol-5-ylmethyl)-1,3-dioxo-decahydropyrrolo[3,4-a]pyrrolizin-4-yl}-benzimidamide
(3aRS,4SR,8aRS,8bSR)-4-(2-cyclohexylmethyl-1,3-dioxo-decahydro-pyrrolo[3,4-a]-pyrrolizin-4-yl)-benzimidamide
(5RS,5aSR,8aRS,8bRS)-4-{7-(benzo[1,3]dioxol-5-ylmethyl)-6,8-dioxo-octahydropyrrolo[3',4':3,4]pyrrolol [1,2-c]thiazol-5-yl}-benzimidamide
(3aR,4S,7S,8aR,8bS)-4-(2-benzyl-7-hydroxy-1,3-dioxo-decahydro-pyrrolo[3,4-a]-pyrrolizin-4-yl)-benzimidamide
(3aRS,4SR,8aRS,8bSR)-N-{4-(2-butyl-1,3-dioxo-decahydro-pyrrolo[3,4-a]pyrrolizin-4-yl)-phenyl}-guanidine.

**13.** Compounds according to any one of claims 1, 2, 4, 5 and 7-9:

(1RS,3SR,3aRS,6aSR)-5-benzyl-1-(3-guanidino-propyl)-4,6-dioxo-octahydro-3-phenyl-pyrrolo[3,4-c]pyrrole-1-carboxylic acid
(1RS,3aSR,6aRS)-4-(5-benzyl-2,3,3-trimethyl-4,6-dioxo-octahydro-pyrrolo[3,4-c]-pyrrol-1-ylmethyl)-benzimidamide
(3aRS,4RS,6aSR)-4-[4-(amino-hydroxyimino-methyl)-benzyl]-2-benzyl-5,6,6-trimethyl-tetrahydro-pyrrolo [3,4-c]pyrrole-1,3-dione
(1RS,3SR,3aRS,6aSR)-4-(5-benzyl-3-methyl-4,6-dioxo-octahydro-pyrrolo[3,4-c]-pyrrol-1-yl)-benzimidamide
(1RS,3SR,3aRS,6aSR)-4-(2-acetyl-5-benzyl-3-methyl-4,6-dioxo-octahydro-pyrrolo-[3,4-c]pyrrol-1-yl)-benzimidamide
(1RS,3SR,3aRS,6aSR)-4-(5-butyl-2,3-dimethyl-4,6-dioxo-octahydro-pyrrolo[3,4-c]-pyrrol-1-yl)-benzimidamide
(1RS,3SR,3aRS,6aSR)-4-(2-acetyl-5-butyl-3-methyl-4,6-dioxo-octahydro-pyrrolo-[3,4-c]pyrrol-1-yl)-benzimidamide
(3aRS,4SR,6RS,6aSR)-4-[4-(amino-hydroxyimino-methyl)-phenyl]-2-benzyl-5,6-dimethyl-tetrahydro-pyrrolo [3,4-c]pyrrole-1,3-dione
(1RS,3SR,3aRS,6aSR)-4-(5-butyl-3-methyl-4,6-dioxo-octahydro-pyrrolo[3,4-c]pyrrol-1-yl)-benzimidamide

(1RS,3SR,3aRS,6aSR)-4-(5-benzyl-3-isobutyl-4,6-dioxo-octahydro-pyrrolo[3,4-c]-pyrrol-1-yl)-benzimidamide
(1RS,3SR,3aRS,6aSR)-4-(5-benzyl-3-isobutyl-2-methyl-4,6-dioxo-octahydro-pyrrolo-[3,4-c]pyrrol-1-yl)-benzimidamide
(1RS,3RS,3aSR,6aRS)-{5-(benzo[1,3]dioxol-5-ylmethyl)-3-(4-carbamimidoylphenyl)-4,6-dioxo-octahydro-pyrrolo[3,4-c]pyrrol-1-yl}-methanesulphinic acid
(1RS,3RS,3aRS,6aSR)-{5-(benzo[1,3]dioxol-5-ylmethyl)-3-(4-carbamimidoylphenyl)-4,6-dioxo-octahydro-pyrrolo[3,4-c]pyrrol-1-yl}-methanesulphinic acid,
(1RS,3SR,3aRS,6aSR)-3-(4-carbamimidoyl-phenyl)-1-(2-methylsulphanyl-ethyl)-4,6-dioxo-5-phenyl-octahydro-pyrrolo[3,4-c]pyrrole-1-carboxylic acid
(1RS,3SR,3aRS,6aSR)-4-{5-(benzo[1,3]dioxol-5-ylmethyl)-3-hydroxymethyl-4,6-dioxo-octahydro-pyrrolo[3,4-c]pyrrol-1-yl}-benzimidamide
(1RS,3RS,3aSR,6aRS}-4-{5-(benzo[ 1,3]dioxol-5-ylmethyl)-3-hydroxymethyl-4,6-dioxo-octahydro-pyrrolo[3,4-c]pyrrol-1-yl}-benzimidamide
3-*tert*.butyl-1-(3-guanidino-propyl)-4,6-dioxo-octahydro-pyrrolo[3,4-c]pyrrole-1-carboxamide
3-phenyl-1-(3-guanidino-propyl)-4,6-dioxo-octahydro-pyrrolo[3,4-c]pyrrole-1-carboxamide
3-(2-phenyl-ethyl)-1-(3-guanidino-propyl)-4,6-dioxo-octahydro-pyrrolo[3,4-c]-pyrrole-1-carboxamide
5-methyl-3-(2-phenyl-ethyl)-1-(3-guanidino-propyl)-4,6-dioxo-octahydro-pyrrolo-[3,4-c]pyrrole-1-carboxamide
5-butyl-3-(2-phenyl-ethyl)-1-(3-guanidino-propyl)-4,6-dioxo-octahydro-pyrrolo-[3,4-c]pyrrole-1-carboxamide
5-cyclohexylmethyl-3-(2-phenyl-ethyl)-1-(3-guanidino-propyl)-4,6-dioxo-octahydro-pyrrolo[3,4-c]pyrrole-1-carboxamide
5-benzyl-3-(2-phenyl-ethyl)-1-(3-guanidino-propyl)-4,6-dioxo-octahydro-pyrrolo-[3,4-c]pyrrole-1-carboxamide
5-phenyl-3-(2-phenyl-ethyl)-1-(3-guanidino-propyl)-4,6-dioxo-octahydro-pyrrolo-[3,4-c]pyrrole-1-carboxamide
5-butyl-3,3-diphenyl-1-(3-guanidino-propyl)-4,6-dioxo-octahydro-pyrrolo[3,4-c]-pyrrole-1-carboxamide
5-butyl-3-phenyl-1-(3-guanidino-propyl)-4,6-dioxo-octahydro-pyrrolo[3,4-c]pyrrole-1-carboxamide
5-cyclohexylmethyl-3-phenyl-1-(3-guanidino-propyl)-4,6-dioxo-octahydro-pyrrolo-[3,4-c]pyrrole-1-carboxamide
5-benzyl-3-phenyl- 1-(3-guanidino-propyl)-4,6-dioxo-octahydro-pyrrolo[3,4-c]-pyrrole-1-carboxamide
3,5-diphenyl-1-(3-guanidino-propyl)-4,6-dioxo-octahydro-pyrrolo[3,4-c]pyrrole-1-carboxamide
5-butyl-3-*tert*.butyl-1-(3-guanidino-propyl)-4,6-dioxo-octahydro-pyrrolo[3,4-c]-pyrrole-1-carboxamide
5-butyl-1-(3-guanidino-propyl)-4,6-dioxo-octahydro-pyrrolo[3,4-c]pyrrole-1-carboxamide
1-(3-guanidino-propyl)-4,6-dioxo-octahydro-5-phenyl-pyrrolo[3,4-c]pyrrole-1-carboxamide
5-methyl-3-(4-carbamimidoyl-phenyl)-4,6-dioxo-octahydro-pyrrolo[3,4-c]pyrrole-1-carboxylic acid
1,5-dimethyl-3-(4-carbamimidoyl-phenyl)-4,6-dioxo-octahydro-pyrrolo[3,4-c]-pyrrole-1-carboxylic acid
5-methyl-1-(2-propyl)-3-(4-carbamimidoyl-phenyl)-4,6-dioxo-octahydro-pyrrolo-[3,4-c]pyrrole-1-carboxylic acid
1-(2-butyl)-5-methyl-3-(4-carbamimidoyl-phenyl)-4,6-dioxo-octahydro-pyrrolo-[3,4-c]pyrrole-1-carboxylic acid
5-methyl-1-(2-methylsulphanyl-ethyl)-3-(4-carbamimidoyl-phenyl)-4,6-dioxo-octahydro-pyrrolo[3,4-c]pyrrole-1-carboxylic acid
5-butyl-3-(4-carbamimidoyl-phenyl)-4,6-dioxo-octahydro-pyrrolo[3,4-c]pyrrole-1-carboxylic acid
5-butyl-1-methyl-3-(4-carbamimidoyl-phenyl)-4,6-dioxo-octahydro-pyrrolo[3,4-c]-pyrrole-1-carboxylic acid
5-butyl-1-(2-propyl)-3-(4-carbamimidoyl-phenyl)-4,6-dioxo-octahydro-pyrrolo-[3,4-c]pyrrole-1-carboxylic acid
5-butyl-1-(2-butyl)-3-(4-carbamimidoyl-phenyl)-4,6-dioxo-octahydro-pyrrolo-[3,4-c]pyrrole-1-carboxylic acid
5-butyt-1-(2-methylsulphanyl-ethyl)-3-(4-carbamimidoyl-phenyl)-4,6-dioxo-octahydro-pyrrolo[3,4-c]pyrrole-1-carboxylic acid
5-cyclohexylmethyl-3-(4-carbamimidoyl-phenyl)-4,6-dioxo-octahydro-pyrrolo-[3,4-c]pyrrole-1-carboxylic acid
5-cyclohexylmethyl-1-(2-propyl)-3-(4-carbamimidoyl-phenyl)-4,6-dioxo-octahydro-pyrrolo[3,4-c]pyrrole-1-carboxylic acid
1-(2-butyl)-5-cyclohexylmethyl-3-(4-carbamimidoyl-phenyl)-4,6-dioxo-octahydro-pyrrolo[3,4-c] pyrrole-1-carboxylic acid
5-cyclohexylmethyl-1-(2-methylsulphanyl-ethyl)-3-(4-carbamimidoyl-phenyl)-4,6-dioxo-octahydro-pyrrolo[3,4-c]pyrrole-1-carboxylic acid
5-phenyl-3-(4-carbamimidoyl-phenyl)-4,6-dioxo-octahydro-pyrrolo[3,4-c]pyrrole-1-carboxylic acid
1-methyl-5-phenyl-3-(4-carbamimidoyl-phenyl)-4,6-dioxo-odahydro-pyrrolo[3,4-c]-pyrrole-1-carboxylic acid

5-phenyl-1-(2-propyl)-3-(4-carbamimidoyl-phenyl)-4,6-dioxo-octahydro-pyrrolo-[3,4-c]pyrrole-1-carboxylic acid

1-(2-butyl)-5-phenyl-3-(4-carbamimidoyl-phenyl)-4,6-dioxo-octahydro-pyrrolo-[3,4-c]pyrrole-1-carboxylic acid

1-(2-methylsulphanyl-ethyl)-5-phenyl-3-(4-carbamimidoyl-phenyl)-4,6-dioxo-octahydro-pyrrolo[3,4-c]pyrrole-1-carboxylic acid

5-benzyl-3-(4-carbamimidoyl-phenyl)-4,6-dioxo-octahydro-pyrrolo[3,4-c]pyrrole-1-carboxylic acid

5-benzyl-1-methyl-3-(4-carbamimidoyl-phenyl)-4,6-dioxo-octahydro-pyrrolo[3,4-c]-pyrrole-1-carboxylic acid

5-benzyl-1-(2-propyl)-3-(4-carbamimidoyl-phenyl)-4,6-dioxo-octahydro-pyrrolo-[3,4-c]pyrrole-1-carboxylic acid

5-benzyl-1-(2-butyl)-3-(4-carbamimidoyl-phenyl)-4,6-dioxo-octahydro-pyrrolo-[3,4-c] pyrrole-1-carboxylic acid

5-benzyl-3-(4-carbamimidoyl-phenyl)-1-(2-methylsulphanyl-ethyl)-4,6-dioxo-octahydro-pyrrolo[3,4-c]pyrrole-1-carboxylic acid

5-methyl-1-(4-carbamimidoyl-phenyl)-2-(2-carbamoyl-ethyl)-4,6-dioxo-octahydro-pyrrolo[3,4-c]pyrrole-3-carboxylic acid

3,5-dimethyl-1-(4-carbamimidoyl-phenyl)-2-(2-carbamoyl-ethyl)-4,6-dioxo-octahydro-pyrrolo[3,4-c]pyrrole-3-carboxylic acid

5-methyl-3-(prop-2-yl)-1-(4-carbamimidoyl-phenyl)-2-(2-carbamoyl-ethyl)-4,6-dioxo-octahydro-pyrrolo[3,4-c] pyrrole-3-carboxylic acid

5-methyl-3-(2-methyl-propyl)-1-(4-carbamimidoyl-phenyl)-2-(2-carbamoyl-ethyl)-4,6-dioxo-octahydro-pyrrolo [3,4-c]pyrrole-3-carboxylic acid

3-hydroxymethyl-5-methyl-1-(4-carbamimidoyl-phenyl)-2-(2-carbamoyl-ethyl)-4,6-dioxo-octahydro-pyrrolo [3,4-c]pyrrole-3-carboxylic acid

3-benzyl-5-methyl-1-(4-carbamimidoyl-phenyl)-2-(2-carbamoyl-ethyl)-4,6-dioxo-octahydro-pyrrolo[3,4-c]pyrrole-3-carboxylic acid

5-butyl-1-(4-carbamimidoyl-phenyl)-2-(2-carbamoyl-ethyl)-4,6-dioxo-octahydro-pyrrolo[3,4-c]pyrrole-3-carboxylic acid

5-butyl-3-methyl-1-(4-carbamimidoyl-phenyl)-2-(2-carbamoyl-ethyl)-4,6-dioxo-octahydro-pyrrolo[3,4-c]pyrrole-3-carboxylic acid

5-butyl-3-(prop-2-yl)-1-(4-carbamimidoyl-phenyl)-2-(2-carbamoyl-ethyl)-4,6-dioxo-octahydro-pyrrolo[3,4-c] pyrrole-3-carboxylic acid

5-butyl-3-(2-methyl-propyl)-1-(4-carbamimidoyl-phenyl)-2-(2-carbamoyl-ethyl)-4,6-dioxo-octahydro-pyrrolo [3,4-c]pyrrole-3-carboxylic acid

5-butyl-3-hydroxymethyl-1-(4-carbamimidoyl-phenyl)-2-(2-carbamoyl-ethyl)-4,6-dioxo-octahydro-pyrrolo [3,4-c]pyrrole-3-carboxylic acid

3-benzyl-5-butyl-1-(4-carbainimidoyl-phenyl)-2-(2-carbamoyl-ethyl)-4,6-dioxo-octahydro-pyrrolo[3,4-c]pyrrole-3-carboxylic acid

3-methyl-5-phenyl-1-(4-carbamimidoyl-phenyl)-2-(2-carbamoyl-ethyl)-4,6-dioxo-octahydro-pyrrolo[3,4-c]pyrrole-3-carboxylic acid

5-phenyl-3-(prop-2-yl)-1-(4-carbamimidoyl-phenyl)-2-(2-carbamoyl-ethyl)-4,6-dioxo-octahydro-pyrrolo[3,4-c] pyrrole-3-carboxylic acid

3-(2-methyl-propyl)-5-phenyl-1-(4-carbamimidoyl-phenyl)-2-(2-carbamoyl-ethyl)-4,6-dioxo-octahydro-pyrrolo [3,4-c]pyrrole-3-carboxylic acid

3-hydroxymethyl-5-phenyl-1-(4-carbamimidoyl-phenyl)-2-(2-carbamoyl-ethyl)-4,6-dioxo-octahydro-pyrrolo [3,4-c]pyrrole-3-carboxylic acid

3-benzyl-5-phenyl-1-(4-carbamimidoyl-phenyl)-2-(2-carbamoyl-ethyl)-4,6-dioxo-octahydro-pyrrolo[3,4-c]pyrrole-3-carboxylic acid

5-benzyl-3-methyl-1-(4-carbamimidoyl-phenyl)-2-(2-carbarnoyl-ethyl)-4,6-dioxo-octahydro-pyrrolo[3,4-c]pyrrole-3-carboxylic acid

5-benzyl-3-(2-methyl-propyl)-1-(4-carbamimidoyl-phenyl)-2-(2-carbamoyl-ethyl)-4,6-dioxo-octahydro-pyrrolo [3,4-c]pyrrole-3-carboxylic acid

5-benzyl-3-hydroxymethyl-1-(4-carbamimidoyl-phenyl)-2-(2-carbamoyl-ethyl)-4,6-dioxo-octahydro-pyrrolo [3,4-c]pyrrole-3-carboxylic acid

3,5-dibenzyl-1-(4-carbamimidoyl-phenyl)-2-(2-carbamoyl-ethyl)-4,6-dioxo-octahydro-pyrrolo[3,4-c]pyrrole-3-carboxylic acid

5-(2-phenyl-ethyl)-1-(4-carbamimidoyl-phenyl)-2-(2-carbamoyl-ethyl)-4,6-dioxo-octahydro-pyrrolo[3,4-c]pyrrole-3-carboxylic acid

5-(2-phenyl-ethyl)-3-(prop-2-yl)-1-(4-carbamimidoyl-phenyl)-2-(2-carbamoylethyl)-4,6-dioxo-octahydro-pyrrolo[3,4-c]pyrrole-3-carboxylic acid
3-(2-methyl-propyl)-5-(2-phenyl-ethyl)-1-(4-carbamimidoyl-phenyl)-2-(2-carbamoyl-ethyl)-4,6-dioxo-octahydro-pyrrolo[3,4-c]pyrrole-3-carboxylic acid
3-hydroxymethyl-5-(2-phenyl-ethyl)-1-(4-carbamimidoyl-phenyl)-2-(2-carbamoylethyl)-4,6-dioxo-octahydro-pyrrolo[3,4-c]pyrrole-3-carboxylic acid
3-benzyl-1-(4-carbamimidoyl-phenyl)-2-(2-carbamoyl-ethyl)-4,6-dioxo-octahydro-5-(2-phenyl-ethyl)-pyrrolo[3,4-c]pyrrole-3-carboxylic acid
(1RS,3RS,3aSR,6aRS)-4-[3-benzyloxy-methyl)-5-(4-nitro-benzyl)-4,6-dioxo-octahydro-pyrrolo[3,4-c]pyrrol-1-yl]-benzimidamide
(1RS,3RS,3aSR,6aRS)-4-[5-(4-aminobenzyl)-3-(benzyloxy-methyl)-4,6-dioxo-octahydro-pyrrolo[3,4-c]pyrrrol-1-yl]benzimidamide.

**14.** Compounds according to any one of claims 1, 3, 4, 6 and 10:

(3aRS,4SR,8aRS,8bSR)-4-(2-butyl-1,3-dioxo-decahydro-pyrrolo[3,4-a]pyrrolizin-4-yl)-benzimidamide
(3aRS,4RS,9aSR,9bSR)-4-(2-benzyl-1,3-dioxo-decahydro-pyrrolo[3,4-c] indolizin-4-yl)-benzimidamide
(3aRS,4SR,9aRS,9bSR)-4-(2-benzyl-1,3-dioxo-decahydro-pyrrolo[3,4-c]indolizin-4-yl)-benzimidamide
(3aRS,4RS,8aSR,8bSR)-4-{2-(benzo[1,3]dioxol-5-ylmethyl)-1,3-dioxo-decahydro-pyrrolo[3,4-a]pyrrolizin-4-yl}-benzimidamide
(5RS,5aRS,8aSR,8bRS)-4-{7-(benzo[1,3]dioxol-5-ylmethyl)-6,8-dioxo-octahydro-pyrrolo[3',4':3,4]pyrrolo[1,2-c]thiazol-5-yl}-benzimidamide
(3aS,4R,8S,8aS,8bR)-4-{2-(benzo[1,3]dioxol-5-ylmethyl)-8-methyl-1,3-dioxo-decahydro-pyrrolo[3,4-a]pyrrolizin-4-yl}-benzimidamide
(3aR,4R,7R,8aS,8bS)-4-(2-benzyl-7-hydroxy-1,3-dioxo-decahydro-pyrrolo[3,4-a]-pyrrolizin-4-yl}-benzimidamide
methyl (R)-2-{(3aS,4S,8aR,8bR)- and -(3aR,4R,8aS,8bS)-4-(4-carbamimidoyl-phenyl)-1,3-dioxo-decahydro-pyrrolo[3,4-a]pyrrolizin-2-yl}-3-phenyl-propionate
methyl (R)-2-{(3aR,4S,8aR,8bS)- and -(3aS,4R,8aR.8bR)-4-(4-carbamimidoyl-phenyl)-1,3-dioxo-decahydro-pyrrolo[3,4-a]pyrrotizin-2 -yl}-3-phenyl-propionate
(1S,2S,5R,5aS,8aR)-{7-benzyl-5-{4-carbamimidoyl-phenyl)-2-(propen-2-yl)-6,8-dioxo-decahydro-pyrrolo[3,4-a]pyrrolizin-1-yl}-acetic acid
(5RS,5aSR,8aRS,8bRS)-4-{7-(benzo[1,3]dioxol-5-ylmethyl)-2,2,6,8-tetraoxo-octahydro-pyrrolo[3',4':3,4]pyrrolo[1,2-c]thiazol-5-yl}-benzimidamide
(3aRS,4SR,8aRS,8bSR)-*N*-{4-(2-benzyl-1,3-dioxo-decahydro-pyrrolo[3,4-a]-pyrrolizin-4-yl)-phenyl}-guanidine
(3aRS,4SR,8aRS,8bSR)-*N*-[4-{2-benzo[ 1,3]dioxol-5-ylmethyl)-1,3-dioxo-decahydro-pyrrolo[3,4-a]pyrrolizin-4-yl)-phenyl}-guanidine
(3aRS,4SR,8aRS,8bSR)-2-benzyl-4-{2,4-diamino-quinazolin-6-yl)-hexahydro-pyrrolo[3,4-a]pyrrolizine-1,3-dione
(3aRS,4RS,8aSR,8bSR)-2-benzyl-4-(2,4-diamino-quinazolin-6-yl)-hexahydro-pyrrolo[3,4-a]pyrrolizine-1,3-dione
(3aRS,4SR,8aRS,8bSR)-2-benzyl-4-(2,4-diamino-quinazolin-7-yl)-hexahydro-pyrrolo[3,4-a]pyrrolizine-1,3-dione
{2-methyl-4-(4-carbamimidoyl-phenyl)-1,3-dioxo-decahydro-pyrrolo[3,4-a]-pyrrolizin-8a-yl}-carboxylic acid
{2-butyl-4-(4-carbamimidoyl-phenyl)-1,3-dioxo-decahydro-pyrrolo[3,4-a]pyrrolizin-8a-yl}-carboxylic acid
{2-cyclohexylmethyl-4-(4-carbamimidoyl-phenyl)-1,3-dioxo-decahydro-pyrrolo-[3,4-a]pyrrolizin-8a-yl}-carboxylic acid
{2-phenyl-4-(4-carbamimidoyl-phenyl)-1,3-dioxo-decahydro-pyrrolo[3,4-a]-pyrrolizin-8a-yl}-carboxylic acid
{2-benzyl-4-(4-carbamimidoyl-phenyl)-1,3-dioxo-decahydro-pyrrolo[3,4-a]-pyrrolizin-8a-yl}-carboxylic acid and
2-{4-(4-carbamimidoyl-phenyl)-1,3-dioxo-decahydro-pyrrolo[3,4-a]pyrrolizin-2-yl}-3-phenyl-propionamide.

**15.** Compounds according to any one of claims 1 to 14 for use as medicaments, especially as inhibitors of thrombin-induced or factor Xa-induced platelet aggregation and fibrinogen clotting in blood plasma.

**16.** A process for the manufacture of the compounds according to any one of claims 1 to 14, **characterized by**

a) reacting a maleimide of the formula

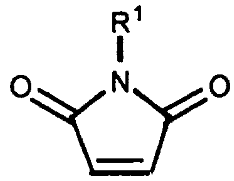

II

with an α-aminocarboxylic acid of the formula

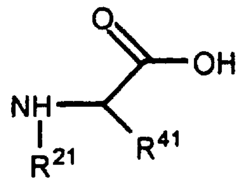

III

wherein $R^{21}$ and $R^{41}$ have the same significance as $R^2$ and, respectively, $R^4$ in claim 1, with the proviso that $R^{21}$ is not lower-alkanoyl and that a group G formed by $R^{21}$ and $R^{41}$ together does not contain a SO or $SO_2$ group,
or a functional derivative thereof and with a compound of the formula

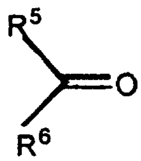

IV

b) for the manufacture of a compound of formula I in which one of $R^4$, $R^5$ and $R^6$ is a phenyl or lower-alkyl group substituted by amidino, N-hydroxyamidino or guanidino, amidinating, N-hydroxyamidinating or guanidinating a compound corresponding to a compound of formula I which contains a phenyl or lower-alkyl group substituted by CN or $NO_2$ in place of the phenyl or lower-alkyl group substituted by amidino, N-hydroxyamidino or guanidino,

c) if desired, functionally modifying a reactive group present in a compound of formula I, and

d) if desired, converting a compound of formula I into a physiologically compatible salt or converting a salt of a compound of formula I into the free acid or base,

wherein the term "lower" has the same significance as in claim 1.

**17.** Pharmaceutical preparations containing a compound according to any one of claims 1 to 14 as the active ingredient.

**18.** The use of a compound according to any one of claims 1 to 14 for the production of medicaments for the treatment or prophylaxis of illnesses which are caused by thrombin-induced or factor Xa-induced platelet aggregation or fibrinogen clotting in blood plasma.

**Revendications**

**1.** Dérivés d'octahydro-1,3-dioxo-pyrrolo[3,4-c]-pyrrole répondant à la formule

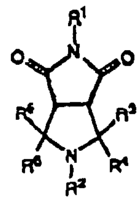

I

| | |
|---|---|
| dans desquels $R^1$ et $R^2$ | indépendamment l'un de l'autre, représentent un atome de H, un groupe alkyle inférieur, aryle, hétéroaryle, cycloalkyle en $C_3$-$C_7$ ou COO alkyle inférieur et/ou un alkyle inférieur substitué par un groupe aryle, hétéroaryle ou cycloalkyle en $C_3$-$C_7$ ou |
| $R^2$ | un alcanoyle inférieur, |
| $R^3$ | H, COOH, $CONH_2$, COO alkyle inférieur, CONH alkyle inférieur ou CON(alkyle inférieur)$_2$ et |
| $R^4$, $R^5$ et $R^6$ | indépendamment de H, représentent un groupe alkyle inférieur, aryle, aryle alkyle inférieur ou cycloalkyle en $C_1$-$C_7$ ou |
| $R^4$ et/ou soit $R^5$ soit $R^6$ | représente(nt) un groupe hétéroaryle ou un alkyle inférieur substitué par un groupe OH, $SO_2H$, $SO_3H$, guanidino, aryle alcoxy inférieur ou alkyle inférieur-(O ou S) ou |
| $R^2$ | forme avec $R^4$ un groupe tri ou tétramethyléne G dans lequel a) un des groupes méthylène peut être remplacé par un groupe OH, SO, $SO_2$ ou substitué par un groupe OH, alkyle inférieur, alcényle inférieur ou carboxy-alkyle inférieur ou b) un des groupes méthylène peut être substitué par un groupe alcényle inférieur et un autre par un groupe alkyle inférieur-COOH. |

au moins un des groupes $R^1$,$R^2$,$R^4$,$R^5$ et $R^6$ est un groupe substitué par amino, amidino, guanidino ou N-hydroxyamidino,
l'expression "inférieur" désignant un groupe linéaire ou ramifié contenant jusqu'à 6 atomes de carbone, l'expression "aryle" désignant un phényle qui peut être substitué par amidino, guanidino, hydroxyamidino, nitro ou méthylènedioxy, l'expression "hétéroaryle" désignant des groupes aromatiques comprenant de 5 à 10 chaînons, contenant un ou plusieurs atomes N et pouvant être substitués par un ou plusieurs groupes $NH_2$,
ainsi que des hydrates et des solvates et les sels physiologiquement acceptables de ces derniers.

2. Composés selon la revendication 1, dans lesquels les groupes $R^1$ à $R^6$ ont les mêmes significations que dans la revendication 1, mais $R^2$ ne forme pas de groupe G avec $R^4$, en particulier dans lesquels $R^2$ représente H, un groupe alkyle inférieur, alcanoyle inférieur ou alkyle inférieur substitué par $CONH_2$, spécialement H, méthyle, carbamoyléthyle ou acétyle, dans lesquels l'expression "inférieur" a la même signification que dans la revendication 1.

3. Composés selon la revendication 1, dans lesquels $R^2$ et $R^4$ forment ensemble un groupe G tel qu'il est défini dans la revendication 1, à savoir qu'il répond à la formule

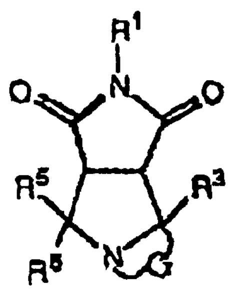

I'

en particulier dans lesquels G est un groupe $(CH_2)_{3\ ou\ 4}$, dans lequel un des groupes $CH_2$ est remplacé par S, CH alkyle inférieur ou CHOH ou un des groupes $CH_2$ est substitué par un alcényl inférieur et un autre par un alkyle inférieur COOH, spécialement dans lesquels G représente $(CH_2)_{3\ ou\ 4}$, $CH(CH_3)CH_2CH_2$, $CH_2SCH_2$. $CH_2S(O)_2CH_2$,$CH_2CH(OH)CH_2$ ou

$CH(CH_2COOH)CH$-(isopropylène)$CH_2$, dans lesquels l'expression "inférieur" a la même signification que dans la revendication 1.

**4.** Composés selon les revendications 1, 2 ou 3 dans lesquels $R_1$ représente H, un alkyle inférieur, un aryle, un alkyle inférieur substitué par $CONH_2$ ou COO alkyle inférieur et/ou par aryle ou cycloalkyle en $C_3$-$C_7$, dans lesquels les expressions "aryle" et "inférieur" ont la même signification que dans la revendication 1.

**5.** Composés selon les revendications 1, 2 ou 4, dans lesquels $R^1$ représente $H_2$, $CH_3$, butyle, phényle, benzyle, phénéthyle, cyclohexylméthyle ou un groupe butyle substitué par nitro, amino ou méthylènedioxy, en particulier le benzo[1,3]dioxol-5-ylméthyle.

**6.** Composés selon les revendications 1, 3 ou 4, dans lesquels $R_1$ représente $CH_3$, butyle, phényle, benzyle, cyclohexylméthyle ou un groupe benzyle substitué par méthylènedioxy ou un groupe alkyle inférieur substitué par CONH2 ou par COO alkyle inférieur et aryle, en particulier le benzo[1,3]dioxol-5-ylméthyle ou le 1-(carbamoyl ou carbométhoxy)-2-phénéthyle, dans lesquels les expressions "aryle" et "inférieur" ont les mêmes significations que dans la revendication 1.

**7.** Composés selon les revendications 1, 2, 4 ou 5, dans lesquels $R^3$ représente H, COOH ou $CONH_2$, en particulier dans lesquels $R^4$ représente H, un groupe alkyle inférieur, aryle, aryle alkyle inférieur ou alkyle inférieur substitué par OH, $SO_2H$, guanidino, aryle alcoxy inférieur ou alkylthio inférieur, dans lesquels les expressions "aryle" et "inférieur" ont les mêmes significations que dans la revendication 1.

**8.** Composés selon la revendication 7, dans lesquels $R^4$ représente H, le méthyle, l'isopropyle, l'isobutyle, le 2-butyle, le tert.butyle, le phényle, le benzyle, le $CH_2OH$, le $CH_2SO_2H$, le guanidinopropyle, le benzyloxyméthyle ou le $(CH_2)_2SCH_3$.

**9.** Composés selon l'une quelconque des revendications 1 2, 4, 5, 7 ou 8, dans lesquels $R^5$ et $R^6$ représentent, indépendamment l'un de l'autre, H, un groupe alkyle inférieur, aryle ou aryle alkyle inférieur, en particulier H, méthyle, tert.butyle, phényle, phénéthyle, amidinophényle ou hydroxyamidinophényle, dans lesquels les expressions "aryle" et "inférieur" ont les mêmes significations que dans la revendication 1.

**10.** Composés selon l'une quelconque des revendications 1, 3, 4 ou 6, dans lesquels $R^3$ représente H ou COOH, en particulier dans lesquels $R^5$ représente H et $R^6$ un groupe aryle ou hétéroaryle, spécialement amidinophényle, gunidinophényle et diaminoquinazoline, dans lesquels les expressions "aryle" et "hétéroaryle" ont les mêmes significations que dans la revendication 1.

**11.** Composés selon l'une quelconque des revendications 1, 2, 4, 5 et 7-9 :

(1RS,3SR,3aRS,6aSR)-4-(5-benzyl-2,3-diméthyl-4,6-dioxo-octahydro-pyrrolo[3,4-c]pyrrol-1-yl)-benzimidamide
acide (1RS,3RS,3aSR,6aRS)-1-(benzyloxy-méthyl)-3-(4-carbamimidoyl-phényl)-5-(4-nitro-benzyl)-4,6-dioxo-octahydro-pyrrolo[3,4-c]pyrrol-1-carboxylique
acide (1RS,3RS,3aSR,6aRS)-5-(4-amino-benzyl)-1-(benzyloxy-méthyl)-3-(4-carbamimidoyl-phényl)-4,6-dioxo-octahydro-pyrrolo[3,4-c]pyrro-1-carboxylique.

**12.** Composés selon l'une quelconque des revendications 1, 3, 4, 6 et 10 :

(3aRS,4SR,8aRS,8bSR)-4-(2-benzyl-1,3-dioxo-décahydro-pyrrolo[3,4-a]-pyrrolizin-4-yl)-benzimidamide
(3aRS,4SR,8aRS,8bSR)-4-[2-(benzo[1,3]dioxo-5-ylméthyl)-1,3-dioxo-décahydro-pyrrolo[3,4-a]pyrrolizin-4-yl)-benzimidamide
(3aRS,4SR,8aRS,8bSR)-4-(2-cyclohexylméthyl-1,3-dioxo-décahydro-pyrrola[3,4-a]pyrrolizin-4-yl)-benzimidamide
(5RS,5aSR,8aRS,8bSR)-4-(7-(benzo[1,3]dioxol-5-ylméthyl)-6,8-dioxo-octahydro-pyrrolo[3',4':3,4]pyrrolo[1,2-c]thiazol-5-yl)-benzimidamide
(3aR,4S,7S,8aR,8bS)-4-(2-benzyl-7-hydroxy-1,3-dioxo-décahydro-pyrrolo[3,4-a]pyrrolizin-4-yl)-benzimidamide
(3aRS,4SR,8aRS,8bSR)-N-(4-(2-butyl-1,3-dioxo-décahydro-pyrrolo[3,4-a]pyrrolizin-4-yl)-phényl)-guanidine.

**13.** Composés selon l'une quelconque des revendications 1, 2, 4, 5 et 7-9 :

acide (1RS,3SR,3aRS,6aSR)-5-benzyl-1-(3-guanidino-propyl)-4,6-dioxo-octahydro-3-phényl-pyrrolo[3,4-c]pyrrol-1-carboxylique
(1RS,3aSR,6aRS)-4-(5-benzyl-2,3,3-triméthyl-4,6-dioxo-octahydro-pyrrolo[3,4-c]pyrrol-1-ylméthyl)-benzimidamide
(3aRS,4RS,6aSR)-4-[4-(amino-hydroxyimino-méthyl)-benzyl]-2-benzyl-5,6,6-triméthyl-tétrahydro-pyrrolo[3,4-c]pyrrol-1,3-dione
(1RS,3SR,3aRS,6aSR)-4-(5-benzyl-3-méthyl]-4,6-dioxo-octahydro-pyrrolo[3,4-c]pyrrol-1-yl)-benzimidamide
(1RS,3SR,3aRS,6aSR)-4-(2-acétyl-5-benzyl-3-méthyl-4,6-dioxo-octahydro-pyrrolo[3,4-c]pyrrol-1-yl)-benzimidamide
(1RS,3SR,3aRS,6aSR)-4-(5-butyl-2,3-diméthyl]-4,6-dioxo-octahydro-pyrrolo[3,4-c]pyrrol-1-yl)-benzimidamide
(1RS,3SR,3aRS,6aSR)-4-(2-acétyl-5-butyl-2,3-méthyl-4,6-dioxo-octahydro-pyrrolo[3,4-c]pyrrol-1-yl)-benzimidamide
(3aRS,4SR,6RS,6aSR)-4-[4-(amino-hydroxyimino-méthyl)-phényl]-2-benzyl-5,6-diméthyl-tetrahydro-pyrrolo[3,4-c]pyrrol-1,3-dione
(1RS,3SR,3aRS,6aSR)-4-(5-butyl-3-méthyl-4,6-dioxo-octahydro-pyrrolo[3,4-c]pyrrol-1-yl)-benzimidamide
(1RS,3SR,3aRS,6aSR)-4-(5-benzyl-3-isobutyl-4,6-dioxo-octahydro-pyrrolo[3,4-c]pyrrol-1-yl)-benzimidamide
(1RS,3SR,3aRS,6aSR)-4-(5-benzyl-3-isobutyl-2-méthyl-4,6-dioxo-octahydro-pyrrolo[3,4-c]pyrrol-1-yl)-benzimidamide
acide (1RS,3RS,3aSR,6aRS)-[5-(benzyl[1,3]dioxol-5-ylméthyl)-3-(4-carbamimidoyl-phényl)-4,6-dioxo-octahydro-pyrrolo[3,4-c]pyrrol-1-yl)-sulfonique
acide (1RS,3RS,3aRS,6aSR)-[5-(benzo[1,3]dioxo]-5-ylméthyl)-3-(4-carbamimidoyl-phényl)-4,6-dioxo-octahydro-pyrrolo[3,4-]pyrrol-1-yl)-méthasulfoninique
acide (1RS,3SR,3aRS,6aSR)-3-(4-carbamimidoyl-phényl)-1-(2-méthylsulfanyl-éthyl)-4,6-dioxo-5-phényl-octahydro-pyrrolo[3,4-c]pyrrol-1-yl)-carboxylique
(1RS,3SR,3aRS,6aSR)-4-(5-benzo[1,3]dioxol-5-ylméthyl)-3-hydroxyméthyl-4-4,6-dioxo-octahydro-pyrrolo[3,4-c]pyrrol-1-yl)-benzimidamide
(1RS,3RS,3aSR,6aRS)-4-(5-benzo[1,3]dioxol-5-ylméthyl)-3-hydroxyméthyl-4,6-dioxo-octahydro-pyrrolo[3,4-c]pyrrol-1-yl)-benzimidamide
3-*tert*.butyl-1-(3-guanidino-propyl)-4,6-dioxo-octahydro-pyrrolo[3,4-c]pyrrol-1-carboxamide
3-phényl-1-(3-guanidino-propyl)-4,6-dioxo-octahydro-pyrrolo[3,4-c]pyrrol-1-carboxamide
3-(2-phényl-éthyl)-1-(3-guanidino-propyl)-4,6-dioxo-octahydro-pyrrolo[3,4-c]pyrrol-1-carboxamide
5-méthyl-3-(2-phényl-éthyl)-1-(3-guanidino-propyl)-4,6-dioxo-octahydro-pyrrolo[3,4-c]pyrrol-1-carboxamide
5-butyl-3-(2-phényl-éthyl)-1-(3-guanidino-propyl)-4,6-dioxo-octahydro-pyrrolo[3,4-c]pyrrol-1-carboxamide
5-cyclohexylméthyl-3-(2-phényl-éthyl)-1-(3-guanidino-propyl)-4,6-dioxo-octahydro-pyrrolo[3,4-c]pyrrol-1-carboxamide
5-benzyl-3-(2-phényl-éthyl)-1-(3-guanidino-propyl)-4,6-dioxo-octahydro-pyrrolo[3,4-c]pyrrol-1-carboxamide
5-phényl-3-(2-phényl-éthyl)-1-(3-guanidino-propyl)-4,6-dioxo-octahydro-pyrrolo[3,4-c]pyrrol-1-carboxamide
5-butyl-3,3-diphényl-1-(3-guanidino-propyl)-4,6-dioxo-octahydro-pyrrolo[3,4-c]pyrrol-1-carboxamide
5-butyl-3-phényl-1-(3-guanidino-propyl)-4,6-dioxo-octahydro-pyrrolo[3,4-c]pyrrol-1-carboxamide
5-cyclohexylméthyl-3-phényl-1-(3-guanidino-propyl)-4,6-dioxo-octahydro-pyrrolo[3,4-c]pyrrol-1-carboxamide
5-benzyl-3-phényl-1-(3-guanidino-propyl)-4,6-dioxo-octahydro-pyrrolo[3,4-c]pyrrol-1-carboxamide
3,5-diphényl-1-(3-guanidino-propyl)-4,6-dioxo-octahydro-pyrrolo[3,4-c]pyrrol-1-carboxamide
5-butyl-3-*tert*.butyl-1-(3-guanidino-propyl)-4,6-dioxo-octahydro-pyrrolo[3,4-c]pyrrol-1-carboxamide
5-butyl-1-(3-guanidino-propyl)-4,6-dioxo-octahydro-pyrrolo[3,4-c]pyrrol-1-carboxamide.
1-(3-guanidino-propyl)-4,6-dioxo-octahydro-5-phényl-pyrrolo[3,4-c]pyrrol-carboxamide-1-carboxamide
acide 5-méthyl-3-(4-carbamimidoyl-phényl)-4,6-dioxo-octahydro-pyrrolo[3,4-c]-pyrrol-1-carboxylique
acide 1,5-dimethyl-3-(4-carbamimidoyl-phényl)-4,6-dioxo-octahydro-pyrrolo[3,4-c]pyrrol-1-carboxylique
acide 5-méthyl-1-(2-propyl)-3-(4-carbamimidoyl-phényl)-4,6-dioxo-octahydro-pyrrolo[3,4-c]-pyrrol-1-carboxylique
acide 1-(butyl)-5-méthyl-3-(4-carbamimidoyl-phényl)-4,6-dioxo-octahydro-pyrrolo[3,4-c]pyrrol-1-carboxylique
acide 5-méthyl-1-(2-méthylsulfanyl)-éthyl)-3-(4-carbamimidoyl-phényl)-4,6-dioxo-octahydro-pyrrolo[3,4-c]-pyrrol-1-carboxylique
acide 5-butyl-3-(4-carbamimidoyl-phényl)-4,6-dioxo-octahydro-pyrrolo[3,4-c]pyrrol-1-carboxylique
acide 5-butyl-1-méthyl-3-(4-carbamimidoyl-phényl)-4,6-dioxo-octahydro-pyrrolo[3,4-c]pyrrol-1-carboxylique
acide 5-butyl-1-(2-propyl)-3-(4-carbamimidoyl-phényl)-4,6-dioxo-octahydro-pyrrolo[3,4-c]pyrrol-1-carboxylique

acide 5-butyl-1-(2-butyl)-3-(4-carbamimidoyl-phényl)-4,6-dioxo-octahydro-pyrrolo[3,4-c]pyrrol-1-carboxylique

acide 5-butyl-1-(2-méthylsulfanyl-éthyl)-3-(4-carbamimidoyl-phényl)-4,6-dioxo-octahydro-pyrrolo[3,4-c]pyrrol-1-carboxylique

acide 5-cyclohexylméthyl-3-(4-carbamimidoyl-phényl)-4,6-dioxo-octahydro-pyrrolo[3,4-c]pyrrol-1-carboxylique

acide 5-cyclohexylméthyl-1-(propyl)-3-(4-carbamimidoyl-phényl)-4,6-dioxo-octahydro-pyrrolo[3,4-c]pyrrol-1-carboxylique

acide 1-(2-butyl)-5-cyclohexylméthyl-3-(4-carbamimidoyl-phényl)-4,6-dioxo-octahydro-pyrrolo[3,4-c]pyrrol-1-carboxylique

acide 5-cyclohexylméthyl-1-(2-méthylsufanyl-éthyl)-3-(4-carbamimidoyl-phényl)-4,6-dioxo-octahydro-pyrrolo[3,4-c]pyrrol-1-carboxylique

acide 5-phényl-3-(4-carbamimidoyl-phényl)-4,6-dioxo-octahydro-pyrrolo[3,4-c]pyrrol-1-carboxylique

acide 1-méthyl-5-phényl-3-(4-carbamimidoyl-phényl)-4,6-dioxo-octahydro-pyrrolo[3,4-c]pyrrol-1-carboxylique

acide 5-phényl-1-(2-propyl)-3-(4-carbamimidoyl-phényl)-4,6-dioxo-octahydro-pyrrolo[3,4-c]pyrrol-1-carboxylique

acide 1-(2-butyl)-5-phényl-3-4-carbamimidoyl-phényl)-4,6-dioxo-octahydro-pyrrolo[3,4-c]pyrrol-1-carboxylique

acide 1-(2-méthylsulfanyl-éthyl)-5-phényl-3-(4-carbamimidoyl-phényl)-4,6-dioxo-octahydro-pyrrolo[3,4-c]pyrrol-1-carboxylique

acide 5-benzyl-3-(4-carbamimidoyl-phényl)-4,6-dioxo-octahydro-pyrrolo[3,4-c]pyrrol-1-carboxylique

acide 5-benzyl-1-méthyl-3-(4-carbamimidoyl-phényl)-4,6-dioxo-octahydro-pyrrolo[3,4-c]pyrrol-1-carboxylique

acide 5-benzyl-1-(2-propyl)-3-(4-carbamimidoyl-phényl)-4,6-dioxo-octahydro-pyrrolo[3,4-c]pyrrol-1-carboxylique

acide 5-benzyl-1-(2-butyl)-3-(4-carbamimidoyl-phényl)-4,6-dioxo-octahydro-pyrrolo[3,4-c]pyrrol-1-carboxylique

acide 5-benzyl-3-(4-carbamimidoyl-phényl)-1-(2-méthylsulfanyl-éthyl)-4,6-dioxo-octahydro-pyrrolo[3,4-c]pyrrol-3-carboxylique

acide 5-benzyl-1-(4-carbamimidoyl-phényl)-2-(2-carbamoyl-éthyl)-4,6-dioxo-octahydro-pyrrolo[3,4-c]pyrrol-3-carboxylique

acide 3,5-diméthyl-1-(4-carbamimidoyl-phényl)-2-(2-carbamoyl-éthyl)-4,6-dioxo-octahydro-pyrrolo[3,4-c]pyrrol-3-carboxylique

acide 5-méthyl-3-(prop-2-yl)-1-(4-carbamimidoyl-phényl)-2-(2-carbamoyl-éthyl)-4,6-dioxo-octahydro-pyrrolo[3,4-c]pyrrol-3-carboxylique

acide 5-méthyl-3-(2-méthyl-propyl)-1-(4-carbamimidoyl-phényl)-2-(2-carbamoyl-éthyl)-4,6-dioxo-octahydro-pyrrolo[3,4-c]pyrrol-3-carboxylique

acide 3-hydroxyméthyl-5-méthyl-1-(4-carbamimidoyl-phényl)-2-(2-carbamoyl-éthyl)-4,6-dioxo-octahydro-pyrrolo[3,4-c]pyrrol-3-carboxylique

acide 3-benzyl-5-méthyl-1-(4-carbamimidoyl-phényl)-2-(2-carbamoyl-éthyl)-4,6-dioxo-octahydro-pyrrolo[3,4-c]pyrrol-3-carboxylique

acide 5-butyl-1-(4-carbamimidoyl-phényl)-2-(2-carbamoyl-éthyl)-4,6-dioxo-octahydro-pyrrolo[3,4-c]pyrrol-3-carboxylique

acide 5-butyl-3-méthyl-1-(4-carbamimidoyl-phényl)-2-(2-carbamoyl-éthyl)-4,6-dioxo-octahydro-pyrrolo[3,4-c]pyrrol-3-carboxylique

acide 5-butyl-3-(prop-2-yl)-1-(4-carbamimidoyl-phényl)-2-(2-carbatnoyl-éthyl)-4,6-dioxo-octahydro-pyrrolo[3,4-c]pyrrol-3-carboxylique

acide 5-butyl-3-(2-méthyl-propyl)-1-(4-carbamimidoyl-phényl)-2-(2-carbamoyl-éthyl)-4,6-dioxo-octahydro-pyrrolo[3,4-c]pyrrol-3-carboxylique

acide 5-butyl-3-hydroxyméthyl-1-(4-carbamimidoyl-phényl)-2-(2-carbamoyl-éthyl)-4,6-dioxo-octahydro-pyrrolo[3,4-c]pyrrol-3-carboxylique

acide 3-benzyl-5-butyl-1-(4-carbamimidoyl-phényl)-2-(2-carbamoyl-éthyl)-4,6-dioxo-octahydro-pyrrolo[3,4-c]pyrrol-3-carboxylique

acide 3-méthyl-5-phényl-1-(4-carbamimidoyl-phényl)-2-(2-carbamoyl-éthyl)-4,6-dioxo-octahydro-pyrrolo[3,4-c]pyrrol-3-carboxylique

acide 5-phényl-3-(prop-2-yl)-1-(4-carbamimidoyl-phényl)-2-(2-carbamoyl-éthyl)-4,6-dioxo-octahydro-pyrrolo

[3,4-c]pyrrol-3-carboxylique
acide 3-(2-méthyl-propyl)-5-phényl-1-(4-carbamimidoyl-phényl)-2-(2-carbamoyl-éthyl)-4,6-dioxo-octahydro-pyrrolo[3,4-c]pyrrol-3-carboxylique
3-hydroxyméthyl-5-phényl-1-(4-carbamimidoyl-phényl)-2-(2-carbamoyl-éthyl)-4,6-dioxo-octahydro-pyrrolo [3,4-c]pyrrol-3-carboxylique
3-benzyl-5-phényl-1-(4-carbamimidoyl-phényl)-2-(2-carbamoyl-éthyl)-4,6-dioxo-octahydro-pyrralo[3,4-c]pyrrol-3-carboxylique
5-benzyl-3-méthyl-1-(4-carbamirnidoyl-phényl)-2-(2-carbamoyl-éthyl)-4,6-dioxo-octahydro-pyrrolo[3,4-c]pyrrol-3-carboxylique
5-benzyl-3-(2-méthyl-propyl)-1-(4-carbamimidoyl-phényl)-2-(2-carbamoyl-éthyl)-4,6-dioxo-octahydro-pyrrolo [3,4-c]pyrrol-3-carboxylique
5-benzyl-3-hydroxyméthyl-1-(4-carbamimidoyl-phényl)-2-(2-carbamoyl-éthyl)-4,6-dioxo-octahydro-pyrrolo [3,4-c]pyrrol-3-carboxylique
3,5-dibenzyl-1-(4-carbamimidoyl-phényl)-2-(2-carbamoyl-éthyl)-4,6-dioxo-octahydro-pyrrolo[3,4-c]pyrrol-3-carboxylique
5-(2-phényl-éthyl)-1-(4-carbamimidoyl-phényl)-2-(2-carbamoyl-éthyl)-4,6-dioxo-octahydro-pyrrolo[3,4-c]pyrrol-3-c;arhoxylique
5-(2-phényl-éthyl)-3-(prop-2-yl)-1-(4-carbamimidoyl-phényl)-2-(2-carbamoyl-éthyl)-4,6-dioxo-octahydro-pyrrolo[3,4-c]pyrrol-3-carboxylique
3-(2-méthyl-propyl)-5-(2-phényl-éthyl)-1-(4-carbamimidoyl-phényl)-2-(2-carbamoyl-éthyl)-4,6-dioxo-octahydro-pyrrolo[3,4-c]pyrrol-3-carboxylique
3-hydroxyméthyl-5-(2-phényl-éthyl)-1-(4-carbamimidoyl-phényl)-2-(2-carbamoyl-éthyl)-4,6-dioxo-octahydro-pyrrolo[3,4-c]pyrrol-3-carboxylique
3-benzyl-1-(4-carbamimidoyl-phényl)-2-(2-carbamoyl-éthyl)-4,6-dioxo-octahydro-5-(2-phényl)-pyrrolo[3,4-c] pyrrol-3-carboxylique
(1RS,3RS,3aSR,6aRS)-4-[3-benxyloxy-méthyl)-5-(4-nitro-benzyl)-4,6-dioxo-octahydro-pyrrolo[3,4-c]pyrrol-1-yl]-benzimidamide
(1RS,3RS,3aSR,6aRS)-4-[5-(4-aminobenzyl)-3-(benzyloxy-méthyl)-4,6-dioxo-octahydro-pyrrolo[3,4-c]pyrrol-1-yl]-benzimidamide

**14.** Composés selon l'une quelconque des revendications 1, 3, 4, 6 et 10 :

(3aRS,4SR,8aRS,8bSR)-4-(2-butyl-1,3-dioxo-décahydro-pyrrolo[3,4-a]pyrrolizinc-4-yl]-benzimidamide
(3aRS,4RS,9aSR,9bSR)-4-(2-benzyl-1,3-dioxo-décahydro-pyrrolo[3,4-c]indolizine-4-yl]-benzimidamide
(3aRS,4SR,9aRS,9bSR)-4-(2-benzyl-1,3-dioxo-décahydro-pyrrolo[3,4-c]indolizine-4-yl]-benzimidamide
(3aRS,4SR,8aSR,8bSR)-4-(2-benzo[1,3]dioxol-5-ylméthyl)-1,3-dioxo-décahydro-pyrrolo[3,4-a]pyrrolizin-4-yl]-benzimidamide
(5RS,5aRS,8aSR,8bRS)-4-(7-benzo[1,3]dioxol-5-ylméthyl)-6,8-dioxo-octahydro-pyrrolo[3',4':3.4]pyrrolo [1,2-c]thiazol-5-yl)-benzimidamide
(3aS,4R,8S,8aS,8bR)-4-(2-benzo[1,3]dioxol-5-ylmethyl)-8-méthyl-1,3-dioxo-décahydro-pyrrolo[3,4-a]pyrrolizin-4-yl)-benzimidamide
(3aR,4R,7R,8aS,RbS)-4-(2-benzyl-7-hydroxy-1,3-dioxo-décahydro-pyrrolo[3,4-a]pyrrolizin-4-yl)-benzimidamide
(R )-2-[(3aS,4S,8aR,8bR)- et -(3aR,4R,8aS,8bS)-4-(4-carbamimidoyl-phényl)-1,3-dioxo-décahydro-pyrrolo [3,4-a]pyrrolizin-2-yl)-3-phényl propionate de méthyle
(R )-2-[(3aR,4S,8aR,8bS)- et -(3aS,4R,8aR,8bR)-4-(4-carbamimidoyl-phényl)-1,3-dioxo-décahydro-pyrrolo [3,4-a]pyrrolizin-2-yl)-3-phényl propionate de méthyle
acide (1S,2S,5R,5aS,8aR)-(7-benzyl-5-(4-carbamimidoyl-phényl)-2-(propène-2-yl)-6,8-dioxo-décahydro-pyrrolo[3,4-a]pyrrolizin-1-yl) acétique
(5RS,5aSR,8aRS,8bRS)-4-(7-benzo[1,3]dioxol-5-ylméthyl)-2,2,6,8-tétraoxo-octahydro-pyrrolo[3',4':3,4]pyrrolo[1,2-c]thiazol-5-yl)-benzimidamide
(3aRS,4SR,8aRS,8bSR)-*N*-(4-(2-benzyl-1,3-dioxo-décahydro-pyrrolo[3,4-a]pyrrolizin-4-yl)-phényl-guanidine
(3aRS,4SR,8aRS,8bSR)-*N*[4-(2-benzo[1,3]dioxol-5-ylméthyl)-1,3-dioxo-décahydro-pyrrolo[3,4-a]pyrrolizin-4-yl)-phényl-guanidine
(3aRS,4SR,8aRS,8bSR)-2-benzyl-4-(2,4-diamino-quinazolin-6-yl)-hexahydro-pyrrolo[3,4-a]pyrrolizin-1,3-dione
(3aRS,4RS,8aSR,8bSR)-2-benzyl-4-(2,4-diamino-quinazolin-6-yl)-hexahydro-pyrrolo[3,4-a]pyrrolizin-1,3-dione

(3aR5,4SR,8aRS,8bSR)-2-benzyl-4-(2,4-diamino-quinazolin-7-yl)-hexahydro-pyrrolo[3,4-a]pyrralizin-1,3-dione
acide (2-méthyl-4-(4-carbamimidoyl-phényl)-1,3-dioxo-décahydro-pyrrolo[3,4-a]pyrrolizin-8a-yl)-carboxylique
acide (2-butyl-4-(4-carbamimidoyl-phényl)-1,3-dioxo-décahydro-pyrrolo[3,4-a]pyrrolizin-8a-yl)-carboxylique
acide (2-cyclohexylméthyl-4-(4-carbamimidoyl-phényl)-1,3-dioxo-décahydro-pyrrolo[3,4-a]pyrrolizin-8a-yl)-carboxylique
acide (2-phényl-4-(4-carbamimidoyl-phényl)-1,3-dioxo-décahydro-pyrrolo[3,4-a]pyrrolizin-8a-yl)-carboxylique
acide (2-benzyl-4-(4-carbamimidoyl-phényl)-1,3-dioxo-décahydro-pyrrolo[3,4-a]pyrrolizin-8a-yl)-carboxylique
amidc de l'acide propionique 2-(4-(4-carbamimidoyl-phényl)-1,3-dioxo-décahydro-pyrrolo[3,4-a]pyrrolizin-2-yl)

**15.** Composés selon l'une quelconque des revendications 1 à 14 destinés à servir de médicament, en particulier d'inhibiteur de l'agrégation plaquettaire induite par la thrombine ou le facteur Xa et de la coagulation du fibrinogène dans le plasma sanguin.

**16.** Procédé de préparation des composés selon une quelconque des revendications de 1 à 14, **caractérisé par le fait que** l'on fait réagir

a) un maléimide répondant à la formule

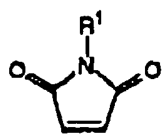

II

avec un acide a-aminocarboxylique répondant à la formule

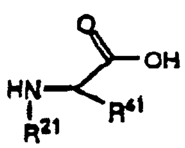

III

dans lequel $R^{21}$ et $R^{41}$ ont la même signification que $R^2$ ou $R^4$ dans la revendication 1 et dans lequel $R^{21}$ n'est pas un alcanoyl inférieur et un groupe G formé par $R^{21}$ et $R^{41}$ ne contient pas de groupe SO ou $SO_2$, ou un de ses dérivés fonctionnels et avec un composé répondant à la formule

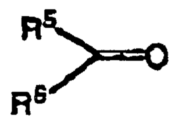

IV

b) et que, dans le but de préparer un composé répondant à la formule 1, dans lequel un des groupes $R^4$, $R^5$ et $R^6$ représente un groupe phényl ou alkyle inférieur substitué par amidino, N-hydroxyamidino ou guanidino, on amidinise, N-hydroxyamidinise ou guanidinise un des composés répondant à la formule I, qui contient un groupe phényl ou alkyle inférieur substitué par CN ou $NO_2$ en remplacement du groupe phényl ou alkyle inférieur substitué par amidino, N-hydroxyamidino ou guanidino.
c) si on le souhaite, on modifie fonctionnellement un groupe réactionnel contenu dans un composé répondant à la formule I, et
d) si on le souhaite, on convertit un composé répondant à la formule 1 en sel acceptable physiologiquement ou on convertit un sel d'un composé répondant à la formule 1 en acide ou base libre.

dans lesquels l'expression "inférieur" a la même signification que dans la revendication 1.

**17.** Préparations pharmaceutiques contenant comme principe actif un composé selon l'une quelconque des revendications 1 à 14.

**18.** Utilisation d'un composé selon l'une quelconque des revendications 1 à 14 pour préparer des médicaments destinés au traitement ou à la prophylaxie de maladies, provoquées par l'agrégation plaquettaire induite par la thrombine ou le facteur Xa ou dues à la coagulation de fibrinogène dans le plasma sanguin.